(19) Europäisches Patentamt
European Patent Office
Office européen des brevets

(11) EP 3 556 848 A1

(12) **EUROPEAN PATENT APPLICATION**
published in accordance with Art. 153(4) EPC

(43) Date of publication:
**23.10.2019 Bulletin 2019/43**

(21) Application number: **17880322.7**

(22) Date of filing: **14.12.2017**

(51) Int Cl.:
***C12N 5/077*** *(2010.01)* ***A61K 35/17*** *(2015.01)*
***A61K 39/395*** *(2006.01)* ***A61K 48/00*** *(2006.01)*
***A61P 43/00*** *(2006.01)* ***C12N 5/0735*** *(2010.01)*
***C12N 5/10*** *(2006.01)* ***C07K 16/28*** *(2006.01)*
***C12N 5/0783*** *(2010.01)* ***C12N 15/09*** *(2006.01)*

(86) International application number:
**PCT/JP2017/044937**

(87) International publication number:
**WO 2018/110654 (21.06.2018 Gazette 2018/25)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(30) Priority: **15.12.2016 JP 2016243755**

(71) Applicants:
- **Heartseed Inc.**
  **Tokyo 106-0045 (JP)**
- **National Cancer Center**
  **Tokyo 104-0045 (JP)**

(72) Inventors:
- **FUKUDA Keiichi**
  **Tokyo 160-8582 (JP)**
- **NAKATSURA Tetsuya**
  **Kashiwa-shi**
  **Chiba 277-8577 (JP)**

(74) Representative: **Cabinet Becker et Associés**
**25, rue Louis le Grand**
**75002 Paris (FR)**

# (54) UNDIFFERENTIATED STEM CELL REMOVAL AGENT AND METHOD FOR REMOVING UNDIFFERENTIATED STEM CELLS

(57) An undifferentiated stem cell removal agent is provided, containing at least one component selected from the group consisting of the following (a) to (d): (a) a cell that is capable of specifically inhibiting proliferation of a glypican-3-expressing cell; (b) a compound that is capable of specifically inhibiting proliferation of a glypican-3-expressing cell; (c) a cell that is capable of inducing a specific immune response against a glypican-3-expressing cell; and (d) a compound that is capable of inducing a specific immune response against a glypican-3-expressing cell.

EP 3 556 848 A1

# Description

## Technical Field

The present invention relates to an undifferentiated stem cell removal agent and a method for removing undifferentiated stem cells. The present invention further relates to a production method of cells for transplantation, and a pharmaceutical composition, which use the undifferentiated stem cell removal agent.

Priority is claimed on Japanese Patent Application No. 2016-243755, filed on December 15, 2016, the content of which is incorporated herein by reference.

## Background Art

Research on pluripotent stem cells such as embryonic stem cells (ES cells) and induced pluripotent stem cells (iPS cells) has progressed in recent years. Because these cells are pluripotent, it is becoming possible to produce cells that differentiate into a desired lineage through differentiation induction, and to use the produced cells in medical transplantation.

Differentiation induction of embryonic stem cells and induced pluripotent stem cells is generally carried out in vitro. However, in vitro, it is difficult to give rise to the differentiation of a target lineage with respect to all stem cells. Therefore, undifferentiated stem cells may remain partly after the differentiation induction manipulation. Because such undifferentiated stem cells have proliferative activity and can differentiate into various kinds of cells, in a case where these undifferentiated stem cells are transplanted into a living body, a teratoma may be formed (for example, NPL 1). For this reason, it is difficult to directly transplant a cell population, which is produced through differentiation induction of stem cells, into a living body as it is to be used in treatment.

Accordingly, it is necessary to remove undifferentiated stem cells in order to safely carry out transplantation of cells differentiation-induced from stem cells into a living body to obtain ideal therapeutic effects. In addition, in a case where transplantation is carried out with undifferentiated stem cells remaining, it is necessary to suppress proliferation of undifferentiated stem cells in a living body.

As a method for removing undifferentiated stem cells in a cell population differentiation-induced from stem cells, a method in which a cell population is cultured by using a culture medium having a specific composition, and undifferentiated stem cells are selectively removed; and the like have been proposed (for example, PTLs 1 to 4 and NPLs 2 to 4).

Meanwhile, glypican-3 is a GPI-anchored protein that belongs to the heparan sulfate proteoglycans (HSPGs) family. Glypican-3 is known to be overexpressed in cancer, particularly liver cancer, and is involved in proliferation and apoptosis of cancer cells. With regard to glypican-3, antibodies (for example, PTL 5) and peptide vaccines (PTL 6 and PTL 7) for use in cancer treatment have been reported.

## Citation List

### Patent Literature

[PTL 1] PCT International Publication No. WO2007/088874
[PTL 2] PCT International Publication No. WO2009/017254
[PTL 3] PCT International Publication No. WO2010/114136
[PTL 4] PCT International Publication No. WO2016/010165
[PTL 5] PCT International Publication No. WO2006/006693
[PTL 6] PCT International Publication No. WO2004/018667
[PTL 7] PCT International Publication No. WO20071018199


### Non-Patent Literature

[NPL 1] Miura et al., (2009) Nature Biotech., 8, 743-745.
[NPL 2] Ben-David, et al., (2013) Cell Stem Cell, 12, 167-179.
[NPL 3] Wang, et al., (2009) Science, 325, 435-439.
[NPL 4] Shiraki, et al., (2014) Cell Metabolism, 19, 780-794.

## Summary of Invention

### Technical Problem

In the method in which a culture medium having a specific composition is used, and undifferentiated stem cells are selectively removed as disclosed in PTLs 1 to 4 and NPL 2 to 4, because a culture medium from which a specific culture medium component such as amino acid has been removed is used, differentiated cells may be affected thereby. In addition, there is no report on a method for removing undifferentiated stem cells mixed into transplanted cells in a living body after transplantation.

An object of the present invention is to provide a technique for selectively removing only undifferentiated stem cells without affecting differentiated cells, at the time of preparation of the differentiated cells induced from the undifferentiated stem cells, and in a living body after transplantation.

### Solution to Problem

As a result of repeated intensive studies to achieve the above-mentioned object, the inventors of the present invention have found that glypican-3 is expressed only in undifferentiated stem cells and is not expressed in differentiated cells. In addition, the inventors of the present invention have found that only undifferentiated stem cells can be selectively removed by targeting glypican-3, and therefore have completed the present invention.

The present invention includes the following aspects.

[1] An undifferentiated stem cell removal agent, containing at least one component selected from the group consisting of the following (a) and (b):

(a) a cell that is capable of specifically inhibiting proliferation of a glypican-3-expressing cell; and
(b) a compound that is capable of specifically inhibiting proliferation of a glypican-3-expressing cell.

[2] The undifferentiated stem cell removal agent according to [1], in which the cell that is capable of specifically inhibiting proliferation of a glypican-3-expressing cell of (a) is a cytotoxic T cell that has cytotoxic activity specific to a glypican-3-expressing cell.

[3] The undifferentiated stem cell removal agent according to [1], in which the compound that is capable of specifically inhibiting proliferation of a glypican-3-expressing cell of (b) is an antibody against glypican-3 protein.

[4] An undifferentiated stem cell removal agent, containing at least one component selected from the group consisting of the following (c) and (d):

(c) a cell that is capable of inducing a specific immune response against a glypican-3-expressing cell; and
(d) a compound that is capable of inducing a specific immune response against a glypican-3-expressing cell.

[5] The undifferentiated stem cell removal agent according to [4], in which the compound that is capable of inducing a specific immune response against a glypican-3-expressing cell of (d) is a glypican-3 protein or a partial peptide thereof, or a salt thereof.

[6] The undifferentiated stem cell removal agent according to [4], in which the compound that is capable of inducing a specific immune response against a glypican-3-expressing cell of (d) is a vector containing a polynucleotide that encodes a glypican-3 protein or a partial peptide thereof.

[7] The undifferentiated stem cell removal agent according to [4], in which the cell that is capable of inducing a specific immune response against a glypican-3-expressing cell of (c) is a dendritic cell presenting a partial peptide of a glypican-3 protein at a cell surface.

[8] The undifferentiated stem cell removal agent according to any one of [1] to [7], in which the undifferentiated stem cell is an induced pluripotent stem cell.

[9] A method for removing an undifferentiated stem cell, including culturing a cell mixture that contains an undifferentiated stem cell and a differentiated cell in the presence of the undifferentiated stem cell removal agent according to any one of [1] to [3].

[10] The method for removing an undifferentiated stem cell according to [9], in which the undifferentiated stem cell is an induced pluripotent stem cell.

[11] The method for removing an undifferentiated stem cell according to [9] or [10], in which the differentiated cell is a cardiomyocyte.

[12] A production method of a cell for transplantation, including the following steps (i) and (ii):

a step (a) of inducing a desired differentiated cell from an undifferentiated stem cell; and

a step (b) of culturing a cell mixture obtained in the step (b) in the presence of the undifferentiated stem cell removal agent according to any one of [1] to [3].

[13] The production method according to [12], in which the undifferentiated stem cell is an induced pluripotent stem cell.

[14] The production method according to [12] or [13], in which the differentiated cell is a cardiomyocyte.

[15] A pharmaceutical composition for treating or preventing a disease which is caused by proliferation of an undifferentiated stem cell, in a subject into which a differentiated cell that is induced from the undifferentiated stem cell is transplanted, the pharmaceutical composition including the undifferentiated stem cell removal agent according to any one of [1] to [8].

[16] The pharmaceutical composition according to [15], in which the undifferentiated stem cell is an induced pluripotent stem cell.

[17] The pharmaceutical composition according to [15] or [16], in which the differentiated cell induced from the undifferentiated stem cell is a cardiomyocyte.

[18] The pharmaceutical composition according to any one of [15] to [17], including a Sendai virus vector containing a polynucleotide that encodes a GPC3 protein or a partial peptide thereof.

[19] The pharmaceutical composition according to [18], which is administered intranasally.

## Advantageous Effects of Invention

According to the present invention, a technique is provided for selectively removing only undifferentiated stem cells without affecting differentiated cells, at the time of preparation of the differentiated cells induced from the undifferentiated stem cells, and in a living body after transplantation.

## Brief Description of Drawings

FIG. 1A shows results of expression analysis of glypican-3 (GPC3) in pluripotent stem cells (ESC-1 and ESC-2, and iPSC1 and iPSC2), cardiomyocytes (iPSC1-CM) derived from pluripotent stem cells, and normal hearts (Heart and Heart-1). FIG. 1A shows the results of expression analysis of GPC3 by microarray.

FIG. 1B shows results of expression analysis of glypican-3 (GPC3) in pluripotent stem cells (ESC-1 and ESC-2, and iPSC1 and iPSC2), cardiomyocytes (iPSC1-CM) derived from pluripotent stem cells, and normal hearts (Heart and Heart-1). FIG. 1B shows the results of expression analysis of GPC3 by RNA sequence analysis.

FIG. 2A shows results of expression analysis of GPC3 in pluripotent stem cells (iPSC1 to iPSC3), cardiomyocytes (iPSC1-CM) derived from pluripotent stem cells, and normal hearts (Heart-1 and Heart-2). FIG. 2A shows the results of expression analysis by quantitative PCR.

FIG. 2B shows results of expression analysis of GPC3 in pluripotent stem cells (iPSC1 to iPSC3), cardiomyocytes (iPSC1-CM) derived from pluripotent stem cells, and normal hearts (Heart-1 and Heart-2). FIG. 2B shows the results of expression analysis by FACS.

FIG. 3A shows fluorescent immunostaining images in pluripotent stem cells and cardiomyocytes derived from pluripotent stem cells. FIG. 3A shows pluripotent stem cells.

FIG. 3B shows fluorescent immunostaining images in pluripotent stem cells and cardiomyocytes derived from pluripotent stem cells. FIG. 3B shows cardiomyocytes derived from pluripotent stem cells.

FIG. 4 shows results of IFN-γ ELISPOT assay in a case where GPC3-specific cytotoxic T cells (CTL) are co-cultured with cardiomyocytes (iPSC1-CM) differentiated from iPSC1 or iPSC1. The symbol "*" indicates that $p < 0.05$ in a t-test (the same applies in the following drawings).

FIG. 5A shows results of fluorescent observation of living cells in a case where iPSC1 or iPSC1-CM is co-cultured with GPC3-specific CTLs for 48 hours. FIG. 5A shows the results of iPSC1. In the drawing, an "inactivated CTL" is a GPC3-specific CTL inactivated by heat treatment.

FIG. 5B shows results of fluorescent observation of living cells in a case where iPSC1 or iPSC1-CM is co-cultured with GPC3-specific CTLs for 48 hours. FIG. 5B shows the results of iPSC1-CM. In the drawing, an "inactivated CTL" is a GPC3-specific CTL inactivated by heat treatment.

FIG. 6A shows results of confirmation of a knockdown effect in iPSC1 in which GPC3 expression is knocked down by siRNA (GPC3 siRNA) against GPC3. FIG. 6A shows the results of confirmation by quantitative PCR. In the drawing, a "negative siRNA" is an AllStars Negative Control siRNA manufactured by QIAGEN.

FIG. 6B shows results of confirmation of a knockdown effect in iPSC1 in which GPC3 expression is knocked down by siRNA (GPC3 siRNA) against GPC3. FIG. 6B shows the results of confirmation by FACS. In the drawing, a "negative siRNA" is an AllStars Negative Control siRNA manufactured by QIAGEN.

FIG. 7 shows results of IFN-γ ELISPOT assay in a case where iPSC1 in which GPC3 expression is knocked down by siRNA against GPC3 is co-cultured with GPC3-specific CTLs.

FIG. 8A shows results of analysis of an abundance ratio of iPSC1 in a cell population in a case where GPC3-specific CTLs are added into and co-cultured with a cell population in which iPSC1 and IPSC1-CM are mixed for 48 hours. FIG. 8A shows the results of analysis by FACS.

FIG. 8B shows results of analysis of an abundance ratio of iPSC1 in a cell population in a case where GPC3-specific CTLs are added into and co-cultured with a cell population in which iPSC1 and iPSC1-CM are mixed for 48 hours. FIG. 8B is a bar graph expressing the results of FIG. 8A in numerical form.

FIG. 9A shows fluorescent immunostaining images in a case where GPC3-specific CTLs are added into and co-cultured with a cell population in which iPSC1 and iPSC1-CM are mixed for 48 hours. FIG. 9A shows the case where GPC3-specific CTLs are not added.

FIG. 9B shows fluorescent immunostaining images in a case where GPC3-specific CTLs are added into and co-cultured with a cell population in which iPSC1 and iPSC1-CM are mixed for 48 hours. FIG. 9B shows the case where GPC3-specific CTLs are added.

## Description of Embodiments

### <<Definition and the like>>

In the present specification, the term "undifferentiated stem cell" is used as a concept encompassing pluripotent stem cells having differentiation pluripotency, and embryonic stem cells (ES cells), induced pluripotent stem cells (iPS cells), cells having differentiation pluripotency induced from these stem cells, and various somatic stem cells. The term "undifferentiated stem cell" is not particularly limited as long as it is a cell having differentiation pluripotency, and also includes unknown cells having properties equivalent to those of the ES cells and iPS cells exemplified above.

The condition that the cell is an undifferentiated stem cell can be judged from the presence or absence of properties specific to undifferentiated stem cells, and the expression of various markers specific to undifferentiated stem cells. For example, examples of properties specific to undifferentiated stem cells include a property that has the ability to self-replicate and can differentiate into a different kind of cell having a property different from that of undifferentiated stem cells. In addition, examples thereof further include teratoma-forming ability, chimeric mouse-forming ability, and the like as properties specific to undifferentiated stem cells.

Various markers specific to undifferentiated stem cells (hereinafter referred to as an "undifferentiated stem cell marker") are factors that are specifically expressed in undifferentiated stem cells, and examples thereof include Oct3/4, Nanog, Sox2, SSEA-1, SSEA-3, SSEA-4, TRA1-60, TRA1-81, Lin28, Fbx15, SSEA-5, GDF3, KLF4, CLDN6, and the like. In a case where expression of at least one of these undifferentiated stem cell markers is observed, it can be judged that this cell is an undifferentiated stem cell. One of the undifferentiated stem cell markers may be used alone, or two or more thereof may be used in combination. In one embodiment, cells expressing Oct3/4 may be judged as undifferentiated stem cells. The expression of the undifferentiated stem cell marker in a cell can be checked using a known method such as RT-PCR and microarray.

The undifferentiated stem cell may be a mammalian undifferentiated stem cell, a rodent undifferentiated stem cell, a primate undifferentiated stem cell, or a human undifferentiated stem cell. Examples thereof include human-derived undifferentiated stem cells, and more specific examples thereof include human iPS cells, human ES cells, and the like.

In the present specification, the term "differentiated cell" refers to a cell that does not have the properties of the "undifferentiated stem cells." Differentiated cells may be cells induced and differentiated from undifferentiated stem cells, but do not have differentiation pluripotency. The differentiated cells may be, for example, cells differentiated from ES cells, or cells differentiated from iPS cells. Examples of differentiated cells include, but are not limited to, cardiomyocytes; muscle cells; fibroblasts; nerve cells; immune cells such as lymphocytes; vascular cells; ocular cells such as retinal pigment epithelial cells; blood cells such as megakaryocytes and erythrocytes; and other tissue cells; and precursor cells thereof.

A glypican-3 (GPC3) protein is a GPI-anchored protein that belongs to the HSPGs family, and is considered to have a function of controlling cell division and cell proliferation. A GPC3 protein binds to CD26 and inhibits dipeptidyl peptidase activity of CD26. In addition, GPC3 knockout mice are known to become gigantic, and humans exhibit gigantism due to GPC3 mutations. GPC3 is expressed in liver tissue at a fetal stage, but is not expressed after birth. In a case where liver cell carcinoma develops, hepatoma cells re-express GPC3. In addition, GPC3 is also considered to be involved in cell proliferation and apoptosis of cancer. In the present specification, a "glypican-3 protein" or a "GPC 3 protein" refers to a protein having the above-mentioned properties.

As examples of amino acid sequences of a GPC3 protein, examples of amino acid sequences of a human GPC3 protein are shown as SEQ ID NOs: 2, 4, 6, and 8. GPC3 proteins consisting of these amino acid sequences are variants of a human GPC3 protein, and all have characteristics of GPC3 protein as described above. However, a GPC3 protein is not limited to proteins having these amino acid sequences, and includes variants and interspecific homologues thereof.

Examples of variants of the human GPC3 protein include proteins that have about 1 to 50, 1 to 30, 1 to 20, or 1 to 10 mutations as compared with the amino acid sequence shown in SEQ ID NOs: 2, 4, 6, or 8, and retain functions of the above-described GPC3 proteins. Mutations may be any of deletion, substitution, insertion, and addition of amino acid residues, or may be a combination thereof. Alternatively, other examples of variants of the human GPC3 protein include proteins that are composed of amino acid sequences having 70% or more, 80% or more, 85% or more, 90% or more, or 95% or more sequence identity (homology) with the amino acid sequence shown in SEQ ID NOs: 2, 4, 6, or 8; and that retain functions of the above-described GPC3 proteins. The sequence identity between amino acid sequences is obtained as a ratio of matched amino acids with respect to the entire amino acid sequences excluding gaps in alignment to be obtained, by allowing juxtaposition of two amino acid sequences with gaps between portions corresponding to insertions and deletions such that corresponding amino acids become the most identical. The sequence identity between amino acid sequences can be obtained by using various homology search software known in the technical field.

In addition to the amino acid sequence shown in SEQ ID NOs: 2, 4, 6, or 8, examples of amino acid sequences of the human GPC3 include amino acid sequences registered as amino acid sequences of the human GPC3 protein in sequence database such as GenBank. The human GPC3 protein also includes proteins composed of such amino acid sequences. The human GPC3 protein also includes variants of such human GPC3 proteins.

In addition, examples of interspecific homologues of the human GPC3 protein include GPC3 proteins that non-human mammals have, such as mouse, rat, cow, sheep, horse, monkey, and the like. Examples of amino acid sequences of these GPC3 proteins include amino acid sequences registered in sequence databases such as GenBank. The GPC protein also includes proteins composed of such amino acid sequences. The GPC3 protein also includes variants of such GPC3 proteins.

In the present specification, a "glypican-3 gene" or a "GPC3 gene" means a nucleic acid containing a base sequence that encodes the GPC3 protein described above. As examples of the base sequence of the GPC3 gene, examples of base sequences of polynucleotides that encode the above-described amino acid sequences shown in SEQ ID NOs: 2, 4, 6, and 8, respectively, are shown in SEQ ID NOs: 1, 3, 5, and 7 (GenBank ID: NM_001164617; NM_001164618.1; NM_001164619.1; NM_004484.3). However, base sequences of the GPC3 gene are not limited to these sequences, and may be any base sequence as long as a base sequence encodes the GPC3 protein described above.

In the present specification, a "glypican-3-expressing cell" or a "GPC3-expressing cell" means a cell having the GPC3 gene described above and expressing the GPC3 protein. The GPC3 gene that a GPC3-expressing cell has may be endogenous or exogenous, but is preferably endogenous.

<<Undifferentiated stem cell removal agent>>

In one embodiment, the present invention provides an undifferentiated stem cell removal agent, containing at least one component selected from the group consisting of the following (a) and (b):

(a) a cell that is capable of specifically inhibiting proliferation of a glypican-3-expressing cell; and
(b) a compound that is capable of specifically inhibiting proliferation of a glypican-3-expressing cell.

In addition, in one embodiment, the present invention provides an undifferentiated stem cell removal agent, containing at least one component selected from the group consisting of the following (c) and (d):

(c) a cell that is capable of inducing a specific immune response against a glypican-3-expressing cell; and
(d) a compound that is capable of inducing a specific immune response against a glypican-3-expressing cell.

As described above, GPC3 is known to be expressed in cancer, particularly liver cancer, but the inventors of the present invention have found that GPC3 is also expressed in undifferentiated stem cells. Meanwhile, differentiated cells induced from undifferentiated stem cells do not express GPC3. Accordingly, only undifferentiated stem cells can be selectively removed by performing treatment that targets GPC3 on a cell population which is differentiation-induced from undifferentiated stem cells. In addition, cells in a living body are generally differentiated cells, and thus do not express GPC3. For this reason, even in a case where a cell population in which undifferentiated stem cells remain is transplanted into a living body, it is possible to selectively remove undifferentiated stem cells in the living body by performing treatment that targets GPC3 on the living body.

In order to perform treatment that targets GPC3, the undifferentiated stem cell removal agent of the present embodiment contains at least one component selected from the group consisting of (a) a cell that is capable of specifically inhibiting proliferation of a glypican-3-expressing cell; (b) a compound that is capable of specifically inhibiting proliferation of a glypican-3-expressing cell; (c) a cell that is capable of inducing a specific immune response against a glypican-3-expressing cell; and (d) a compound that is capable of inducing a specific immune response against a glypican-3-expressing cell. The components (a) to (d) may be used alone or may be used in combination thereof. In addition, the undifferentiated cell removal agent of the present embodiment may contain any of the components (a) to (d) or a combination thereof, and other components.

Hereinafter, each of the following components (a) to (d) will be described.

< (a) Cell that is capable of specifically inhibiting proliferation of a glypican-3-expressing cell>

The component (a) is a cell that is capable of specifically inhibiting proliferation of a glypican-3-expressing cell. In the present specification, the phrase "capable of specifically inhibiting proliferation of a glypican-3-expressing cell" means an action that inhibits proliferation of a GPC3-expressing cell but does not inhibit proliferation of a GPC3-non-expressing cell. In addition, the "cell that is capable of specifically inhibiting proliferation of a glypican-3-expressing cell" means a cell having such an action.

In the cell that is capable of specifically inhibiting proliferation of a GPC3-expressing cells, the proliferation inhibition mechanism is not particularly limited as long as the cells can specifically inhibit proliferation of a GPC3-expressing cell. Typical examples of inhibition of cell proliferation include, but are not limited to, induction of cell death.

The types of cells that are capable of specifically inhibiting proliferation of a GPC3-expressing cell are not particularly limited as long as the cells can specifically inhibit proliferation of a GPC3-expressing cell. Examples of cells that are capable of specifically inhibiting proliferation of a GPC3-expressing cell include immune cells that exhibit cytotoxic activity against a GPC3-expressing cell, and the like. Preferred examples of such immune cells include cytotoxic T cells (CTLs) having cytotoxic activity specific to a GPC3-expressing cell.

CTLs having cytotoxic activity specific to a GPC3-expressing cell (hereinafter referred to as "GPC3-specific CTL") recognize a GPC3-expressing cell via a T cell receptor (TCR) and exhibit cytotoxic activity specific to a GPC3-expressing cell. Herein, the phrase "exhibit cytotoxic activity specific to GPC3" means that CTLs exhibit cytotoxic activity against a GPC3-expressing cell but do not exhibit cytotoxic activity against a GPC3-non-expressing cell.

In GPC3-expressing cells, parts of GPC3 proteins which are generated by transcription and translation from a GPC3 gene are ubiquitinated and processed by proteasome to be fragmented. The fragmented peptide derived from GPC3 protein is incorporated into the endoplasmic reticulum to form a complex with MHC Class I, is transferred to a cell surface, and is presented on the cell surface. CTLs having cytotoxic activity specific to a GPC3-expressing cell can bind, via their TCR, to a complex of the peptide derived from a GPC3 protein and major histocompatibility complex (MHC) Class I, which is presented on a cell surface. CTLs that bind to the complex release cytotoxic cytokines such as IFN-$\gamma$ and perforin against a GPC3-expressing cell, thereby damaging the GPC3-expressing cell. Such a cytotoxic action by CTLs leads to cell death of a GPC3-expressing cell. Accordingly, the GPC3-specific CTLs can be said to be CTLs having a binding ability to the complex of the peptide derived from a GPC3 protein and the MHC class I.

GPC3-specific CTLs can be obtained by, for example, a method described in PCT International Publication No. WO2004/018667 or PCT International Publication No. WO2007/018199. Specifically, a GPC3-specific CTL can be obtained by co-culturing a dendritic cell with a CD8-positive T cell in the presence of a CTL-inducing peptide derived from GPC3. Alternatively, a dendritic cell may be cultured in advance in the presence of the CTL-inducing peptide derived from GPC, and then co-cultured with a CD8-positive T cell by adding the CD8-positive T cell. Herein, the "CTL-inducing peptide derived from GPC3" means a peptide that contains a partial peptide of a GPC3 protein and has CTL-inducing activity. In addition, the "peptide having CTL-inducing activity" means a peptide is capable of forming a complex by binding to MHC Class I, and is capable of maturing a CD8-positive T cell that is co-cultured with a dendritic cell to CTL in a case where the complex is presented on the cell surface of the dendritic cell.

MHC is different from species to species. Human MHC is referred to as human leukocyte antigen (HLA). MHC is classified into Class I and Class II, and MHC Class I binds to an antigen peptide and is involved in CTL induction. A plurality of subtypes in MHC Class I are present, and peptides that can bind to each subtype differ. For example, the CTL-inducing peptides derived from GPC described in PCT International Publication No. WO2004/018667are peptides that bind to HLA-A24 (more specifically, to HLA-A*24:02), and induce a CTL having cytotoxic activity specific to an HLA-A24-positive GPC3-expressing cell. In addition, the CTL-inducing peptides derived from GPC3 described in PCT International Publication No. WO2007/018199 are peptides that bind to HLA-A2 (more specifically, to HLA-A*02:01, HLA-A*02:06, or HLA-A*02:07), and induce a CTL having cytotoxic activity specific to a HLA-A2-positive GPC3-expressing cell. Accordingly, in a case where undifferentiated stem cells are HLA-A24-positive cells, for example, GPC3-specific CTLs induced by using the peptide described in PCT International Publication No. WO2004/018667 can be used as cells of the component (a). Furthermore, in a case where undifferentiated stem cells are HLA-A2-positive cells, for example, GPC3-specific CTLs induced by using the peptide described in PCT International Publication No.

WO2007/018199 can be used as cells of the component (a). In the present specification, a "CTL having cytotoxic activity specific to an HLA-A2-positive GPC3-expressing cell" and a "CTL having cytotoxic activity specific to an HLA-A24-positive GPC3-expressing cell" are referred to as an "HLA-A2-restricted GPC3-specific CTL" and an "HLA-A24-restricted GPC3-specific CTL," respectively. The amino acid sequences of the CTL-inducing peptides derived from GPC3 described in PCT International Publication No. WO2004/018667and PCT International Publication No. WO2007/018199 are shown in SEQ ID NOs: 9 to 20 and SEQ ID NOs: 21 to 23, respectively. However, the CTL-inducing peptide derived from GPC3 which is used for inducing a GPC3-specific CTL is not limited thereto, and an appropriate peptide can be appropriately used for each type of MHC Class I that an undifferentiated stem cell has.

The CTL-inducing peptide derived from GPC3 is a fragment peptide of a GPC3 protein and is generally composed of 8 to 11 amino acid residues. However, even in a case where the peptide has 12 or more amino acids in length, the peptide may be incorporated into a dendritic cell and processed by a proteasome to become a peptide having about 8 to 11 amino acids in length, thereby forming a complex with MHC Class I. For this reason, the CTL-inducing peptide used for inducing a GPC3-specific CTL is not limited to peptides having an amino acid length of 8 to 11, and can be a fragment peptide of a GPC3 protein, which has an amino acid length of about 8 to 100, about 8 to 60, about 8 to 50, or about 8 to 30. Furthermore, the CTL-inducing peptide derived from GPC3 may be a fragment peptide of a GPC3 protein as described above, or a peptide which contains a mutation. Examples of peptides containing such a mutation include peptides having amino acid sequences in which 10% or less, preferably 5% or less, and more preferably 3% or less of amino acid residues are mutated with respect to the total number of amino acid residues constituting the fragment peptide of a GPC3 protein. The type of mutation may be any of deletion, substitution, insertion, and addition of amino acid residues, or may be a combination thereof.

Dendritic cells and CD8-positive T cells which are used for inducing GPC3-specific CTLs can be prepared from peripheral blood mononuclear cells by known methods. Peripheral blood mononuclear cells used for preparation of dendritic cells and CD8-positive T cells are preferably collected from a subject having the same MHC class I as MHC class 1 that undifferentiated stem cells to be removed have.

In addition, GPC3-specific CTLs usable as the component (a) are not limited to CTLs induced by using the CTL-inducing peptide derived from GPC3 as described above, and may be CTLs genetically transformed with a gene that encodes a TCR having binding ability to a complex of the CTL-inducing peptide derived from GPC3 and MHC Class 1. Such a gene that encodes the TCR can be obtained from a GPC3-specific CTL induced as described above by using known methods. Genetic transformation can be performed by using known methods.

In addition, GPC3-specific CTLs may be CTLs modified to express a chimeric antigen receptor (hereinafter referred to as a "GPC3-specific chimeric antigen receptor") having binding ability with respect to a GPC3 protein. Such CTLs can be obtained by genetically transforming CTLs with a gene that encodes the GPC3-specific chimeric antigen receptor. A gene that encodes the GPC3-specific chimeric antigen receptor can be created by a known method by using a base sequence that encodes a single chain antibody (scFv) having binding ability to a GPC3 protein. Examples of creation methods include a method in which genes that encode scFV having binding ability to a GPC3 protein, an intracellular signal transduction domain of T cell co-stimulators such as CD28, and an intracellular signal transduction domain of CD3 ζ-chain are connected in the above order; and the like.

In a case where the undifferentiated stem cell removal agent of the present embodiment includes the component (a), the undifferentiated stem cell removal agent may include only the component (a), or may include any one or more of the components (b) to (d) which will be described later at the same time. In addition, the undifferentiated stem cell removal agent may contain components other than the components (a) to (d). Examples of other components include components of a culture medium generally used for culturing CTLs, and the like. Examples of culture media generally used for culturing CTLs include AIM-V culture medium, RPMI 1640, DMEM, X-VIVO5, and the like. In addition, examples of components that can be used for culturing CTLs include human AB serum, serum of non-human animals such as cow, cytokines such as IL-2, IL-7, and IL-15, and the like.

In a case where the undifferentiated stem cell removal agent of the present embodiment includes the component (a), the undifferentiated stem cell removal agent can adopt a form such as a culture of cells which are the component (a); a cell suspension obtained by suspending the cells which are the component (a) in a suitable carrier such as a culture medium, physiological saline, or phosphate buffer; a cell pellet obtained by recovering the cells which are the component (a) by centrifugation or the like; a cryopreserved cells obtained by cryopreserving the cells which are the component (a); and the like.

< (b) Compound that is capable of specifically inhibiting proliferation of a glypican-3-expressing cell>

The component (b) is a compound that is capable of specifically inhibiting proliferation of a glypican-3-expressing cell. As described in the case of the component (a) described above, the phrase "capable of specifically inhibiting proliferation of a glypican-3-expressing cell" means an action that inhibits proliferation of a GPC3-expressing cell but does not inhibit proliferation of a GPC3-non-expressing cell. The "compound that is capable of specifically inhibiting proliferation of a glypican-3-expressing cell" means a compound having such an action.

In the compound that is capable of specifically inhibiting proliferation of a GPC3-expressing cell, the proliferation inhibition mechanism is not particularly limited as long as the compound can specifically inhibit proliferation of a GPC3-expressing cell. Typical examples of inhibition of cell proliferation include, but are not limited to, induction of cell death.

The types of the compound that is capable of specifically inhibiting proliferation of a GPC3-expressing cell are not particularly limited as long as the compound can specifically inhibit proliferation of a GPC3-expressing cell. Examples of compounds that are capable of specifically inhibiting proliferation of a GPC3-expressing cell include an antibody having binding ability to a GPC3 protein, a double-stranded small interfering nucleic acid against GPC3, an antisense nucleic acid against GPC3, and the like.

Several antibodies (hereinafter referred to as an "anti-GPC3 antibody") having binding ability to a GPC3 protein have been reported (for example, in PTL 5) for use in cancer treatment. Because these antibodies have a proliferation suppression effect on cancer cells expressing GPC3, development of these antibodies as cancer therapeutic agents is proceeding. The anti-GPC3 antibody has been confirmed to have a proliferation suppression effect on cancer cells that are GPC3-expressing cells, and is also effective in suppressing proliferation of undifferentiated stem cells that are GPC3-expressing cells. Therefore, the anti-GPC3 antibody can be exemplified as a preferred example of the component (b) for the undifferentiated stem cell removal agent of the present embodiment.

As the anti-GPC3 antibody, it is possible to use a known antibody that has been reported to be used in cancer treatment, or a new antibody may be created by a known method of creating an antibody. Examples of methods of creating an antibody include a method of immunizing an animal with a GPC3 protein, a phage display method, and the like.

The anti-GPC3 antibody may be a polyclonal antibody or a monoclonal antibody. An organism from which the anti-GPC3 antibody is derived is not particularly limited. For example, it is possible to use antibodies of mammals such as humans, mice, rats, rabbits, dogs, and monkeys; antibodies of birds such as chickens; and the like. In addition, as long as binding ability to a GPC3 protein is maintained, an antibody fragment or a modified antibody may be used. Examples of such antibody fragments include scFv, Fab, F(ab')2, Fv, and the like. In addition, examples of modified antibodies include a chimeric antibody. Specific examples of chimeric antibodies include an antibody in which a variable region of a heterologous antibody is transplanted into a variable region of a human antibody (that is, an antibody containing a constant region of a human antibody and a variable region of a heterologous antibody), a humanized antibody in which complementarity determining regions (CDRs) of a heterologous antibody are transplanted into CDRs of a human antibody (that is, an antibody in which CDRs of a human antibody are substituted with CDRs of a heterologous antibody), and the like. In addition, the anti-GPC3 antibody may also be a multispecific antibody capable of binding to two or more antigenic epitopes. The anti-GPC3 antibody may be an antibody of any class (IgA, IgD, IgE, IgG, IgM, and the like).

In addition, the anti-GPC3 antibody may be an antibody to which a medicine having cell cytotoxicity, and the like binds. Examples of such a medicine include a medicine used as an anticancer agent. More specific examples thereof include alkylating agents such as ifosfamide and cyclophosphamide; antimetabolites such as enositabine, capecitabine, and gemcitane; plant alkaloids such as doxitaxel and paclitaxel; anti-cancer antibiotics such as actinomycin D and idarubicin; platinum preparations such as oxaliplatin and cisplatin; and the like. In a case where an anti-GPC3 antibody to which such a medicine binds is used, the anti-GPC3 antibody binds to a GPC3-expressing cell, and the medicine can be efficiently acted on the GPC3-expressing cell.

The double-stranded small interfering nucleic acid against GPC3 is a double-stranded nucleic acid that contains a base sequence complementary to a part of a sense strand of a GPC3 gene and a base sequence complementary to the base sequence, and has a function of inhibiting GPC3 expression. In a case where the double-stranded small nucleic acid against GPC3 is incorporated into a cell, the double-stranded small nucleic acid is incorporated into an RNA induced silencing complex (RISC) to become single-stranded. The single-stranded nucleic acid incorporated into the RISC functions as a guide for guiding the RISC to an mRNA of GPC3. The RISC that binds to the mRNA of GPC3 by the single-stranded nucleic acid cleaves the mRNA of GPC3 by endonuclease activity of a protein called Argonaute, which constitutes the RISC. As a result, expression of GPC3 is inhibited.

The double-stranded small interfering nucleic acid against GPC3 may be any of small interfering RNAs (siRNA; double-stranded ribonucleic acid (dsRNA) or small hairpin RNA (shRNA)) and small interfering DNAs/RNAs (siD/R-NA; double-stranded chimera (dsD/R-NA) of DNA and RNA or small hairpin chimera (shD/R-NA) of DNA and RNA).

It has been reported that knockdown of GPC3 by siRNA causes suppression of proliferation of a GPC3-expressing cell, in hepatoma cells and the like (for example, Sun CK et al., Neoplasia. 2011 Aug; 13(8):735-47). Accordingly, the double-stranded small interfering nucleic acid having the function of inhibiting GPC3 expression is also effective in suppressing proliferation of undifferentiated stem cells which are GPC3-expressing cells.

In the double-stranded small interfering nucleic acid against GPC3, the double-stranded portion is composed of a partial base sequence (target sequence) of a sense strand of a GPC3 gene and a base sequence complementary to the target sequence. The selection of a target sequence can be performed by a method generally used for selection of a target sequence of siRNA (for example, Tuschl et al. Genes Cev 1999, 13(24):3191-7). A target sequence is not particularly limited as long as the target sequence expresses ability to inhibit GPC3 expression. Examples of target sequences include a target sequence of siRNA used in the examples of the present specification (SEQ ID NO: 28: 5'-GGAGGCUCUGGUGAUGGAAUGAUAA-3'), but are not limited thereto. In a case where the double-stranded small interfering nucleic acid is RNA, a thymidine residue contained in a target sequence is replaced with a uracil residue in the double-stranded small interfering nucleic acid.

In the double-stranded small interfering nucleic acid, a target sequence is generally 19 to 25 base pairs, preferably 21 to 23 base pairs, but a length thereof is not particularly limited as long as the double-stranded small interfering nucleic acid has ability to inhibit GPC3 expression. In addition, the double-stranded small interfering nucleic acid may have an overhang sequence of about 2 to 5 bases at the 3' end. An overhang sequence is generally composed of, but is not limited to, a uracil residue or a thymidine residue.

An antisense nucleic acid against GPC3 is a single-stranded nucleic acid having a sequence complementary to at least a part of a sense strand of a GPC3 gene, and can bind to mRNA transcribed from the GPC3 gene. When the antisense nucleic acid against GPC3 binds to mRNA of GPC3, translation of a GPC3 protein is inhibited, and degradation of mRNA is promoted. As a result, expression of GPC3 is inhibited.

The antisense nucleic acid against GPC3 has ability to inhibit GPC3 expression as well as the double-stranded small interfering nucleic acid against GPC3, and thus is also effective for suppressing proliferation of undifferentiated stem cells that are GPC3-expressing cells.

The antisense nucleic acid against GPC3 has a base sequence complementary to a part or all of a sense strand of a GPC3 gene, but a complementary base sequence is preferably 12 residues or more, and generally is about 12 to 50 residues. The complementary base sequence is not particularly limited as long as a complementary base sequence is a base sequence complementary to at least a part of a sense strand of a GPC3 gene. In addition, as long as the antisense nucleic acid has binding ability to mRNA of GPC3, the complementary base sequence does not have to be a completely complementary base sequence, and may contain one to several mismatched residues. The antisense nucleic acid may be RNA or DNA, but is preferably RNA.

The above-mentioned double-stranded small interfering nucleic acid and the antisense nucleic acid may be not only natural nucleic acids but also modified nucleic acids in which a base moiety, a phosphate group, and/or a sugar moiety have been modified.

In a case where the undifferentiated stem cell removal agent of the present embodiment includes the component (b), the component (b) may be one of the components as described above, or a combination of two or more of the components as described above. In addition, the undifferentiated stem cell removal agent may include only the component (b), or may include any one or more of the component (a), and the component (c) and component (d) which will be described later at the same time. In addition, the undifferentiated stem cell removal agent may contain a component other than the components (a) to (d). The other components are not particularly limited, and examples thereof include pharmaceutically acceptable carriers, transfection accelerators, buffers, excipients, stabilizers, antioxidants, osmotic pressure regulators, pH adjusters, chelating agents, and the like.

In a case where the undifferentiated stem cell removal agent of the present embodiment includes the component (b), dosage forms of the undifferentiated stem cell removal agent are not particularly limited and may be in various forms such as a liquid matter, a powdery substance, a granular substance, a gel-like substance, and a solid matter. In addition, the undifferentiated stem cell removal agent may also be in an emulsion form in which the compound that is the component (b) is encapsulated in a micelle; or in a liposome form in which the compound that is the component (b) is encapsulated in a liposome.

< (c) Cell that is capable of inducing a specific immune response against a glypican-3-expressing cell>

The component (c) is a cell that is capable of inducing a specific immune response against a glypican-3-expressing cell. In the present specification, the phrase "capable of inducing a specific immune response against a glypican-3-expressing cell" means an action in which, in a living body, an immune response against a GPC3-expressing cell is induced, but an immune response against a GPC3-non-expressing cell is not induced. The "cell that is capable of inducing a specific immune response against a glypican-3-expressing cell" is a cell having such an action.

In the cell that is capable of inducing a specific immune response against GPC3-expressing cells, the mechanism of inducing an immune response is not particularly limited as long as the cell can induce a specific immune response against a GPC3-expressing cell in a living body. In addition, the type of immune response to be induced is not particularly limited, and may be cell-mediated immunity or humoral immunity.

The type of cell that is capable of inducing a specific immune response against a GPC3-expressing cell are not particularly limited as long as the cell can induce a specific immune response against a GPC3-expressing cell. Examples of cells that are capable of inducing a specific immune response against a GPC3-expressing cell include immune cells that promote an immune response against a GPC3-expressing cell, and the like. Preferred examples of such immune cells include dendritic cells in which a partial peptide of a GPC3 protein is presented on a cell surface, and the like.

The "dendritic cell which presents a partial peptide of a GPC3 protein on the cell surface" can induce GPC3-specific CTLs. Herein, the "dendritic cell which presents a partial peptide of a GPC3 protein on the cell surface" means a dendritic cell in which a partial peptide of a GPC3 protein binds to MHC Class I existing on the cell surface, and have ability to induce a GPC3-specific CTL. Herein, the sentence the "dendritic cell has ability to induce a GPC3-specific CTL" means that, in a case where the dendritic cell and a CD8-positive T cell are co-cultured, the dendritic cell can mature the CD8-positive T cell to a GPC3-specific CTL. Accordingly, in a case where a dendritic cell (hereinafter referred to as a "GPC3 peptide-presenting dendritic cell") that presents a partial peptide of a GPC3 protein on the cell surface is administered to a living body, a GPC3-specific CTL is induced in a living body. In other words, a specific immune response against a GPC3-expressing cell is induced.

The GPC3-presenting dendritic cell can be obtained by, for example, methods described in PCT International Publication No. WO2004/018667or PCT International Publication No. WO2007/018199. Specifically, it is possible to obtain the GPC3 peptide-presenting dendritic cell by culturing a dendritic cell in the presence of a CTL-inducing peptide derived from GPC3. As the CTL-inducing peptide, it is possible to use peptides described in the section of the component (a) described above.

Dendritic cells used to create the GPC3 peptide-presenting dendritic cells can be prepared from peripheral blood mononuclear cells by known methods. In a case where the undifferentiated stem cell removal agent of the present embodiment is administered to a living body, peripheral blood mononuclear cells used in preparation of dendritic cells are preferably collected from a subject having the same MHC class I as MHC class 1 that a subject to be administered has. More preferably, peripheral blood mononuclear cells used in preparation of dendritic cells are peripheral blood mononuclear cells collected from a subject to which the undifferentiated stem cell removal agent is administered.

In a case where the undifferentiated stem cell removal agent of the present embodiment includes the component (c), the undifferentiated stem cell removal agent may include only the component (c), or may include any one or more of the component (a), the component (b), and the component (d) which will be described later at the same time. In addition, the undifferentiated stem cell removal agent may contain a component other than the components (a) to (d). Examples of other components include components of a culture medium generally used for culturing dendritic cells, and the like. Examples of culture media generally used for culturing dendritic cells include AIM-V culture medium, RPMI 1640, DMEM, X-VIVO5, and the like. In addition, examples of components that can be used for culturing dendritic cells include human AB serum; serum of non-human animals such as cow; cytokines such as IL-4, 1L-6, 1L-1$\beta$, GM-CSF, SCF, and TNF-$\alpha$; prostaglandins such as PGE2; and the like.

In a case where the undifferentiated stem cell removal agent of the present embodiment includes the component (c), the undifferentiated stem cell removal agent can adopt a form such as a culture of cells which are the component (c); a cell suspension obtained by suspending the cells which are the component (c) in a suitable carrier such as a culture medium, physiological saline, or phosphate buffer; a cell pellet obtained by recovering the cells which are the component (c) by centrifugation or the like; a cryopreserved cells obtained by cryopreserving the cells which are the component (c); and the like.

< (d) Compound that is capable of inducing a specific immune response against a glypican-3-expressing cell>

The component (d) is a compound that is capable of inducing a specific immune response against a glypican-3-expressing cell. In the present specification, as described in the component (c), the phrase "capable of inducing a specific immune response against a glypican-3-expressing cell" means an action in which, in a living body, an immune response against a GPC3-expressing cell is induced, but an immune response against a GPC3-non-expressing cell is not induced. The "compound that is capable of inducing a specific immune response against a glypican-3-expressing cell" is a compound having such an action.

In the compound that is capable of inducing a specific immune response against GPC3-expressing cells, the mechanism of inducing an immune response is not particularly limited as long as the compound can induce a specific immune response against a GPC3-expressing cell in a living body. In addition, the type of immune response to be induced is not particularly limited, and may be cell-mediated immunity or humoral immunity.

The type of compound that is capable of inducing a specific immune response against a GPC3-expressing cell is not particularly limited as long as the compound can induce a specific immune response against a GPC3-expressing cell. Examples of compounds that are capable of inducing a specific immune response against a GPC3-expressing cell include a GPC3 protein or a partial peptide thereof, a polynucleotide that encodes the protein or peptide, a vector that contains the polynucleotide, and the like.

A GPC3 protein or a partial peptide thereof induces various immune responses in a living body in a case where the GPC3 protein or the partial peptide thereof is administered to the living body. For example, generation of an anti-GPC3 antibody, generation of GPC3 peptide-presenting dendritic cells, induction of a GPC3-specific CTL, and the like occur. Anti-GP3 antibodies and GPC3 specific CTLs generated due to such an immune response have an action of inhibiting proliferation of a GPC3-expressing cell. In addition, a GPC3 peptide-presenting dendritic cell has an action of inducing a GPC3-specific CTL.

In the undifferentiated stem cell removal agent of the present embodiment, a GPC3 protein used as the component (d) is not particularly limited, but is preferably a GPC3 protein of the same species as a subject to which the undifferentiated stem cell removal agent is administered. In this case, an unexpected immune response can be avoided. Accordingly, an immune response against a GPC3-expressing cell in a living body can be efficiently provoked. For example, in a case of an undifferentiated stem cell removal agent to be administered to humans, it is preferable to use a human GPC3 protein or a partial peptide thereof.

As long as a GPC3 protein can induce an immune response against a GPC3-expressing cell, a GPC3 protein is not required to be a wild type and may have a mutation. For example, it is possible to use, for the undifferentiated stem cell removal agent of the present embodiment, proteins that have about 1 to 50, 1 to 30, 1 to 20, or 1 to 10 mutations as compared with an amino acid sequence of a wild-type GPC3 protein, and can induce a specific immune response against a GPC3-expressing cell. Mutations may be any of deletion, substitution, insertion, and addition of amino acid residues, or may be a combination thereof. Alternatively, it is possible to use, for the undifferentiated stem cell removal agent of the present embodiment, a protein that is composed of an amino acid sequence having 70% or more, 80% or more, 85% or more, 90% or more, or 95% or more sequence identity (homology) with an amino acid sequence of a wild-type GPC3 protein, and that can induce a specific immune response against a GPC3-expressing cell.

In a case of using a partial peptide of a GPC3 protein as the component (d), a length thereof is not particularly limited as long as the partial peptide can induce an immune response against a GPC3-expressing cell. Because a CTL-inducing peptide is generally 8 to 12 amino acids in length, the CTL-inducing peptide is preferably 8 amino acids or more in order to induce a GPC3-specific CTLs. For example, a partial peptide of a GPC3 protein may have 8 amino acids or more, 15 amino acids or more, 20 amino acids or more, 30 amino acids or more, 50 amino acids or more, and the like in length.

The sequence of a partial peptide of a GPC3 protein is not particularly limited as long as the partial peptide can induce an immune response against a GPC3-expressing cell. As an example, in a case of administration to an HLA-A24-positive subject, a peptide described in PCT International Publication No. WO2004/018667(SEQ ID NOs: 9 to 20) may be used, and in a case of administration to an HLA-A2-positive subject, a peptide described in PCT International Publication No. WO2007/018199 (SEQ ID NOs: 21 to 23) may be used. In addition, a partial peptide of a GPC3 protein may contain any two or more amino acid sequences among amino acid sequences shown in SEQ ID NOs: 9 to 23. However, sequences of a partial peptide of a GPC3 protein are not limited thereto.

In addition, a GPC3 protein or a partial peptide thereof may be in a form of a pharmaceutically acceptable salt. Preferred examples of salts include, but are not limited to, salts with alkali metals, salts with alkaline earth metals, salts with organic bases, salts with amines, salts with organic acids (for example, acetic acid, formic acid, propionic acid, fumaric acid, maleic acid, succinic acid, tartaric acid, citric acid, malic acid, oxalic acid, benzoic acid, methanesulfonic acid, and the like), and salts with inorganic acids (for example, hydrochloric acid, phosphoric acid, hydrobromic acid, sulfuric acid, nitric acid, and the like). In the present specification, the term "pharmaceutically acceptable salt" refers to a salt in which pharmaceutical efficacy of the compound is retained.

In the undifferentiated stem cell removal agent of the present embodiment, a GPC3 protein or a partial peptide thereof as described above may be included in a form of a polynucleotide that encodes the GPC3 protein or partial peptide thereof, or in a form of a vector that contains the polynucleotide. The vector is preferably an expression vector capable of expressing a GPC3 protein or a partial peptide thereof. Herein, the "expression vector capable of expressing a GPC3 protein or a partial peptide thereof" means a vector by which the GPC3 protein or partial peptide thereof is expressed in a cell in a case where the vector is introduced into the cell.

The vector (hereinafter referred to as a "GPC3 vector") that contains the polynucleotide (hereinafter referred to as a "GPC3" polynucleotide) which encodes a GPC3 protein or a partial peptide thereof may contain, in addition to the GPC3 polynucleotide, a regulatory sequence such as a promoter to regulate expression of the GPC3 polynucleotide. Examples of promoters include an SR$\alpha$ promoter, an SV40 early promoter, LTR of retrovirus, a cytomegalovirus (CMV) promoter, a rous sarcoma virus (RSV) promoter, a herpes simplex virus thymidine kinase (HSV-TK) promoter, an EF1$\alpha$ promoter, a metallothionein promoter, a heat shock promoter, and the like. In addition, an enhancer of an IE gene of human CMV may be used together with a promoter. Examples thereof include a CAG promoter (containing a cytomegalovirus enhancer, a chicken $\beta$-actin promoter, and a poly A signal site of a $\beta$-globin gene).

In addition to the promoter, the GPC3 vector may have sequences of enhancers, poly A addition signals, marker genes, replication origins, genes that encode proteins that bind to replication origins to control replication, and the like.

Regarding the GPC3 vector, the type of the vector is not particularly limited. Examples of vectors that can be used for the GPC3 vector include virus vectors, plasmid vectors, episomal vectors, artificial chromosome vectors, and the like.

Examples of virus vectors include a Sendai virus vector, a retrovirus (including lentivirus) vector, an adenovirus vector, an adeno-associated virus vector, a herpes virus vector, a vaccinia virus vector, a pox virus vector, a polio virus vector, a sindbis virus vector, a rhabdovirus vector, a paramyxovirus vector, an orthomyxovirus vector, and the like.

As an example, the GPC3 vector can be a vector in which a GPC3 polynucleotide is inserted into a Sendai virus vector. A Sendai virus is a virus classified into Parainfluenza virus type 1 that belongs to the genus Paramyxovirus of the family Paramyxoviridae, and has (-) strand RNA genome. A Sendai virus is utilized as an expression vector, and a virus reconstitution method has been developed (PCT International Publication No. WO97/16338, and PCT International Publication No. WO97/16339).

In a case where a Sendai vector is used as a GPC3 vector, a complex including all genes of M, F, and HN genes involved in transmission capacity may be used in a Sendai vector. In addition, a complex in which these genes involved in transmission capacity are deleted or functionally inactivated may be used. Furthermore, a complex may contain, for example, an adhesion factor, a ligand, a receptor, and the like, which can adhere to specific cells on a surface of an envelope. Furthermore, for example, a complex may be a complex in which a gene involved in immunogenicity is inactivated, or a complex in which some genes are modified to enhance transcription efficiency and replication efficiency of RNA.

In the construction of a GPC3 vector, a GPC3 polynucleotide can be inserted at an appropriate site of RNA contained in a Sendai virus vector. For example, a GPC3 polynucleotide can be inserted between an R1 sequence and an R2 sequence, as a sequence having a base number that is a multiple of 6. An expression level of GPC3 polynucleotide can be regulated by a position of gene insertion and an RNA base sequence before and after the gene. For example, in a Sendai virus RNA, it is known that an expression level of an inserted gene increases as an insertion position is closer to a NP gene.

A Sendai virus vector can be reconstructed by using a suitable host cell. As a reconstruction method, for example, it is possible to use methods described in PCT International Publication No. WO97/16338 and PCT International Publication No. WO97/16339. For example, it is possible to reconstruct a complex of Sendai virus by using cultured cells such as CV1 cells and LLCMK2 cells which are derived from a monkey kidney, and BHK cells derived from a hamster kidney. A large amount of complexes can be obtained by amplifying reconstructed complexes by using embryonated chicken eggs. The production of a vector using a chicken egg can be carried out by a known method (Edited by Nakanishi et al. (1993), "Advanced Technology Protocol III for Neuroscience Research, Molecular Neuronal Cell Physiology," Welfare Company, Osaka, pp. 153-172). Specifically, for example, a fertilized egg is put into a culture instrument, and cultured at 37°C to 38°C for 9 to 12 days to grow an embryo. A Sendai virus vector is inoculated into the chorioallantoic cavity. The Egg is cultured for several days so that a virus vector proliferates. Condition such as culture period may vary depending on recombinant Sendai viruses used. Thereafter, a chorioallantoic liquid containing the virus is recovered. In addition, separation and purification of a Sendai virus from the chorioallantoic liquid can be performed according to a general method (Makoto Tashiro, "Virus Experimental Protocol," Nagai, supervised by Ishihama, Medical View Co., Ltd, pp. 68-73 (1995)).

A Sendai virus vector containing a GPC3 polynucleotide can be used as a live vaccine. In the present specification, the term "live vaccine" refers to a vaccine having an action in which a Sendai virus vector proliferates in an individual to which the vaccine is administered, thereby imparting specific immunity against a GPC3-expressing cell to the individual. A subject to be inoculated with such a live vaccine is not limited. Examples thereof include all animals such as humans, birds, pigs, horses, and cows. In addition, by using a Sendai virus vector in which the above-mentioned transmission capacity is lost, it is possible to produce a live vaccine which is a vaccine in which a vector does not transmit. The undifferentiated stem cell removal agent of the present embodiment using a Sendai virus vector can be used as a nasal vaccine, as an example.

Examples of plasmid vectors include plasmid vectors for animal cell expression, such as pA1-11, pXT1, pRc/CMV, pRc/RSV, and pcDNAI/Neo.

An episomal vector is a vector capable of autonomous replication outside the chromosome. Specific means for using the episomal vectors is known (Yu et al., Science, 324, 797-801 (2009)). Examples of episomal vectors include a vector containing, as a vector element, a sequence required for autonomous replication, which is derived from EBV, SV40, and the like. Specific examples of vector elements required for autonomous replication include a gene encoding a replication origin and a protein that binds to the replication origin to control replication. In a case of EBV, examples of vector elements include a replication origin oriP and an EBNA-1 gene. In a case of SV40, examples of vector elements include a replication origin ori and an SV40LT gene.

Examples of artificial chromosome vectors include a Yeast artificial chromosome (YAC) vector, a Bacterial artificial chromosome (BAC) vector, a P1-derived artificial chromosome (PAC) vector, and the like.

In a case where the undifferentiated stem cell removal agent of the present embodiment includes the component (d), the component (d) may be one of the components as described above, or a combination of two or more of the components as described above. In addition, the undifferentiated stem cell removal agent may include only the component (d), or may include any one or more of the component (a), the component (b), and the component (c) at the same time. In addition, the undifferentiated stem cell removal agent may contain a component other than the components (a) to (d). The other components are not particularly limited, and examples thereof include pharmaceutically acceptable carriers, transfection accelerators, buffers, excipients, stabilizers, antioxidants, osmotic pressure regulators, pH adjusters, chelating agents, and the like.

In a case where the undifferentiated stem cell removal agent of the present embodiment includes the component (d), dosage forms of the undifferentiated stem cell removal agent are not particularly limited and may be in various forms such as a liquid matter, a powdery substance, a granular substance, a gel-like substance, and a solid matter. In addition, the undifferentiated stem cell removal agent may also be in an emulsion form in which the compound that is the component (d) is encapsulated in a micelle; or in a liposome form in which the compound that is the component (d) is encapsulated in a liposome.

In the undifferentiated stem cell removal agent of the present embodiment, a content of the components (a) to (d) is not particularly limited. Examples of contents of the components (a) to (d) include 0.01 to 900 μg/mg, 0.1 to 500 μg/mg, 0.5 to 300 μg/mg, 1 to 200 μg/mg, and the like.

The undifferentiated stem cell removal agent of the present embodiment can be used in a method for removing an undifferentiated stem cell, a production method of a differentiated cell, a pharmaceutical composition for treating or preventing a disease caused by proliferation of an undifferentiated stem cell, a method for treating or preventing a disease caused by proliferation of an undifferentiated stem cell, and the like, which will be described later.

An undifferentiated stem cell to be removed by the undifferentiated stem cell removal agent of the present embodiment is not particularly limited, and is preferably iPS cell or ES cell and more preferably iPS cell.

In another aspect, the present invention provides use of at least one component selected from the group consisting of the following (a) to (d) for the manufacture of an undifferentiated stem cell removal agent:

(a) a cell that is capable of specifically inhibiting proliferation of a glypican-3-expressing cell;
(b) a compound that is capable of specifically inhibiting proliferation of a glypican-3-expressing cell;
(c) a cell that is capable of inducing a specific immune response against a glypican-3-expressing cell; and
(d) a compound that is capable of inducing a specific immune response against a glypican-3-expressing cell.

<<Kit>>

In one embodiment, the present invention provides a kit including the undifferentiated stem cell removal agent of the above embodiment.

In addition to the undifferentiated stem cell removal agent of the above embodiment, the kit of the present embodiment further may include reagents for inducing a differentiated cell from an undifferentiated stem cell, culture media, cell culture instrument, instruction manual, and the like. The reagents for inducing a differentiated cell can be appropriately selected depending on a differentiated cell to be induced. Examples of reagents for inducing a cardiomyocyte include, but are not limited to, chromosomal DNA demethylating agents such as demethylase, 5-azacytidine, and DMSO; cytokines such as PDGF, fibroblast growth factor 8 (FGF-8), endothelin 1 (ET1), midkine and bone morphogenetic protein 4 (BMP-4), and G-CSF; adhesive molecules such as gelatin, laminin, collagen, and fibronectin; vitamins such as retinoic acid; transcription factors such as Nkx2.5/Csx, GATA4, MEF-2A, MEF-2B, MEF-2C, MEF-2D, dHAND, eHAND, TEF-1, TEF-3, TEF-5, and MesP1; an extracellular matrix derived from cardiomyocytes; BMP antagonists such as noggin, chordin, fetuin, follistatin, sclerostin, Dan, Cerberus, gremlin, and dante; and the like. In addition, examples of the media include, but are not limited to, Dulbecco's Modified Eagle's Medium culture solution (DMEM), MEM culture solution (a-MEM, MEM [Hank's Bss], and the like), RPMI culture solution (RPMI 1640 and the like), F12 culture solution, StemPre34, mTeSRI, and the like. Furthermore, examples of the cell culture instrument and the like include a cell culture plate and the like, but are not limited thereto.

The kit of the present embodiment can be suitably used for the method for removing an undifferentiated stem cell, which is to be described later, and can further include instructions for explaining the method for removing an undifferentiated stem cell, and the like. The method for removing an undifferentiated stem cell can be easily carried out in a short time by kitting reagents used for the method for removing an undifferentiated stem cell.

<<Method for removing an undifferentiated stem cell>>

In one embodiment, the present invention provides the method for removing an undifferentiated stem cell, which includes culturing a cell mixture that contains an undifferentiated stem cell and a differentiated cell in the presence of the undifferentiated stem cell removal agent.

Among the undifferentiated stem cell removal agents of the above-described embodiment, the undifferentiated stem cell removal agent used in the method for removing an undifferentiated stem cell of the present embodiment is the undifferentiated stem cell removal agent containing at least one component selected from the group consisting of the following (a) and (b):

(a) a cell that is capable of specifically inhibiting proliferation of a glypican-3-expressing cell; and

(b) a compound that is capable of specifically inhibiting proliferation of a glypican-3-expressing cell.

Accordingly, the method for removing an undifferentiated stem cell of the present embodiment can also be said to be a method including culturing a cell mixture that contains an undifferentiated stem cell and a differentiated cell in the presence of at least one component selected from the group consisting of the above-mentioned (a) and (b). The components (a) and (b) are the same as those described in the aforementioned <<Undifferentiated stem cell removal agent>>.

In the present specification, the term "cell mixture" means a cell population including two or more cells. In addition to two or more cells, the "cell mixture" may include components such as a culture medium. The "cell mixture containing an undifferentiated stem cell and a differentiated cell" includes one or more undifferentiated stem cells and one or more differentiated cells and may optionally include components of the culture medium and the like. A form of the cell mixture containing the undifferentiated stem cell and the differentiated cell is not particularly limited, and may be in an aggregated state, a dispersed state, a state of being adhered to a culture container, a state of being adhered to an adhesion factor such as an extracellular matrix, a sheet state, a lumpy state, a colony form, an embryoid body form, a cell clump form, a tissue form, an organ form, and the like.

When an undifferentiated stem cell is subjected to differentiation induction treatment, a differentiated cell is induced from an undifferentiated stem cell. However, in vitro, it is difficult to induce all of undifferentiated stem cells into differentiated cells. For this reason, in general, undifferentiated stem cells remain. Therefore, a cell mixture of an undifferentiated stem cell and a differentiated cell is formed. According to the method of the present embodiment, only an undifferentiated stem cell can be selectively removed from such a cell mixture of an undifferentiated stem cell and a differentiated cell. In the method of the present embodiment, the cell mixture containing an undifferentiated stem cell and a differentiated cell may be a mixture obtainable by mixing an undifferentiated stem cell and a differentiated cell.

In the method of the present embodiment, an undifferentiated stem cell is not particularly limited, and is preferably iPS cell or ES cell and more preferably iPS cell.

In the method of the present embodiment, a differentiated cell is not particularly limited, and is preferably a cell differentiation-induced from an undifferentiated stem cell of the same kind as the undifferentiated stem cell that coexist. Several methods for differentiation induction from an undifferentiated stem cell to a differentiated cell have been reported so far, and a differentiated cell can be induced using these known methods.

Undifferentiated stem cells express GPC3, but differentiated cells do not express GPC3. The method of the present embodiment pays attention to this point to perform selective removal of undifferentiated stem cells by performing cell treatment that targets the GPC3-expressing cells. Therefore, in the method of the present embodiment, differentiated cells are cells not expressing GPC3. Herein, the phrase "not expressing GPC3" means that an expression level of GPC3 is 1/5 or less, is preferably 1/10 or less, is more preferably 1/20 or less, and is even more preferably 1/30 or less, as compared with an expression level of GPC3 in an undifferentiated stem cell. An expression level of GPC3 can be checked by known methods such as flow cytometry, quantitative PCR, microarray, northern hybridization, immunohistochemical staining using an anti-GPC3 antibody, western blotting, and the like.

In the method of the present embodiment, the type of the differentiated cell is not particularly limited as long as a differentiated cell is a cell not expressing GPC3. Examples thereof include cardiomyocytes, muscle cells, fibroblasts, nerve cells, immune cells such as lymphocytes; vascular cells; ocular cells such as retinal pigment epithelial cells; blood cells such as megakaryocytes and erythrocytes; and other tissue cells; and precursor cells thereof. Preferred examples of differentiated cells include cardiomyocytes.

In a case where cardiomyocytes are prepared from pluripotent stem cells, it is perceived that the cells differentiate into cardiomyocytes via undifferentiated mesoderm, cardiac mesoderm (or predeterminate cardiomyocyte) as differentiation into cardiomyocytes progresses. Here, undifferentiated mesoderm is a stage in which expression of Brachyury protein specific to undifferentiated mesoderm is recognized. Meanwhile, cardiac mesoderm (or a predeterminate cardiomyocyte) means a cell in which expression of a protein specific to undifferentiated mesoderm, such as Brachyury, is recognized, but expression of a protein specific to cardiomyocytes, such as Nkx2.5 and actinin is not recognized in the same cell; and also means a cell that has the ability to differentiate exclusively into cardiomyocytes without requiring the addition of a new substance to a culture solution. In addition, cardiomyocyte means a cell performing autonomous pulsation. Cardiomyocytes express markers such as troponin, Nkx2.5, GATA4, and actinin. In the present specification, the term "cardiomyocyte" is used as a concept encompassing cardiomyocytes and cardiac mesoderm (or a predeterminate cardiomyocyte) not expressing GPC3.

Differentiation induction from an undifferentiated stem cell into a cardiomyocyte can be carried out by using methods described in, for example, PCT International Publication No. WO01/048151, PCT International Publication No. WO2005/033298, PCT International Publication No. WO2008/150030, and the like.

For example, differentiation induction into a cardiomyocyte may be performed by adding a substance that gives rise to differentiation into a cardiomyocyte into the culture medium for culturing an undifferentiated stem cell. Examples of such substances include chromosomal DNA demethylating agents such as demethylase, 5-azacytidine, and DMSO; cytokines such as PDGF, fibroblast growth factor 8 (FGF-8), endothelin 1 (ET1), midkine and osteogenic factor 4 (BMP-4), and G-CSF; adhesive molecules such as gelatin, laminin, collagen, and fibronectin; vitamins such as retinoic acid; transcription factors such as Nkx2.5/Csx, GATA4, MEF-2A, MEF-2B, MEF-2C, MEF-2D, dHAND, eHAND, TEF-1, TEF-3, TEF-5, and MesP1; an extracellular matrix derived from cardiomyocytes; BMP antagonists such as noggin, chordin, fetuin, follistatin, sclerostin, Dan, Cerberus, gremlin, and dante; and the like.

The method of the present embodiment includes a step of culturing a cell mixture containing an undifferentiated stem cell and a differentiated cell in the presence of the undifferentiated stem cell removal agent. In the present specification, the term "culturing" means that cells are maintained or proliferate outside a living body (individual), and includes so-called handling of cells in vitro. In addition, a component used to provide the above-mentioned culture environment to cells is a "culture medium."

In the method of the present embodiment, the culture medium used to culture the cell mixture is not particularly limited, and a general culture medium used in the related art as a culture medium for cell culture can be used. Examples of culture media include Dulbecco's Modified Eagle's Medium culture solution (DMEM), MEM culture solution (α-MEM, MEM [Hank's Bss], and the like), RPMI culture solution (RPMI 1640 and the like), F12 culture solution, StemPre34, mTeSRI, and the like.

The culturing step in the method of the present embodiment can be carried out by adding the undifferentiated stem cell removal agent into the culture medium for cell culture as described above, and culturing the cell mixture containing the undifferentiated stem cell and differentiated cell. Alternatively, the undifferentiated stem cell removal agent may be added to a culture medium in which the cell mixture containing undifferentiated stem cell and differentiated cell is cultured.

An amount of the undifferentiated stem cell removal agent added to the culture medium is not particularly limited. Examples thereof include 0.01 to 1000 μg/mL, 0.05 to 500 μg/mL, 0.1 to 300 μg/mL, and the like, as a final concentration when the undifferentiated stem cell removal agent is added to the culture medium. In addition, in a case where the undifferentiated stem cell removal agent includes the component (a), examples of cell density of cells of the component (a) when the undifferentiated stem cell removal agent is added to culture medium include $1 \times 10$ to $1 \times 10^8$ cells/mL, $1 \times 10^2$ to $1 \times 10^7$ cells/mL, $1 \times 10^3$ to $1 \times 10^6$ cells/mL, $1 \times 10^4$ to $1 \times 10^6$ cells/mL, and the like.

Culturing of the cell mixture in the presence of the undifferentiated stem cell removal agent may be carried out at a temperature generally used for cell culture. For example, a culturing temperature may be 20°C to 40°C, preferably 25°C to 38°C, and more preferably 30°C to 37°C.

A culturing time of the cell mixture in the presence of the undifferentiated stem cell removal agent is not particularly limited, and is preferably 24 hours or more and more preferably 48 hours or more. The cells may be subcultured if necessary. Before and after the subculture, a composition of the culture medium may be the same as or different from each other as long as it contains the undifferentiated stem cell removal agent.

A cell density when the cell mixture is cultured in the presence of the undifferentiated stem cell removal agent is not particularly limited, and is preferably $1 \times 10$ to $1 \times 10^7$ cells/mL, more preferably $1 \times 10^3$ to $1 \times 10^6$ cells/mL, and even more preferably $1 \times 10^4$ to $1 \times 10^6$ cells/mL.

According to the method of the present embodiment, the cell mixture from which undifferentiated stem cells have been removed has a reduced ratio of undifferentiated stem cells and is composed exclusively of differentiated cells. For this reason, according to the method for removing undifferentiated stem cells of the present embodiment, it is possible to obtain a cell mixture in which undifferentiated stem cells are substantially not contained, or a ratio of undifferentiated stem cells is reduced. Therefore, the cell mixture obtained by the method of the present embodiment can be suitably used as cells for transplantation, which are to be transplanted into a living body.

In another aspect, the present invention provides use of at least one component selected from the group consisting of the following (a) and (b), for removing undifferentiated stem cells:

(a) a cell that is capable of specifically inhibiting proliferation of a glypican-3-expressing cell; and
(b) a compound that is capable of specifically inhibiting proliferation of a glypican-3-expressing cell.

<<Production method of cells for transplantation>>

In one embodiment, the present invention provides a production method of cells for transplantation, which includes the following steps (i) and (ii):

a step (i) of inducing a desired differentiated cell from an undifferentiated stem cell; and
a step (ii) of culturing the cell mixture obtained in the step (i) in the presence of the undifferentiated stem cell removal agent.

Among the undifferentiated stem cell removal agents of the above-described embodiment, the undifferentiated stem cell removal agent used in the production method of cells for transplantation of the present embodiment is the undifferentiated stem cell removal agent containing at least one component selected from the group consisting of the following (a) and (b):

(a) a cell that is capable of specifically inhibiting proliferation of a glypican-3-expressing cell; and
(b) a compound that is capable of specifically inhibiting proliferation of a glypican-3-expressing cell.

Accordingly, the step (ii) in the production method of cells for transplantation of the present embodiment can also be said to be a step of culturing the cell mixture obtained in the step (i) in the presence of at least one component selected from the group consisting of the above-mentioned (a) and (b). The components (a) and (b) are the same as those described in the aforementioned <<Undifferentiated stem cell removal agent>>.

The step (i) in the method of the present embodiment is a step of inducing a desired differentiated cell from an undifferentiated stem cell. An undifferentiated stem cell in the step (i) is not particularly limited, and is preferably iPS cell or ES cell and more preferably iPS cell.

In the step (i), the type of a differentiated cell induced from an undifferentiated stem cells is not particularly limited as long as the differentiated cell is induced into a desired differentiated cell. As a method for inducing an undifferentiated stem cell into a differentiated cell, a known method can be appropriately selected and used according to a target differentiated cell. Examples of differentiated cells induced from a undifferentiated stem cell include, but are not limited to, cardiomyocytes, muscle cells, fibroblasts, nerve cells, immune cells such as lymphocytes, vascular cells, ocular cells such as retinal pigment epithelial cells, blood cells such as megakaryocytes and erythrocytes, other tissue cells, precursor cells thereof, and the like. Preferred examples of differentiated cells include cardiomyocytes. The induction from undifferentiated stem cells into cardiomyocytes can be carried out by the method exemplified in the aforementioned <<Method for removing undifferentiated stem cells>>.

Generally, in vitro, because it is difficult to induce all undifferentiated stem cells into differentiated cells, undifferentiated stem cells usually remain in the cell mixture obtained in the step (i), and thereby forming a cell mixture of undifferentiated stem cells and differentiated cells. In addition, the cell mixture may optionally contain components of the culture medium and the like. A form of the cell mixture is not particularly limited, and may be in an aggregated state, a dispersed state, a state of being adhered to a culture container, a state of being adhered to an adhesion factor such as an extracellular matrix, a sheet state, a lumpy state, a colony form, an embryoid body form, a cell clump form, a tissue form, an organ form, and the like.

The step (ii) in the production method of the present embodiment is a step of culturing the cell mixture obtained in the step (i) in the presence of the undifferentiated stem cell removal agent. The culture in this step (ii) can be carried out by the method exemplified in the aforementioned <<Method for removing undifferentiated stem cells>>.

In the production method of the present embodiment may include other steps in addition to the above steps (i) and (ii). Examples of other steps include a step of purifying differentiated cells, a step of recovering differentiated cells, and the like. In a case where the production method of the present embodiment includes these steps, these steps are carried out after the step (ii).

In the step of purifying and the step of recovering differentiated cells, an appropriate method can be appropriately selected according to the type of differentiated cells. For example, in a case where a differentiated cell is a cardiomyocyte, methods described in PCT International Publication No. WO2006/022377, PCT International Publication No. WO2007/088874, PCT International Publication No. WO2016/010165, and the like may be applied to the purification step. In addition, as the recovery step, a centrifugal separation method or the like may be applied.

In the cells for transplantation, which are obtained by the production method of the present embodiment, for example, a differentiated cell ratio represented by differentiated cell/(undifferentiated stem cell + differentiated cell) $\times$ 100 may be 50% or more, 70% or more, 80% or more, 90% or more, or 95% or more. Dead cells are not included in the number of cells described above.

In the cells for transplantation produced by the production method of the present embodiment, since undifferentiated stem cells have been selectively removed, a ratio of undifferentiated stem cells is reduced. Therefore the cells for transplantation are exclusively composed of differentiated cells. For this reason, according to the production method of the present embodiment, it is possible to obtain cells for transplantation, in which undifferentiated stem cells are substantially not contained, or a ratio of undifferentiated stem cells is reduced. Therefore, even in a case where the cells for transplantation are transplanted into a living body, the risk of teratoma formation and the like is reduced.

In another aspect, the present invention provides use of at least one component selected from the group consisting of the following (a) and (b), for the manufacture of a cell for transplantation,:

(a) a cell that is capable of specifically inhibiting proliferation of a glypican-3-expressing cell; and
(b) a compound that is capable of specifically inhibiting proliferation of a glypican-3-expressing cell.

In still another aspect, the present invention provides cells for transplantation, which is produced by the production method of cells for transplantation, the method including the following steps (i) and (ii):

a step (i) of inducing a desired differentiated cell from an undifferentiated stem cell; and
a step (ii) of culturing the cell mixture obtained in the step (i) in the presence of the undifferentiated stem cell removal agent.

<<Pharmaceutical composition>>

In one embodiment, the present invention provides a pharmaceutical composition for treating or preventing a disease which is caused by proliferation of an undifferentiated stem cell, in a subject into which a differentiated cell induced from the undifferentiated stem cell is transplanted, the pharmaceutical composition including the undifferentiated stem cell removal agent.

Among the undifferentiated stem cell removal agents of the above-described embodiment, the undifferentiated stem cell removal agent contained in the pharmaceutical composition of the present embodiment is the undifferentiated stem cell removal agent containing at least one component selected from the group consisting of the following (a) to (d):

(a) a cell that is capable of specifically inhibiting proliferation of a glypican-3-expressing cell;
(b) a compound that is capable of specifically inhibiting proliferation of a glypican-3-expressing cell;
(c) a cell that is capable of inducing a specific immune response against a glypican-3-expressing cell; and
(d) a compound that is capable of inducing a specific immune response against a glypican-3-expressing cell.

Accordingly, the pharmaceutical composition of the present embodiment can also be said to be a pharmaceutical composition containing, as an active ingredient, at least one component selected from the group consisting of the above-described (a) to (d). The components (a) to (d) are the same as those described in the aforementioned

<<Undifferentiated stem cell removal agent>>.

The pharmaceutical composition of the present embodiment contains a therapeutically effective amount of at least one component selected from the group consisting of the above-described (a) to (d). A content of the components (a) to (d) is not particularly limited as long as the content is a therapeutically effective amount. Examples thereof include 0.01 to 900 μg/mg, 0.1 to 500 μg/mg, 0.5 to 300 μg/mg, and 1 to 200 μg/mg, and the like.

The pharmaceutical composition of the present embodiment may contain one of the components selected from the group consisting of (a) to (d), or may contain two or more thereof in combination. In addition, the pharmaceutical composition of the present embodiment may contain components other than the components (a) to (d). The other components are not particularly limited, and the components described in the aforementioned <<Undifferentiated stem cell removal agent>>, and the like can be exemplified.

In addition, the pharmaceutical composition of the present embodiment may contain an immunostimulating agent such as an adjuvant in addition to the other components described in the aforementioned <<Undifferentiated stem cell removal agent>>. Examples of the adjuvant include, but are not limited to, aluminum phosphate, aluminum hydroxide, alum, cholera toxin, Salmonella toxin, incomplete Freund's adjuvant (IFA), complete Freund's adjuvant (CFA) ISCOMATRIX, GM-CSF, CpG, O/W emulsion, and the like. In addition, the pharmaceutical composition of the present embodiment may contain a medicine having pharmacological activity, other than the components (a) to (d). Examples of other medicines include anti-inflammatory agents, analgesic agents, antipyretic agents, other compounds capable of inducing a specific immune response against undifferentiated stem cells, and the like.

Dosage forms of the pharmaceutical composition of the present embodiment are not particularly limited and can be various dosage forms such as a liquid agent, a powder agent, a granule, a tablet, a powder, a suspension, an emulsion, an emulsion preparation, and a liposome preparation. In a case where the component (a) or the component (c) is contained, the pharmaceutical composition of the present embodiment can be in a form of a cell suspension, a cell pellet, a cryopreserved cell, and the like.

The pharmaceutical composition of the present embodiment is used for treating or preventing a disease which is caused by proliferation of an undifferentiated stem cell, in a subject into which a differentiated cell induced from the undifferentiated stem cell is transplanted. In general, because it is difficult to induce all undifferentiated stem cells into differentiated cells in vitro, undifferentiated stem cells may remain in differentiated cells prepared as cells for transplantation in some cases. In a case where undifferentiated stem cells remain transplanted, undifferentiated stem cells proliferate in a living body and may cause diseases such as teratoma. In order to treat or prevent such a disease, the pharmaceutical composition of the present embodiment is administered to a subject into which the differentiated cells are transplanted. Diseases caused by proliferation of undifferentiated stem cells are not particularly limited, and examples thereof include teratoma, cancer, and the like.

Differentiated cells to be transplanted into an administration subject of the pharmaceutical composition of the present embodiment are not particularly limited as long as they are differentiated cells induced from undifferentiated stem cells. Differentiated cells are preferably differentiated from iPS cells or ES cells, and more preferably differentiated from iPS cells. As an example, differentiated cells are cardiomyocytes. In addition, differentiated cells may be cells for transplantation, which are produced by the method described in the aforementioned <<Production method of cells for transplantation>>.

An administration route of the pharmaceutical composition of the present embodiment can be appropriately selected depending on the type of active ingredients, a form of preparations, the type of differentiated cells transplanted, a place where differentiated cells are transplanted, and the like. Examples thereof include intranasal, transdermal, or transbronchial administration; subcutaneous, intradermal, intramuscular, intravenous, intraperitoneal, intraosseous injection; and the like. In addition, administration may be local administration or systemic administration.

For example, in a case where the pharmaceutical composition of the present embodiment includes the component (d), and the component (d) is a Sendai virus vector containing a polynucleotide that encodes a GPC3 protein or a partial peptide thereof, the pharmaceutical composition can be administered intranasally, for example.

A dosage and an administration interval of the pharmaceutical composition of the present embodiment can be appropriately selected according to the type and an amount of transplanted differentiated cells, a place of transplantation, and the like; an age, sex, body weight, and the like of a subject who has undergone transplantation; an administration method; and the like. A dosage can be a therapeutically effective amount of the components (a) to (d). Examples of therapeutically effective amounts of the components (a) to (d) include 0.001 to 1000 mg, 0.01 to 100 mg, 0.1 to 30 mg, 0.1 to 10 mg, 0.5 to 5 mg, and the like per kg of body weight per dosages. In addition, examples of administration intervals include every several days to several months, for example, once a week.

According to the pharmaceutical composition of the present embodiment, it is possible to perform selective removal of undifferentiated stem cells in a living body even in a case where undifferentiated stem cells remain in cells transplanted into a living body. Therefore, diseases caused by proliferation of undifferentiated stem cells in a living body can be treated or prevented.

In still another aspect, the present invention provides use of at least one component selected from the group consisting of the following (a) to (d), for the manufacture of a pharmaceutical composition for treating or preventing a disease which is caused by proliferation of undifferentiated stem cells in a subject into which differentiated cells induced from the undifferentiated stem cells are transplanted:

(a) a cell that is capable of specifically inhibiting proliferation of a glypican-3-expressing cell;
(b) a compound that is capable of specifically inhibiting proliferation of a glypican-3-expressing cell;
(c) a cell that is capable of inducing a specific immune response against a glypican-3-expressing cell; and
(d) a compound that is capable of inducing a specific immune response against a glypican-3-expressing cell.

In still another aspect, the present invention provides use of at least one component selected from the group consisting of the following (a) to (d), for treating or preventing a disease which is caused by proliferation of the undifferentiated stem cells in a subject into which differentiated cells induced from undifferentiated stem cells are transplanted:

(a) a cell that is capable of specifically inhibiting proliferation of a glypican-3-expressing cell;
(b) a compound that is capable of specifically inhibiting proliferation of a glypican-3-expressing cell;
(c) a cell that is capable of inducing a specific immune response against a glypican-3-expressing cell; and
(d) a compound that is capable of inducing a specific immune response against a glypican-3-expressing cell.

In still another aspect, the present invention provides at least one component selected from the group consisting of the following (a) to (d) for use in treating or preventing a disease which is caused by proliferation of the undifferentiated stem cells in a subject into which differentiated cells induced from undifferentiated stem cells are transplanted:

(a) a cell that is capable of specifically inhibiting proliferation of a glypican-3-expressing cell;
(b) a compound that is capable of specifically inhibiting proliferation of a glypican-3-expressing cell;
(c) a cell that is capable of inducing a specific immune response against a glypican-3-expressing cells; and
(d) a compound that is capable of inducing a specific immune response against a glypican-3-expressing cell.

<<Treatment method and the like>>

In one embodiment, the present invention provides a method for treating or preventing a disease which is caused by proliferation of undifferentiated stem cells, the method including administering, to a subject into which the differentiated cells induced from undifferentiated stem cells are transplanted, at least one component selected from the group consisting of the following (a) to (d):

(a) a cell that is capable of specifically inhibiting proliferation of a glypican-3-expressing cell;
(b) a compound that is capable of specifically inhibiting proliferation of a glypican-3-expressing cell;
(c) a cell that is capable of inducing a specific immune response against a glypican-3-expressing cell; and
(d) a compound that is capable of inducing a specific immune response against a glypican-3-expressing cell.

The above-described components (a) to (d) may be administered in a form of the pharmaceutical composition of the above-described embodiment. The above-described components (a) to (d) are the same as those described in the aforementioned <<Undifferentiated stem cell removal agent>>. In the method of the present embodiment, one of the components selected from the group consisting of the (a) to (d) may be administered alone, or two or more thereof may be administered in combination. In addition, the components (a) to (d) may be administered in combination with components other than the components (a) to (d). The other components are not particularly limited. Examples thereof include the components described in the aforementioned <<Undifferentiated stem cell removal agent>> and <<Pharmaceutical composition>>.

A therapeutically effective amount of the above-described components (a) to (d) is administered to a subject into which differentiated cells induced from undifferentiated stem cells are transplanted. An effective amount varies according to the type and an amount of transplanted differentiated cells, a place of transplantation, and the like; an age, sex, body weight, and the like of a subject who has undergone transplantation; an administration method; and the like. Examples of effective amounts of the components (a) to (d) include 0.001 to 1000 mg, 0.01 to 100 mg, 0.1 to 30 mg, 0.1 to 10 mg, 0.5 to 5 mg, and the like per kg of body weight per dosages. In addition, examples of administration intervals include every several days to several months, for example, once a week.

An administration subject and a target disease can be the same as those described in the aforementioned <<Pharmaceutical composition>>. In addition, an administration method can be the same as that described in the aforementioned

<<Pharmaceutical composition>>.

In addition, in one embodiment, the present invention provides a method for transplanting the cells for transplantation, the method including the following steps (i) to (iii):

a step (i) of inducing a desired differentiated cell from an undifferentiated stem cell;
a step (ii) of culturing the cell mixture obtained in the step (i) in the presence of the undifferentiated stem cell removal agent so as to obtain the cells for transplantation; and
a step (iii) of transplanting the cells for transplantation obtained in the step (iii) to a subject requiring transplantation.

The steps (i) and (ii) in the transplantation method of the present embodiment can be carried out in the same manner as the steps (i) and (ii) described in the aforementioned <<Production method of cells for transplantation>>. In addition, among the undifferentiated stem cell removal agents of the above-described embodiment, the undifferentiated stem cell removal agent used in the step (ii) of the transplantation method of the present embodiment is the undifferentiated stem cell removal agent containing at least one component selected from the group consisting of the following (a) and (b):

(a) a cell that is capable of specifically inhibiting proliferation of a glypican-3-expressing cell; and
(b) a compound that is capable of specifically inhibiting proliferation of a glypican-3-expressing cell.

The components (a) and (b) are the same as those described in the aforementioned <<Undifferentiated stem cell removal agent>>.

The step (iii) in the transplantation method of the present embodiment is a step of transplanting the cells for transplantation obtained in the step (ii) to a subject requiring transplantation. The "subject requiring transplantation" is a subject in which defects, damage, or the like have occurred in a cell of the same type as the cells for transplantation obtained in the step (ii) in a living body of the object, and by transplanting the cells for transplantation, symptoms caused due to defects, damage, or the like of the cells are expected to be ameliorated. Transplantation can be carried out by a general method of cell transplantation.

The transplantation method of the present embodiment may include other steps in addition to the above-described steps (i) to (iii). Examples of other steps include a step of purifying differentiated cells, a step of recovering differentiated cells, and the like. In a case where the transplantation method of the present embodiment includes these steps, these steps are carried out between the step (ii) and the step (iii). Such a purification step and recovery step can be carried out as described in the aforementioned

<<Production method of cells for transplantation>>.

According to the transplantation method of the present embodiment, the cells for transplantation in which undifferentiated stem cells are substantially not contained, or a ratio of undifferentiated stem cells is reduced, can be transplanted into a living body. Therefore the risk of teratoma formation or the like is reduced.

After the step (iii), the transplantation method of the present embodiment may further include the following step (iv):
the step (iv) of administering, to a subject into which the cells for transplantation are transplanted in the step (iii), at least one component selected from the group consisting of the following (a) to (d):

(a) a cell (a) that is capable of specifically inhibiting proliferation of a glypican-3-expressing cell;
(b) a compound that is capable of specifically inhibiting proliferation of a glypican-3-expressing cell;
(c) a cell that is capable of inducing a specific immune response against a glypican-3-expressing cell; and
(d) a compound that is capable of inducing a specific immune response against a glypican-3-expressing cell.

The above step (iv) can be carried out in the same manner as the method for treatment and prevention of the above-described embodiment. By carrying out the step (iv), even in a case where undifferentiated stem cells remain transplanted into the cells for transplantation, it is possible to prevent the onset of diseases caused by proliferation of the undifferentiated stem cells, such as teratomas.

Examples

Hereinafter, the present invention will be described based on experimental examples, but the present invention is not limited to the following experimental examples.

[Method and the like]

(Culture of cells)

Among iPSC1 to iPSC3, which are iPS cell lines, iPSC1 and iPSC2 were provided by the Center for iPS Cell Research and Application, Kyoto University. iPSC3 was established from the peripheral blood of a healthy person by using a Sendai virus vector. Heart-1 and Heart-2, which are cardiomyocytes, were purchased from Cosmo Bio and Takara Bio Inc., respectively.

The cells were cultured in an incubator having conditions set at 37°C and 5% $CO_2$. The human iPS cells were subjected to undifferentiation-maintaining culture by using Matrigel (BD Bioscience cat 354277). As a culture solution, mTeSR1 (STEMCELL Technologies Inc. cat 11875-119) was used. In addition to mTeSR1, any solution for undifferentiation maintenance can be used as long as it is generally used as a feeder-free culture medium such as Essential 8 (Life Technologies), TeSR2 (STEMCELL Technologies Inc.), and the like. In addition, matrices include Vitronectin (Life Technologies), iMatrix-511 (Takara no. 892001), and the like, in addition to Matrigel, and any matrix can be used as long as it is commonly used as other feeder-free matrix.

Upon passage, human embryonic stem cells and induced pluripotent stem cells were dispersion-treated at 37°C for 5 minutes with StemPro Accutase (Life Technologies no. 1110501). In addition to StemPro Accutase, CTK solution (Repro CELL) and TrypLE Express/Select (Life Technologies) can also be used for dispersion treatment of cells.

(Induction of cardiomyocytes from undifferentiated stem cells)

The differentiation induction of cardiomyocytes from undifferentiated stem cells was carried out in the following order.

- When iPSC1 reached 50% to 90% confluence per myocardial differentiation, a culture medium was exchanged to a culture medium in which 6 μM of B27 (no insulin, Invitrogen) and CHIR99021 (Selleckchemor Wako) was added into RPMI culture medium (Invitrogen) (Day 0).
- On Day 1 and Day 2, culturing in RPMI/B27 insulin(-) culture medium was carried out.
- Next, on Day 3 to Day 5, culturing in a culture medium into which 5 μM of IWP2 or 5 μM of IWR-1 (Sigma I0161) was further added to the RPMI/B27 insulin(-) culture medium was carried out.
- Furthermore, on Day 6 and Day 7, culturing in the RPMI/B27 insulin(-) culture medium was carried out.

- In addition, from Day 8 onwards, culturing was carried out in RPMI/B27 insulin(+) culture medium (Lian, X., et al., Nat Protocol, 2013, 8, 162-175). At the stage of Day 8 to Day 11, pulsating cardiomyocytes could be confirmed.

iPSC1-CM is cardiomyocytes which are differentiation-induced from iPSC1.

(Quantitative RT-PCR analysis)

Total RNA samples were isolated by using TRIZOL reagent (manufactured by Life Technologies) according to the instructions attached to the product. A concentration and a degree of purity of the RNA sample were measured with an ND-1000 spectrophotometer (manufactured by Thermo Fisher Scientific). Preparation of cDNA was performed by using ReverTra Ace qPCR RT Master Mix (manufactured by Toyobo Co., Ltd.). Quantitative PCR (QT-PCR) was performed by using SYBR Premix Ex Taq (Takara Bio Inc.) with a 7500 real-time PCR system (manufactured by Life Technologies). An amount of mRNA was standardized by an amount of mRNA of GAPDH. Nucleotide sequences of primer sets used for quantitative PCR are shown in Table 1.

[Table 1]

| Gene name | Forward primer | SEQ ID NO | Reverse primer | SEQ ID NO |
|---|---|---|---|---|
| GPC3 | GAGCCAGTGGTCAGTCAAAT | 24 | CTTCATCATCACCGCAGTC | 25 |
| GAPDH | GTGGACCTGACCTGCCGTCT | 26 | GGAGGAGTGGGTGTCGCTGT | 27 |

(FACS analysis)

The dispersion-treated cells were washed once with PBS, and incubated for 30 minutes at 4°C in the presence of a primary antibody, GPC3 antibody [B0025R, manufactured by Biomosaics]. The cells were washed with PBS and incubated at 4°C for 30 minutes with a secondary antibody (anti-mouse IgG antibody conjugated with Alexa Fluor 488 (manufactured by Life Technologies)). After washing the cells with PBS, measurement was performed by flow cytometry (manufactured by BD).

(Immunohistochemical staining method)

iPS cells seeded on a 24-black well plate (manufactured by Porvair Sciences) or cardiomyocytes derived from iPS cells, or cells in which these cells are mixed were washed once with PBS, and subjected to fixation treatment at 4°C for 15 minutes with 4% paraformaldehyde (manufactured by MUTO PURE CHEMICALS CO.,LTD.). The fixation-treated cells were permeabilized with 0.5% Triton X-100 in PBS for 10 minutes at room temperature. Thereafter, the cells were incubated overnight at 4°C under the presence of two types of primary antibody Oct3/4 antibody [MAB1759, manufactured by R & D Systems] and GPC3 antibody [B0025R, manufactured by Biomosaics], or Oct3/4 antibody [MAB1759, manufactured by R & D Systems] and anti-Troponin I antibody [ab52862, manufactured by Abcam] or anti-Troponin T antibody [13-11, manufactured by Thermo Fisher]. The cells were washed with 10% FBS in PBS and incubated with secondary antibodies (anti-mouse IgG antibody, anti-rabbit IgG antibody, or anti-rat IgG antibody conjugated with Alexa Fluor 488 or Alexa Fluor 546 [manufactured by Life Technologies]) corresponding to each of primary antibodies at room temperature for 90 minutes. After staining the cell nucleus with 6-diamidino-2-phenylindole (DAPI; manufactured by Life Technologies), fluorescent observation was performed using a fluorescence microscope (BZ-9000; manufactured by Keyence).

[Experimental Example 1] Analysis of GPC3 expression

FIG. 1A shows analysis results of GPC3 expression by microarray in ES cell lines (ESC-1 and ESC-2), iPS cell lines (iPSC-1 and iPSC-2), and a normal heart (Heart). Data from NIH Stem cell database was used. FIG. 1B shows analysis results of GPC3 expression by RNA sequencing in iPS cells (iPSC1), cardiomyocytes (iPSC1-CM) induced from iPS cells, and cardiomyocytes of a normal heart (Heart-1). RNA sequence analysis was performed according to the following procedure. RNA was extracted from iPSC1 and iPSC1-CM by using RNA extraction kit [RNeasy mini kit (manufactured by QIAGEN)] to confirm a degree of purity by a bioanalyzer (manufactured by Agilent Technologies). Thereafter, a library was produced by using TruSeq RNA Sample Prep Kit v2 (manufactured by illumina) to perform analysis by using HiSeq 1500 (manufactured by illumina). Read count data obtained from the RNA sequence was normalized, and values of fragments per kilobase of exon per million reads mapped (FPKM) were calculated. The calculation formula is shown below.

$$\text{FPKM} = (\text{fragments} \times 1{,}000{,}000 \times 1{,}000)/(\text{all fragments} \times \text{gene length})$$

As shown in FIG. 1A and FIG. 1B, expression of GPC3 was observed in pluripotent stem cells (ES cells and iPS cells). On the other hand, expression of GPC3 was hardly observed in cardiomyocytes of the normal heart and cardiomyocytes differentiation-induced from iPS cells.

FIG. 2A and FIG. 2B show analysis results of GPC3 expression in iPS cells (iPSC1 to iPSC3), cardiomyocytes (iPSC1-CM) differentiation-induced from iPSC1, and cardiomyocytes of a normal heart (Heart-1 and Heart-2). FIG. 2A is the analysis results by quantitative PCR, and FIG. 2B is the analysis results by FACS.

As shown in FIG. 2A and FIG. 2B, expression of GPC3 was observed in iPS cells. On the other hand, expression of GPC3 was hardly observed in cardiomyocytes differentiation-induced from iPS cells and cardiomyocytes of the normal cells.

FIG. 3A and FIG. 3B are observation images of GPC3 staining by fluorescent immunostaining in iPS cells (iPSC1) and cardiomyocytes (iPSC1-CM) differentiation-induced from iPSC1. FIG. 3A shows stained images of iPS cells, and FIG. 3B shows stained images of cardiomyocytes derived from iPSC1. As markers for each of the cells, Oct3/4 was used in FIG. 3A, and troponin 1 was used in FIG. 3B.

As shown in FIG. 3A and FIG. 3B, expression of GPC3 was observed in iPS cells. On the other hand, expression of GPC3 was hardly observed in cardiomyocytes differentiation-induced from iPS cells.

[Experimental Example 2] Response test of GPC3-specific CTLs with respect to cardiomyocytes derived from iPS cells

(Response test of GPC3-specific CTLs with respect to iPS cells)

As HLA-A2-restricted GPC3-specific CTLs, a clone previously established by using a peptide (SEQ ID NO: 22: FVGEFFTDV) derived from GPC3 was used (PCT International Publication No. WO2007/018199).

GPC3-specific CTLs were co-cultured with iPSC1 or cardiomyocytes (iPSC1-CM) induced from iPSC1, and responsiveness of the GPC3-specific CTLs with respect to these cells was tested. The responsiveness of the GPC3-specific CTLs was confirmed by IFN-$\gamma$ ELISPOT assay. Detection of IFN-$\gamma$ was performed by using ELISPOT Human IFN-$\gamma$ set (BD). First, an anti-human IFN-$\gamma$ antibody was coated on an ELISPOT plate (BD Bioscience) for 18 hours. Thereafter, blocking was performed for 2 hours with 10% FCS/RPM1. The GPC3-specific CTLs (100 $\mu$L/well) and iPS cells (100 $\mu$L/well) were mixed and cultured at 37°C for 22 hours. A ratio of the GPC3-specific CTL/the iPS cells was 5:1. Thereafter, the plate was washed with sterile water, reacted with a biotinylated anti-human IFN-$\gamma$ antibody for 2 hours, and further reacted with streptavidin-HRP for 1 hour. IFN-$\gamma$-positive spots were detected with a substrate solution. Spot counting was performed by using automatic analysis software of MINE MiNERVA TECH.

The results of IFN-$\gamma$ ELISPOT assay are shown in FIG. 4. As shown in FIG. 4, the GPC3-specific CTLs showed high responsiveness to iPSC1. On the other hand, the responsiveness of the GPC3-specific CTLs to iPSC1-CM, which is a cardiomyocyte induced from iPSC1, was extremely low.

(Response test of GPC3-specific CTLs to iPS cells and cardiomyocytes)

iPSC1 or cardiomyocytes (iPSC1-CM) induced from iPSC1 were cultured in a 96-well plate. When the cells reached 50% to 60% confluence, $3 \times 10^5$ cells of the GPC3-specific CTLs were added and cultured at 37°C for 48 hours. The cells were washed with PBS. Living cells were fluorescently labelled by using calcein AM, and then fluorescent intensity of each well was detected. The results are shown in FIG. 5A and FIG. 5B. FIG. 5A shows the results for iPSC1, and FIG. 5B shows the results for iPSC1-CM. Bar graphs show relative fluorescent intensity when fluorescent intensity in a case where the GPC3-specific CTLs were not added (not co-cultured) was 1. In the drawings, "inactivated CTL" is a GPC3-specific CTL inactivated by heat treatment, and the results of co-culture with the inactivated CTLs are shown as a negative control.

As shown in FIG. 5A and FIG. 5B, living cells were hardly observed when iPSC1 was co-cultured with GPC3-specific CTLs. On the other hand, when cardiomyocytes (iPSC1-CM) induced from iPSC1 were co-cultured with GPC3-specific CTLs, living cells were observed to the same extent as in the case where GPC3-specific CTLs were not added.

Based on the results of FIGS. 4A and 4B and FIGS. 5A and 5B, it was confirmed that the GPC3-specific CTLs show responsiveness to iPS cells but hardly show responsiveness to cardiomyocytes induced from iPS cells.

[Experimental Example 3] Response test of GPC3-specific CTL to GPC3 knockdown cells

(Creation of GPC3 knockdown cells)

GPC3 siRNA (SEQ ID NO: 28: 5'-GGAGGCUCUGGUGAUGGAAUGAUAA-3') which is siRNA against GPC3 was synthesized and introduced into iPS-G4. As a negative control, a negative siRNA [AllStars Negative Control siRNA (manufactured by QIAGEN)] was used. A knockdown effect due to GPC3 siRNA was confirmed by quantitative PCR and FACS. Confirmation results of the knockdown effect of GPC3 siRNA by quantitative PCR and FACS are shown in FIGS. 6A and 6B. FIG. 6A shows the confirmation results by quantitative PCR, and FIG. 6B shows the confirmation results by FACS. Based on the results of FIG. 6A and FIG. 6B, the knockdown effect of GPC3 was confirmed in the cells into which GPC3 siRNA was introduced.

(Response test of GPC3-specific CTL to GPC3 knockdown cells)

The GPC3-specific CTLs were co-cultured with iPSC1 into which GPC3 siRNA was introduced, or iPSC1 into which a negative siRNA was introduced, and responsiveness of the GPC3-specific CTLs to these cells was tested. The responsiveness of the GPC3-specific CTLs was confirmed by IFN-$\gamma$ ELISPOT assay. The IFN-$\gamma$ ELISPOT assay was performed in the same manner as in Experimental Example 2.

The results of IFN-$\gamma$ ELISPOT assay are shown in FIG. 7. As shown in FIG. 7, the GPC3-specific CTLs showed high responsiveness to iPSC1 into which a negative siRNA was introduced. On the other hand, the responsiveness of the GPC3-specific CTLs to iPSC1 into which GPC3 siRNA was introduced was extremely low. Based on the results in FIG. 7, it was confirmed that the responsiveness of GPC3-specific CTLs with respect to iPS cells was GPC3-specific.

[Experimental Example 4] Response test of GPC3-specific CTL to cell mixture of iPS cells and cardiomyocytes

iPSC1 and iPSC1-CM were mixed at a ratio of 1:20. $3 \times 10^6$ cells of GPC3-specific CTLs were added to the mixture, and co-cultured at 37°C for 48 hours. iPSC1 which was stained with cell tracker Green CMFDA [C7025 (manufactured by Thermo Fisher)] was used in FACS analysis. After co-culture, FACS analysis was performed.

The results of FACS analysis are shown in FIGS. 8A and 8B. FIG. 8A shows the results of FACS analysis, and FIG. 8B shows the results of FACS analysis in numerical form. As shown in FIGS. 8A and 8B, in a case of co-culturing with GPC3-specific CTLs (co-cultured), a ratio of iPSC1 dropped significantly as compared with a case of not co-culturing with GPC3-specific CTLs (not co-cultured).

Next, cells obtained by co-culturing of the cell mixture of iPSC1 and iPSC1-CM with GPC3-specific CTLs were subjected to immunohistochemical staining, and fluorescent observation was performed. The results are shown in FIG. 9A and FIG. 9B. FIG. 9A shows a case of co-culturing with GPC3-specific CTLs, and FIG. 9B shows a case of not co-culturing with GPC3-specific CTLs. As shown in FIG. 9A and FIG. 9B, in the case of not co-culturing with GPC3-specific CTLs, iPS cells expressing Oct3/4 were observed. On the other hand, in the case of co-culturing with GPC3-specific CTLs, iPS cells expressing Oct3/4 were hardly observed.

Based on the results of FIGS. 8A and 8B, and FIGS. 9A and 9B, it was confirmed that GPC3-specific CTLs can selectively remove only iPS cells in the cell mixture of iPS cells and cardiomyocytes.

Industrial Applicability

According to the present invention, a technique is provided for selectively removing only undifferentiated stem cells without affecting differentiated cells, at the time of preparation of the differentiated cells induced from the undifferentiated stem cells, and in a living body after transplantation. The technique of the present invention can be applied to a preparation of cells for transplantation which are differentiation-induced from undifferentiated stem cells such as iPS cells and ES cells, and to prevention or treatment of teratoma formation after transplantation.

## Claims

1. An undifferentiated stem cell removal agent, comprising at least one component selected from the group consisting of the following (a) and (b):

   (a) a cell that is capable of specifically inhibiting proliferation of a glypican-3-expressing cell; and
   (b) a compound that is capable of specifically inhibiting proliferation of a glypican-3-expressing cell.

2. The undifferentiated stem cell removal agent according to Claim 1, wherein the cell that is capable of specifically inhibiting proliferation of a glypican-3-expressing cell of (a) is a cytotoxic T cell that has cytotoxic activity specific to a glypican-3-expressing cell.

3. The undifferentiated stem cell removal agent according to Claim 1, wherein the compound that is capable of specifically inhibiting proliferation of a glypican-3-expressing cell of (b is an antibody against glypican-3 protein.

4. An undifferentiated stem cell removal agent, comprising at least one component selected from the group consisting of the following (c) and (d):

   (a) a cell that is capable of inducing a specific immune response against a glypican-3-expressing cell; and
   (b) a compound that is capable of inducing a specific immune response against a glypican-3-expressing cell.

5. The undifferentiated stem cell removal agent according to Claim 4, wherein the compound that is capable of inducing a specific immune response against a glypican-3-expressing cell of (d) is a glypican-3 protein or a partial peptide thereof, or a salt thereof.

6. The undifferentiated stem cell removal agent according to Claim 4, wherein the compound that is capable of inducing a specific immune response against a glypican-3-expressing cell of (d) is a vector containing a polynucleotide that encodes a glypican-3 protein or a partial peptide thereof.

7. The undifferentiated stem cell removal agent according to Claim 4, wherein the cell that is capable of inducing a specific immune response against a glypican-3-expressing cell of (c) is a dendritic cell presenting a partial peptide of a glypican-3 protein at a cell surface.

8. The undifferentiated stem cell removal agent according to any one of Claims 1 to 7, wherein the undifferentiated stem cell is an induced pluripotent stem cell.

9. A method for removing an undifferentiated stem cell, comprising culturing a cell mixture that contains an undifferentiated stem cell and a differentiated cell in the presence of the undifferentiated stem cell removal agent according to any one of Claims 1 to 3.

10. The method for removing undifferentiated stem cells according to Claim 9, wherein the undifferentiated stem cell is an induced pluripotent stem cell.

11. The method for removing an undifferentiated stem cell according to Claim 9 or 10, wherein the differentiated cell is a cardiomyocyte.

12. A production method of a cell for transplantation, comprising the following steps (i) and (ii):

    a step (i) of inducing a desired differentiated cell from an undifferentiated stem cell; and
    a step (ii) of culturing a cell mixture obtained in the step (i) in the presence of the undifferentiated stem cell removal agent according to any one of Claims 1 to 3.

13. The production method according to Claim 12, wherein the undifferentiated stem cell is an induced pluripotent stem cell.

14. The production method according to Claim 12 or 13, wherein the differentiated cell is a cardiomyocyte.

15. A pharmaceutical composition for treating or preventing a disease which is caused by proliferation of an undifferentiated stem cell, in a subject into which a differentiated cell that is differentiated from the undifferentiated stem cell is transplanted, the pharmaceutical composition comprising the undifferentiated stem cell removal agent according to any one of Claims 1 to 8.

16. The pharmaceutical composition according to Claim 15, wherein the undifferentiated stem cell is an induced pluripotent stem cell.

17. The pharmaceutical composition according to Claim 15 or 16, wherein the differentiated cell induced from the undifferentiated stem cell is a cardiomyocyte.

18. The pharmaceutical composition according to any one of Claims 15 to 17, further comprising a Sendai virus vector containing a polynucleotide that encodes a GPC3 protein or a partial peptide thereof.

19. The pharmaceutical composition according to Claim 18, which is administered intranasally.

FIG. 1A

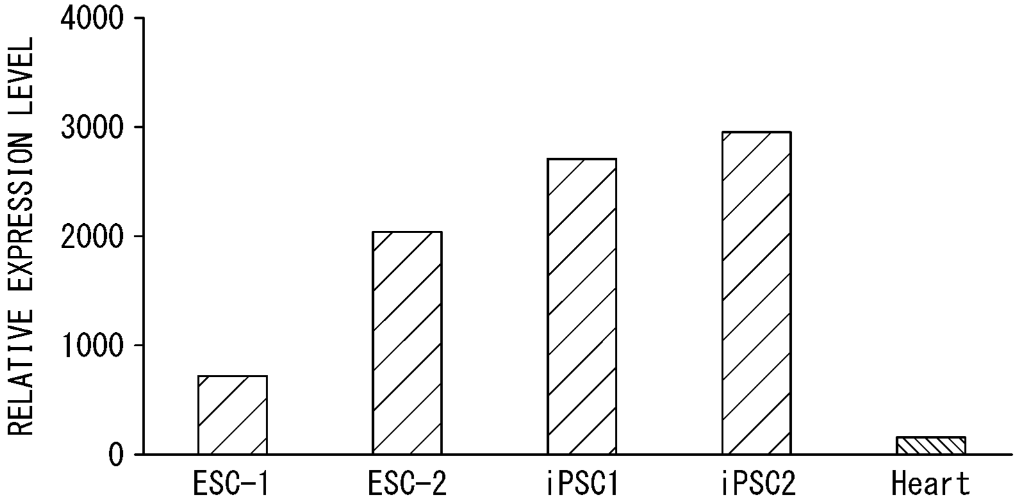
4000
3000
2000
1000
0
RELATIVE EXPRESSION LEVEL
ESC-1
ESC-2
iPSC1
iPSC2
Heart

FIG. 1B

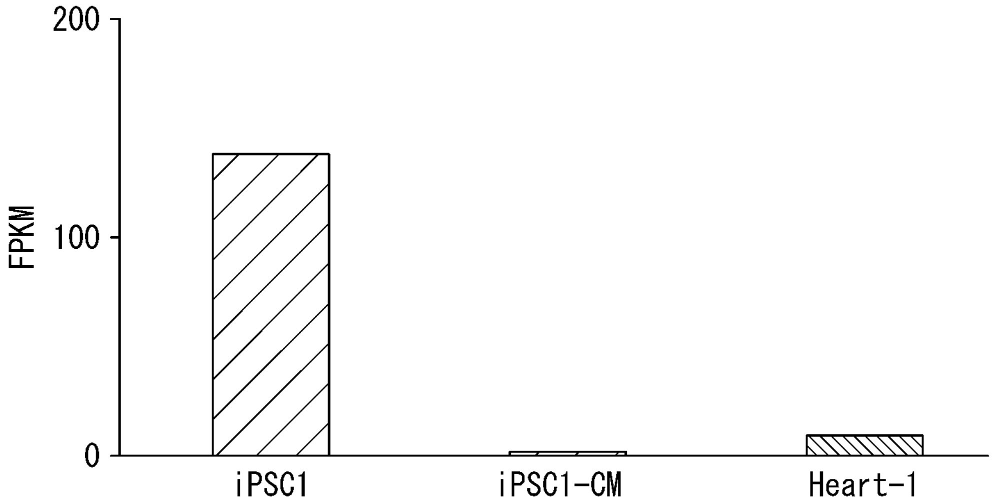
200
100
0
FPKM
iPSC1
iPSC1-CM
Heart-1

FIG. 2A

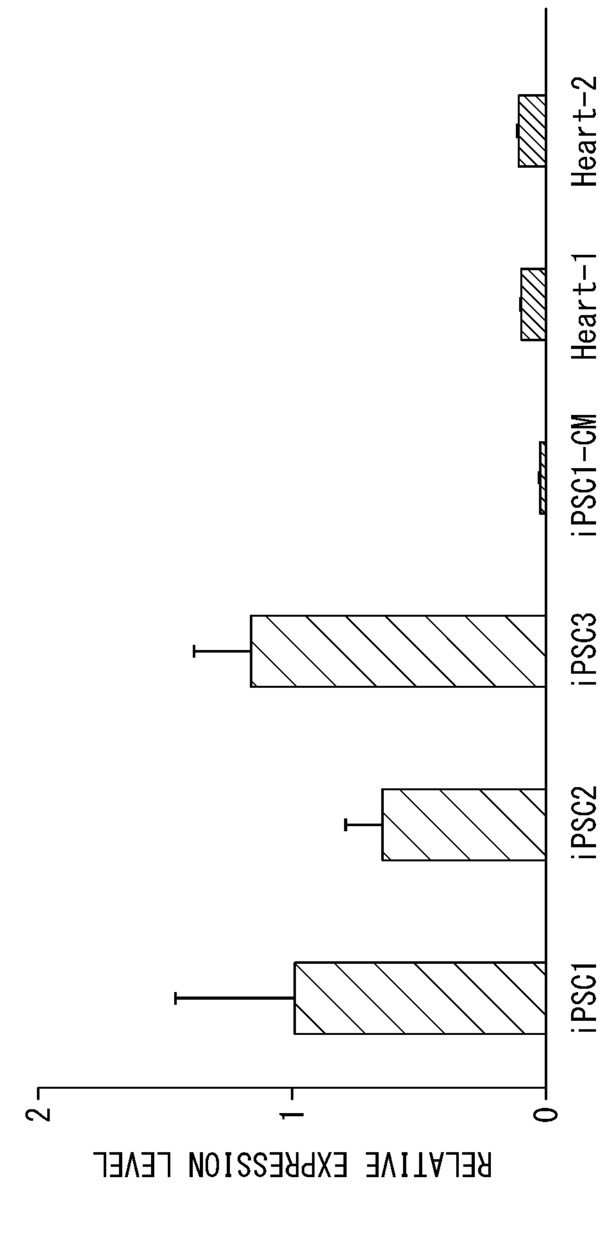
RELATIVE EXPRESSION LEVEL
0
1
2
iPSC1
iPSC2
iPSC3
iPSC1-CM
Heart-1
Heart-2

FIG. 2B

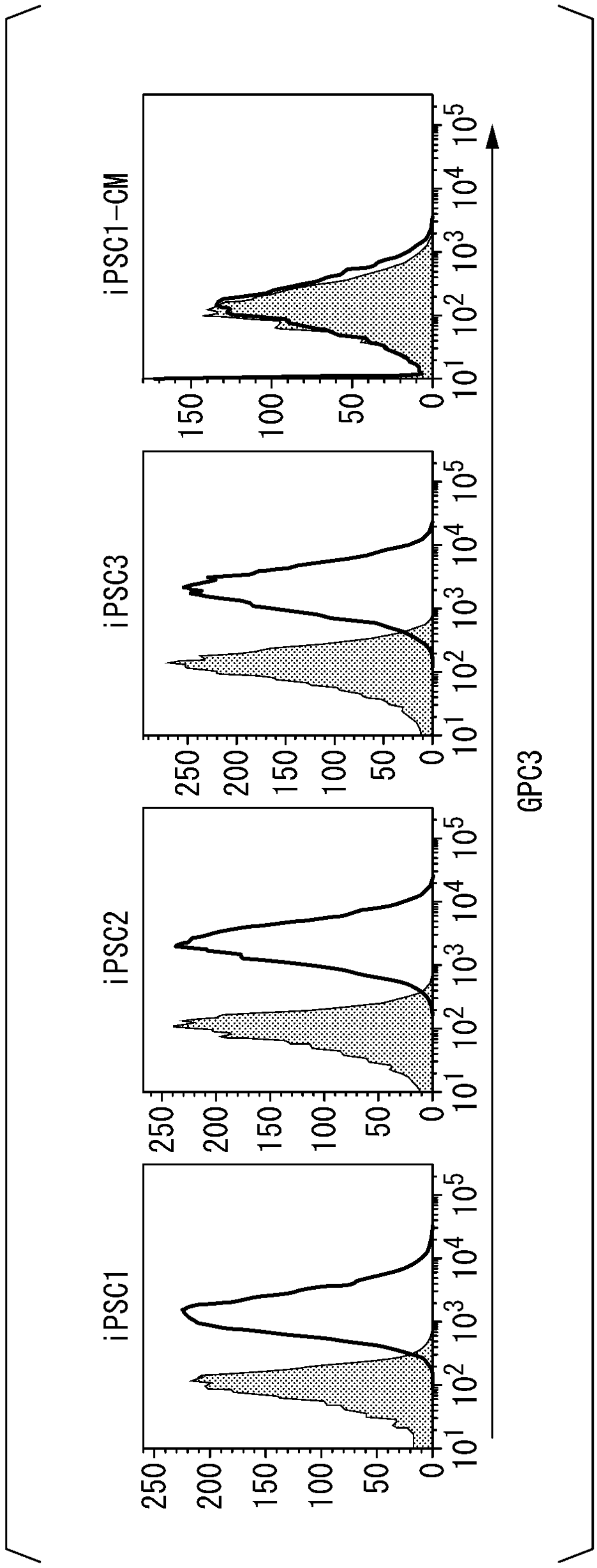
iPSC1
iPSC2
iPSC3
iPSC1-CM
250
200
150
100
50
0
$10^1$
$10^2$
$10^3$
$10^4$
$10^5$
GPC3

FIG. 3A

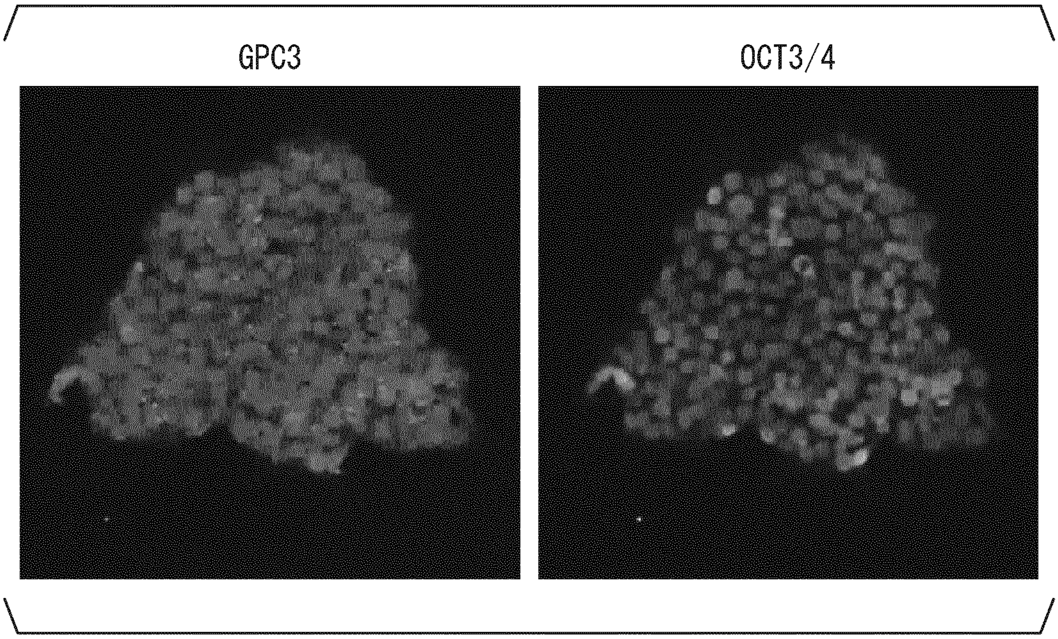


FIG. 3B

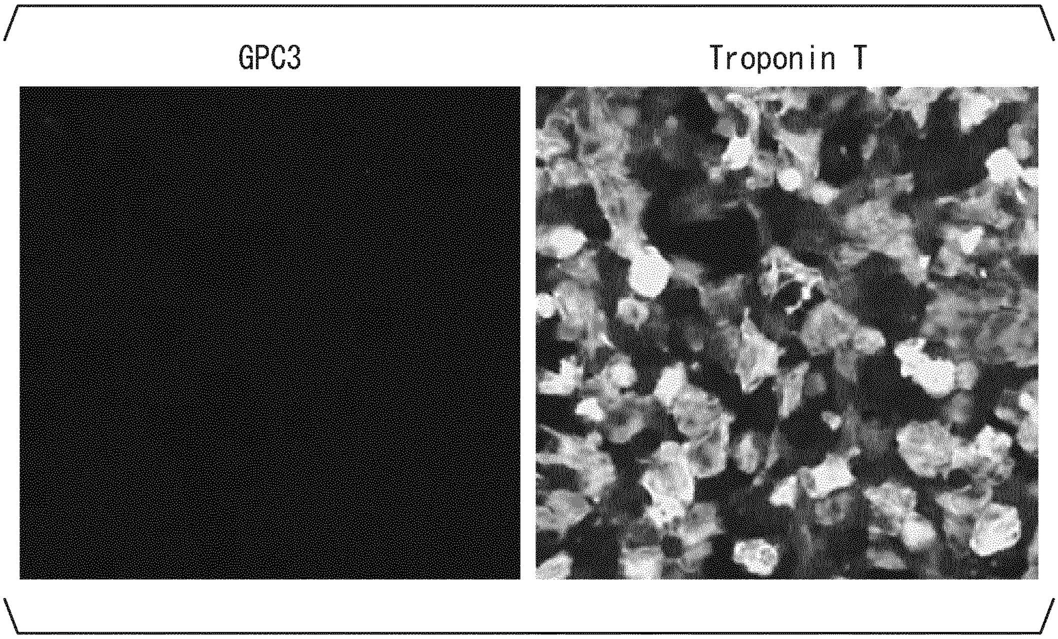

FIG. 4

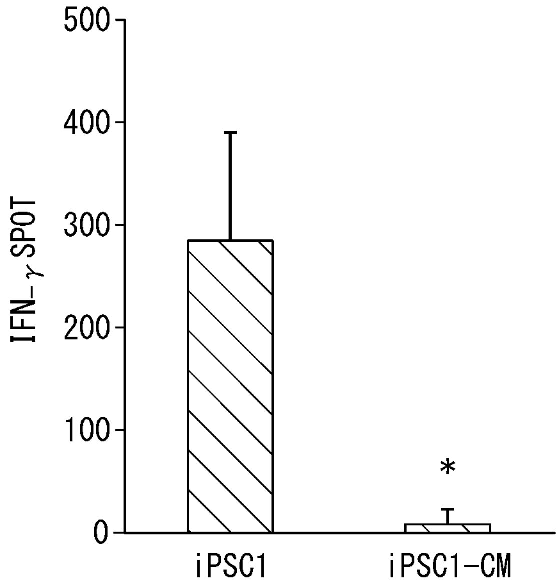

FIG. 5A

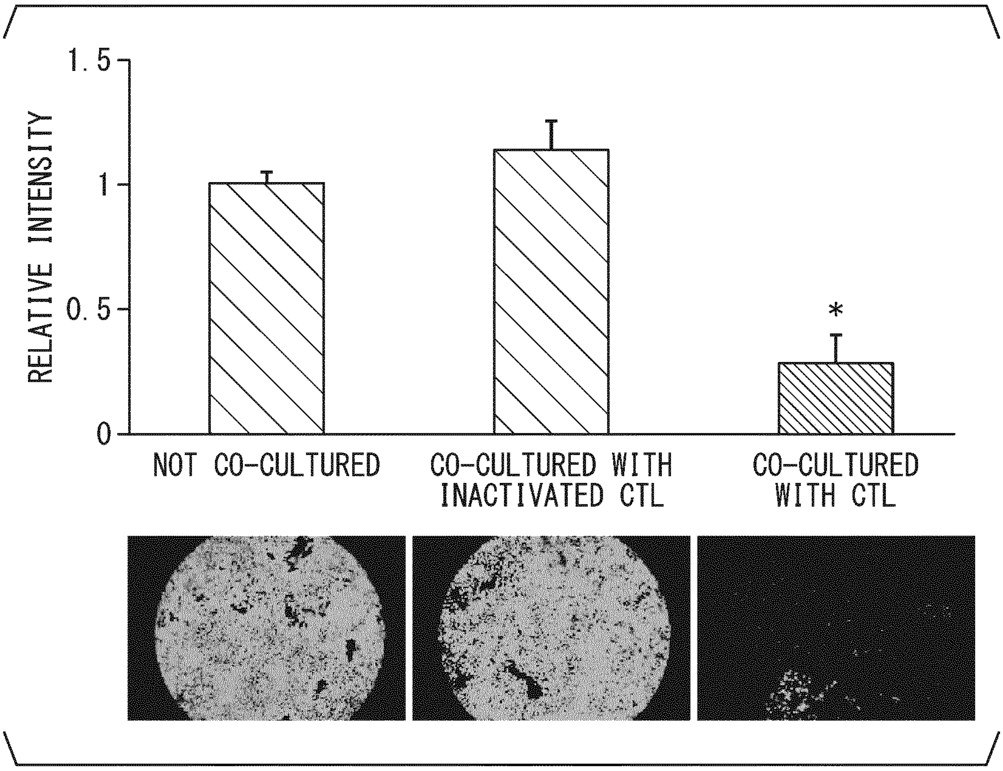
1.5
1
0.5
0
RELATIVE INTENSITY
*
NOT CO-CULTURED
CO-CULTURED WITH INACTIVATED CTL
CO-CULTURED WITH CTL

FIG. 5B

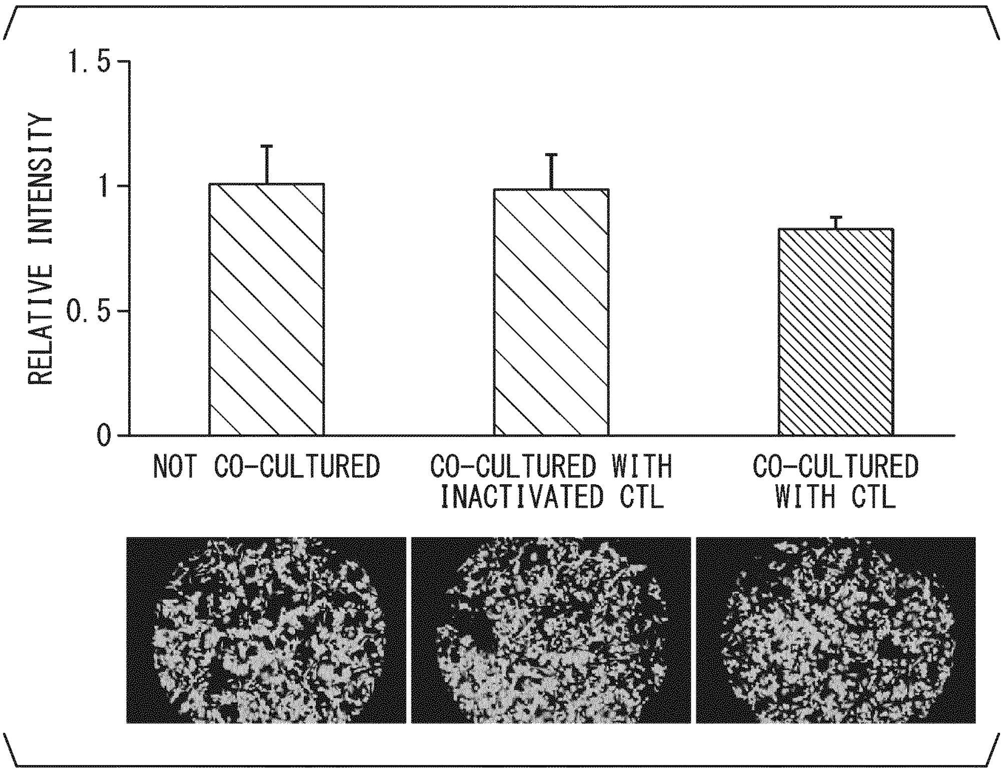
1.5
1
0.5
0
RELATIVE INTENSITY
NOT CO-CULTURED
CO-CULTURED WITH INACTIVATED CTL
CO-CULTURED WITH CTL

FIG. 6A

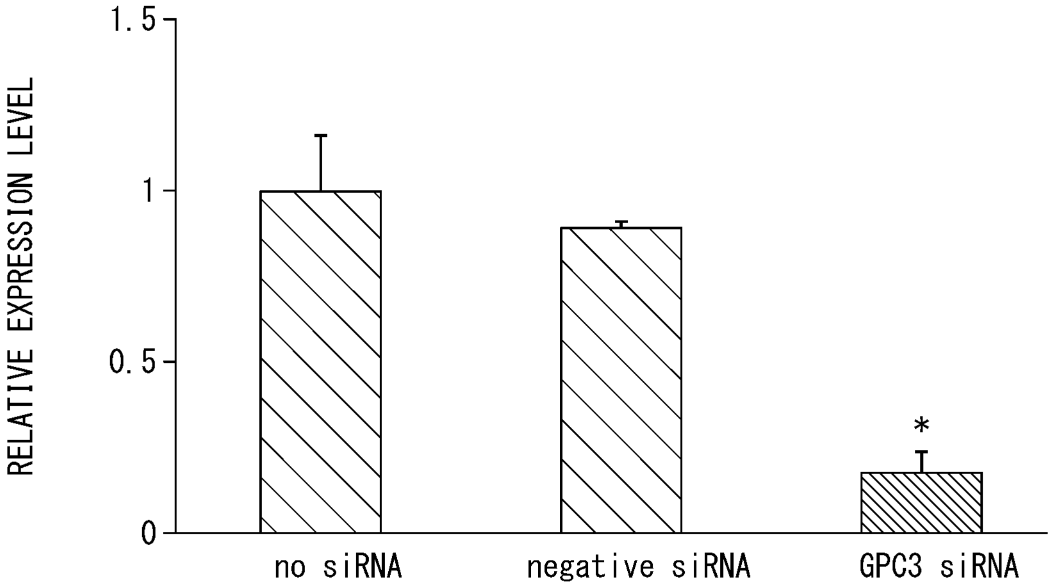
RELATIVE EXPRESSION LEVEL
1.5
1
0.5
0
*
no siRNA
negative siRNA
GPC3 siRNA

FIG. 6B

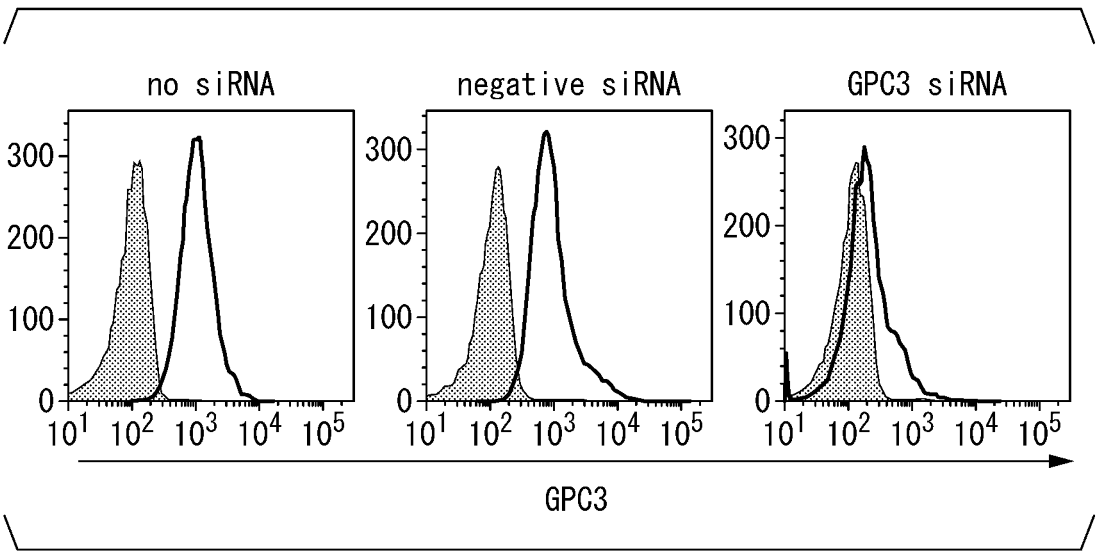
no siRNA
negative siRNA
GPC3 siRNA
300
200
100
0
$10^1$ $10^2$ $10^3$ $10^4$ $10^5$
GPC3

FIG. 7

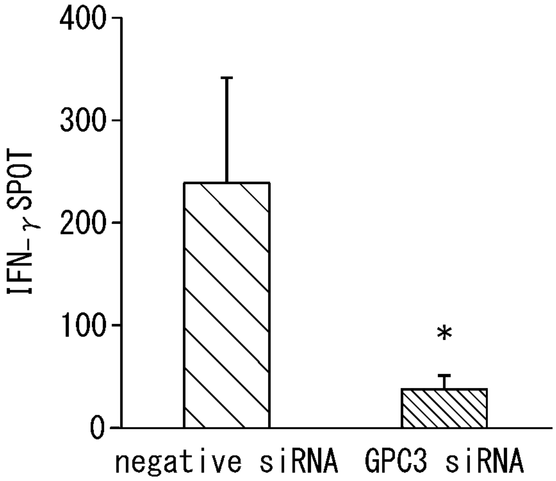

FIG. 8A

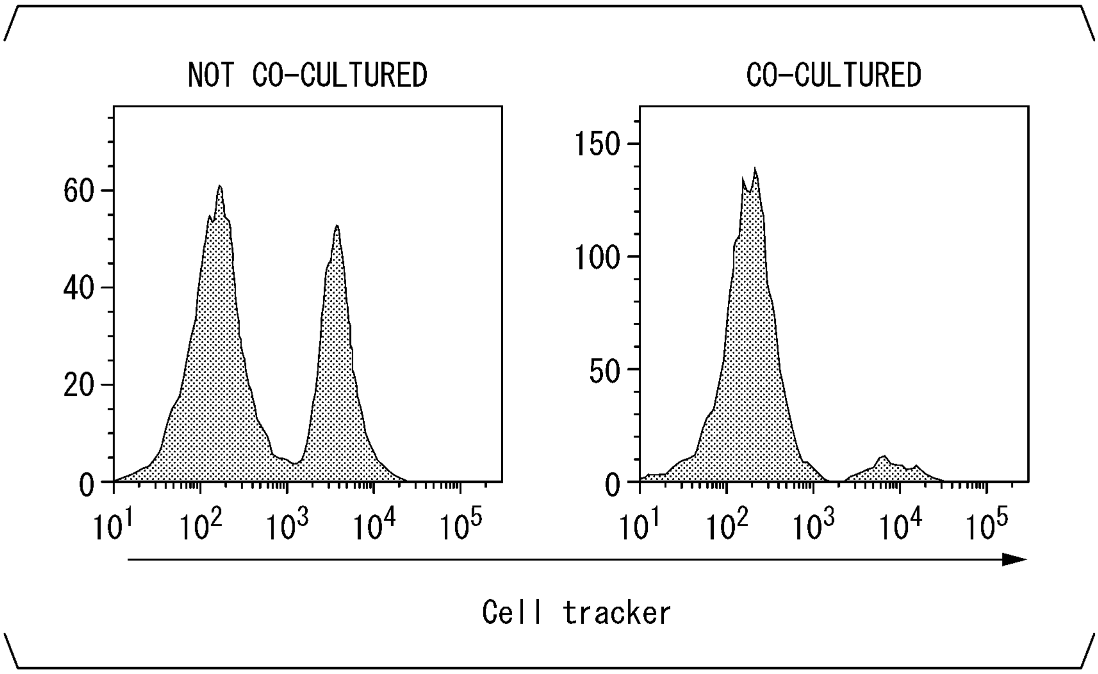
NOT CO-CULTURED
CO-CULTURED
60
40
20
0
150
100
50
0
$10^1$ $10^2$ $10^3$ $10^4$ $10^5$
$10^1$ $10^2$ $10^3$ $10^4$ $10^5$
Cell tracker

FIG. 8B

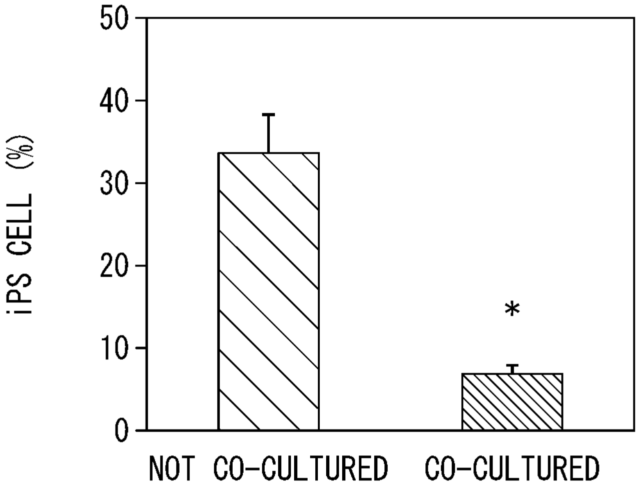
iPS CELL (%)
50
40
30
20
10
0
*
NOT CO-CULTURED
CO-CULTURED

FIG. 9A

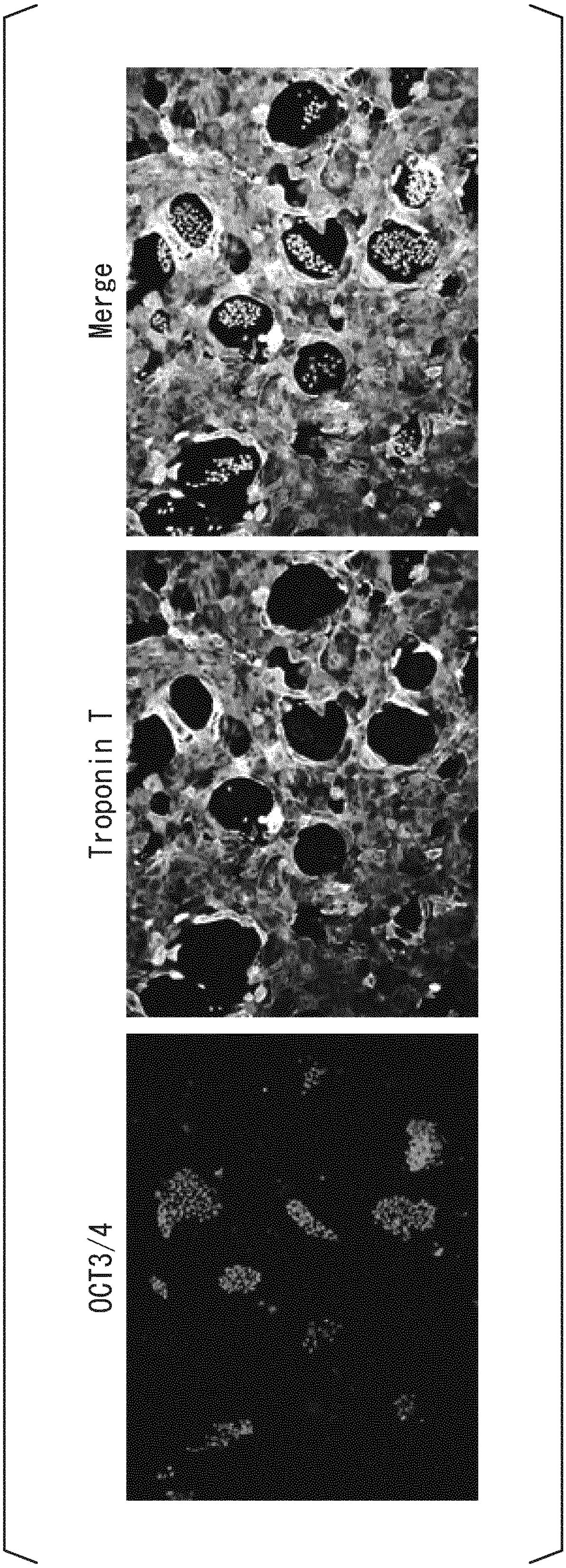

FIG. 9B

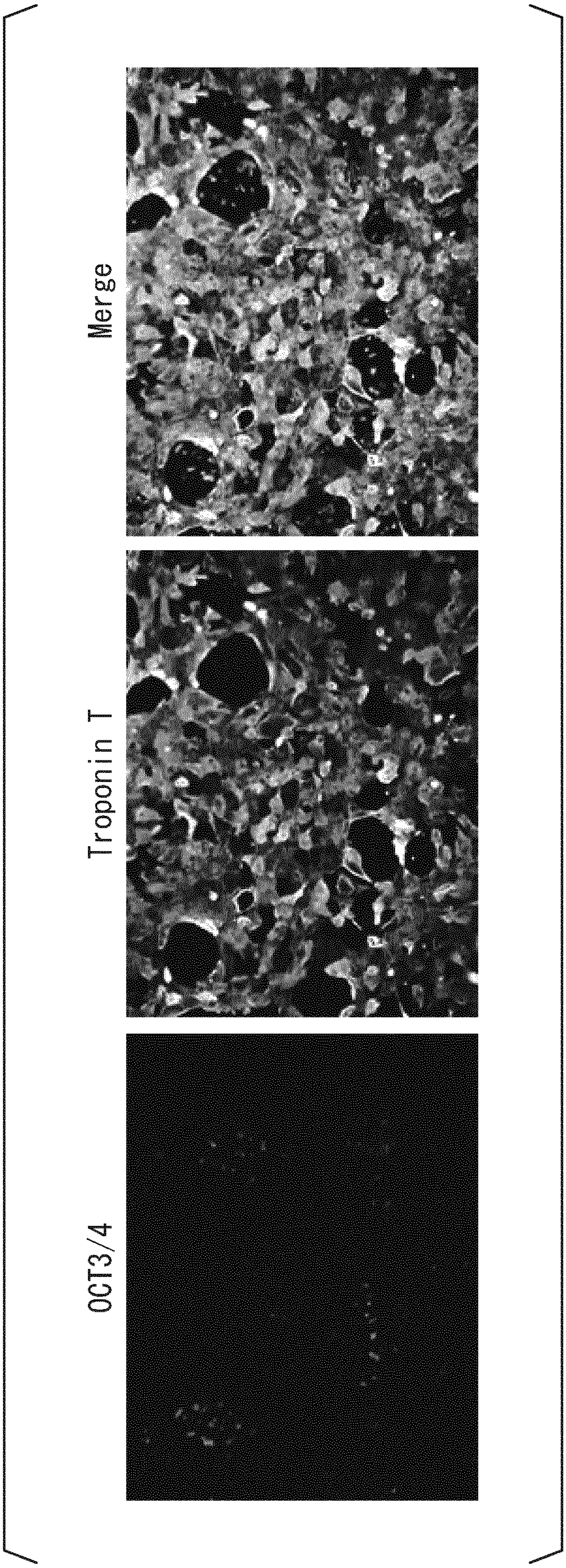

**INTERNATIONAL SEARCH REPORT**

International application No.
PCT/JP2017/044937

A. CLASSIFICATION OF SUBJECT MATTER
Int.Cl. C12N5/077(2010.01)i, A61K35/17(2015.01)i, A61K39/395(2006.01)i, A61K48/00(2006 01)i, A61P43/00(2006.01)i, C12N5/0735(2010.01)i, C12N5/10(2006.01)i, C07K16/28(2006.01)n, C12N5/0783(2010.01)n, C12N15/09(2006.01)n

According to International Patent Classification (IPC) or to both national classification and IPC

B. FIELDS SEARCHED

Minimum documentation searched (classification system followed by classification symbols)
Int.Cl. C12N5/077, A61K35/17, A61K39/395, A61K48/00, A61P43/00, C12N5/0735, C12N5/10, C07K16/28, C12N5/0783, C12N15/09

Documentation searched other than minimum documentation to the extent that such documents are included in the fields searched

| | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)
JSTPlus/JMEDPlus/JST7580 (JDreamIII), CAplus/MEDLINE/BIOSIS/WPIDS (STN)

C. DOCUMENTS CONSIDERED TO BE RELEVANT

| Category* | Citation of document, with indication, where appropriate, of the relevant passages | Relevant to claim No. |
|---|---|---|
| Y | WO 2016/043168 A1 (OSAKA UNIVERSITY) 24 March 2016, claim 2, paragraph [0020] & EP 3196297 A1, claim 2, paragraph [0021] & CN 106715685 A | 1-19 |
| Y | CHAUDHARY, K. W., et al., "Embryonic stem cells in predictive cardiotoxicity: laser capture microscopy enables assay development, Toxicol". Sci., March 2006, vol. 90, no. 1, pp. 149-158, in particular, page 155, right column, paragraph [0002], fig. 2 | 1-19 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 01 March 2018 (01.03.2018) | 13 March 2018 (13.03.2018) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No. PCT/JP2017/044937

C (Continuation). DOCUMENTS CONSIDERED TO BE RELEVANT

| Category* | Citation of document, with indication, where appropriate, of the relevant passages | Relevant to claim No. |
|---|---|---|
| Y | WO 2007/018199 A1 (KUMAMOTO UNIVERSITY) 15 February 2007, claims & US 2009/0239806 A1, claims & EP 1923463 A1 | 1-19 |
| Y | WO 2016/049459 A1 (BAYLOR COLLEGE OF MEDICINE) 31 March 2016, abstract & JP 2017-529851 A & US 2017/0281683 A1 & EP 3198010 A1 | 1-19 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2016243755 A
- WO 2007088874 A
- WO 2009017254 A
- WO 2010114136 A
- WO 2016010165 A
- WO 2006006693 A
- WO 2004018667 A
- WO 20071018199 A
- WO 2007018199 A
- WO 9716338 A
- WO 9716339 A
- WO 01048151 A
- WO 2005033298 A
- WO 2008150030 A
- WO 2006022377 A


### Non-patent literature cited in the description


- **MIURA et al.** *Nature Biotech.,* 2009, vol. 8, 743-745
- **BEN-DAVID et al.** *Cell Stem Cell,* 2013, vol. 12, 167-179
- **WANG et al.** *Science,* 2009, vol. 325, 435-439
- **SHIRAKI et al.** *Cell Metabolism,* 2014, vol. 19, 780-794
- **SUN CK et al.** *Neoplasia.,* August 2011, vol. 13 (8), 735-47
- **TUSCHL et al.** *Genes Cev,* 1999, vol. 13 (24), 3191-7
- Advanced Technology Protocol III for Neuroscience Research, Molecular Neuronal Cell Physiology. Welfare Company, 1993, 153-172
- **MAKOTO TASHIRO.** Virus Experimental Protocol. Medical View Co., Ltd, 1995, 68-73
- **YU et al.** *Science,* 2009, vol. 324, 797-801
- **LIAN, X. et al.** *Nat Protocol,* 2013, vol. 8, 162-175

SEQUENCE LISTING

<110> National Cancer Center Japan
Heartseed Inc.

<120> Agent and Method for Removing an Undifferentiated Stem Cell

<130> PC-24878

<150> JP2016-243755
<151> 2016-12-15

<160> 28

<170> PatentIn version 3.5

<210> 1
<211> 2398
<212> DNA
<213> Homo sapiens

<220>
<221> CDS
<222> (198)..(2009)

<400> 1
```
agccccgccc tgccccgcgc cgccaagcgg ttcccgccct cgcccagcgc ccaggtagct       60

gcgaggaaac ttttgcagcg gctgggtagc agcacgtctc ttgctcctca gggccactgc      120

caggcttgcc gagtcctggg actgctctcg ctccggctgc cactctcccg cgctctccta      180

gctccctgcg aagcagg atg gcc ggg acc gtg cgc acc gcg tgc ttg gtg         230
                   Met Ala Gly Thr Val Arg Thr Ala Cys Leu Val
                   1               5                   10

gtg gcg atg ctg ctc agc ttg gac ttc ccg gga cag gcg cag ccc ccg        278
Val Ala Met Leu Leu Ser Leu Asp Phe Pro Gly Gln Ala Gln Pro Pro
            15                  20                  25

ccg ccg ccg ccg gac gcc acc tgt cac caa gtc cgc tcc ttc ttc cag        326
Pro Pro Pro Pro Asp Ala Thr Cys His Gln Val Arg Ser Phe Phe Gln
        30                  35                  40

aga ctg cag ccc gga ctc aag tgg gtg cca gaa act ccc gtg cca gga        374
Arg Leu Gln Pro Gly Leu Lys Trp Val Pro Glu Thr Pro Val Pro Gly
    45                  50                  55

tca gat ttg caa gta tgt ctc cct aag ggc cca aca tgc tgc tca aga        422
Ser Asp Leu Gln Val Cys Leu Pro Lys Gly Pro Thr Cys Cys Ser Arg
60                  65                  70                  75

aag atg gaa gaa aaa tac caa cta aca gca cga ttg aac atg gaa cag        470
Lys Met Glu Glu Lys Tyr Gln Leu Thr Ala Arg Leu Asn Met Glu Gln
                80                  85                  90

ctg ctt cag tct gca agt atg gag ctc aag ttc tta att att cag aat        518
Leu Leu Gln Ser Ala Ser Met Glu Leu Lys Phe Leu Ile Ile Gln Asn
            95                  100                 105

gct gcg gtt ttc caa gag gcc ttt gaa att gtt gtt cgc cat gcc aag        566
Ala Ala Val Phe Gln Glu Ala Phe Glu Ile Val Val Arg His Ala Lys
```

```
        110                 115                 120

aac tac acc aat gcc atg ttc aag aac aac tac cca agc ctg act cca        614
Asn Tyr Thr Asn Ala Met Phe Lys Asn Asn Tyr Pro Ser Leu Thr Pro
    125                 130                 135

caa gct ttt gag ttt gtg ggt gaa ttt ttc aca gat gtg tct ctc tac        662
Gln Ala Phe Glu Phe Val Gly Glu Phe Phe Thr Asp Val Ser Leu Tyr
140                 145                 150                 155

atc ttg ggt tct gac atc aat gta gat gac atg gtc aat gaa ttg ttt        710
Ile Leu Gly Ser Asp Ile Asn Val Asp Asp Met Val Asn Glu Leu Phe
                160                 165                 170

gac agc ctg ttt cca gtc atc tat acc cag cta atg aac cca ggc ctg        758
Asp Ser Leu Phe Pro Val Ile Tyr Thr Gln Leu Met Asn Pro Gly Leu
            175                 180                 185

cct gat tca gcc ttg gac atc aat gag tgc ctc cga gga gca aga cgt        806
Pro Asp Ser Ala Leu Asp Ile Asn Glu Cys Leu Arg Gly Ala Arg Arg
        190                 195                 200

gac ctg aaa gta ttt ggg aat ttc ccc aag ctt att atg acc cag gtt        854
Asp Leu Lys Val Phe Gly Asn Phe Pro Lys Leu Ile Met Thr Gln Val
    205                 210                 215

tcc aag tca ctg caa gtc act agg atc ttc ctt cag gct ctg aat ctt        902
Ser Lys Ser Leu Gln Val Thr Arg Ile Phe Leu Gln Ala Leu Asn Leu
220                 225                 230                 235

gga att gaa gtg atc aac aca act gat cac ctg aag ttc agt aag gac        950
Gly Ile Glu Val Ile Asn Thr Thr Asp His Leu Lys Phe Ser Lys Asp
                240                 245                 250

tgt ggc cga atg ctc acc aga atg tgg tac tgc tct tac tgc cag gga        998
Cys Gly Arg Met Leu Thr Arg Met Trp Tyr Cys Ser Tyr Cys Gln Gly
            255                 260                 265

ctg atg atg gtt aaa ccc tgt ggc ggt tac tgc aat gtg gtc atg caa       1046
Leu Met Met Val Lys Pro Cys Gly Gly Tyr Cys Asn Val Val Met Gln
        270                 275                 280

ggc tgt atg gca ggt gtg gtg gag att gac aag tac tgg aga gaa tac       1094
Gly Cys Met Ala Gly Val Val Glu Ile Asp Lys Tyr Trp Arg Glu Tyr
    285                 290                 295

att ctg tcc ctt gaa gaa ctt gtg aat ggc atg tac aga atc tat gac       1142
Ile Leu Ser Leu Glu Glu Leu Val Asn Gly Met Tyr Arg Ile Tyr Asp
300                 305                 310                 315

atg gag aac gta ctg ctt ggt ctc ttt tca aca atc cat gat tct atc       1190
Met Glu Asn Val Leu Leu Gly Leu Phe Ser Thr Ile His Asp Ser Ile
                320                 325                 330

cag tat gtc cag aag aat gca gga aag ctg acc acc act gaa act gag       1238
Gln Tyr Val Gln Lys Asn Ala Gly Lys Leu Thr Thr Thr Glu Thr Glu
            335                 340                 345

aag aaa ata tgg cac ttc aaa tat cct atc ttc ttc ctg tgt ata ggg       1286
Lys Lys Ile Trp His Phe Lys Tyr Pro Ile Phe Phe Leu Cys Ile Gly
        350                 355                 360

cta gac tta cag att ggc aag tta tgt gcc cat tct caa caa cgc caa       1334
```

```
Leu Asp Leu Gln Ile Gly Lys Leu Cys Ala His Ser Gln Gln Arg Gln
    365                 370                 375

tat aga tct gct tat tat cct gaa gat ctc ttt att gac aag aaa gta       1382
Tyr Arg Ser Ala Tyr Tyr Pro Glu Asp Leu Phe Ile Asp Lys Lys Val
380                 385                 390                 395

tta aaa gtt gct cat gta gaa cat gaa gaa acc tta tcc agc cga aga       1430
Leu Lys Val Ala His Val Glu His Glu Glu Thr Leu Ser Ser Arg Arg
                400                 405                 410

agg gaa cta att cag aag ttg aag tct ttc atc agc ttc tat agt gct       1478
Arg Glu Leu Ile Gln Lys Leu Lys Ser Phe Ile Ser Phe Tyr Ser Ala
            415                 420                 425

ttg cct ggc tac atc tgc agc cat agc cct gtg gcg gaa aac gac acc       1526
Leu Pro Gly Tyr Ile Cys Ser His Ser Pro Val Ala Glu Asn Asp Thr
        430                 435                 440

ctt tgc tgg aat gga caa gaa ctc gtg gag aga tac agc caa aag gca       1574
Leu Cys Trp Asn Gly Gln Glu Leu Val Glu Arg Tyr Ser Gln Lys Ala
    445                 450                 455

gca agg aat gga atg aaa aac cag ttc aat ctc cat gag ctg aaa atg       1622
Ala Arg Asn Gly Met Lys Asn Gln Phe Asn Leu His Glu Leu Lys Met
460                 465                 470                 475

aag ggc cct gag cca gtg gtc agt caa att att gac aaa ctg aag cac       1670
Lys Gly Pro Glu Pro Val Val Ser Gln Ile Ile Asp Lys Leu Lys His
                480                 485                 490

att aac cag ctc ctg aga acc atg tct atg ccc aaa ggt aga gtt ctg       1718
Ile Asn Gln Leu Leu Arg Thr Met Ser Met Pro Lys Gly Arg Val Leu
            495                 500                 505

gat aaa aac ctg gat gag gaa ggg ttt gaa agt gga gac tgc ggt gat       1766
Asp Lys Asn Leu Asp Glu Glu Gly Phe Glu Ser Gly Asp Cys Gly Asp
        510                 515                 520

gat gaa gat gag tgc att gga ggc tct ggt gat gga atg ata aaa gtg       1814
Asp Glu Asp Glu Cys Ile Gly Gly Ser Gly Asp Gly Met Ile Lys Val
    525                 530                 535

aag aat cag ctc cgc ttc ctt gca gaa ctg gcc tat gat ctg gat gtg       1862
Lys Asn Gln Leu Arg Phe Leu Ala Glu Leu Ala Tyr Asp Leu Asp Val
540                 545                 550                 555

gat gat gcg cct gga aac agt cag cag gca act ccg aag gac aac gag       1910
Asp Asp Ala Pro Gly Asn Ser Gln Gln Ala Thr Pro Lys Asp Asn Glu
                560                 565                 570

ata agc acc ttt cac aac ctc ggg aac gtt cat tcc ccg ctg aag ctt       1958
Ile Ser Thr Phe His Asn Leu Gly Asn Val His Ser Pro Leu Lys Leu
            575                 580                 585

ctc acc agc atg gcc atc tcg gtg gtg tgc ttc ttc ttc ctg gtg cac       2006
Leu Thr Ser Met Ala Ile Ser Val Val Cys Phe Phe Phe Leu Val His
        590                 595                 600

tga ctgcctggtg cccagcacat gtgctgccct acagcaccct gtggtcttcc            2059

tcgataaagg gaaccacttt cttatttttt tctatttttt ttttttgtt atcctgtata      2119
```

cctcctccag ccatgaagta gaggactaac catgtgttat gttttcgaaa atcaaatggt 2179

atcttttgga ggaagataca ttttagtggt agcatataga ttgtcctttt gcaaagaaag 2239

aaaaaaaacc atcaagttgt gccaaattat tctcctatgt ttggctgcta gaacatggtt 2299

accatgtctt tctctctcac tccctccctt tctatcgttc tctctttgca tggatttctt 2359

tgaaaaaaaa taaattgctc aaataaaaaa aaaaaaaaa 2398

<210> 2
<211> 603
<212> PRT
<213> Homo sapiens

<400> 2

```
Met Ala Gly Thr Val Arg Thr Ala Cys Leu Val Val Ala Met Leu Leu
1               5                   10                  15

Ser Leu Asp Phe Pro Gly Gln Ala Gln Pro Pro Pro Pro Pro Pro Asp
            20                  25                  30

Ala Thr Cys His Gln Val Arg Ser Phe Phe Gln Arg Leu Gln Pro Gly
        35                  40                  45

Leu Lys Trp Val Pro Glu Thr Pro Val Pro Gly Ser Asp Leu Gln Val
    50                  55                  60

Cys Leu Pro Lys Gly Pro Thr Cys Cys Ser Arg Lys Met Glu Glu Lys
65                  70                  75                  80

Tyr Gln Leu Thr Ala Arg Leu Asn Met Glu Gln Leu Leu Gln Ser Ala
                85                  90                  95

Ser Met Glu Leu Lys Phe Leu Ile Ile Gln Asn Ala Ala Val Phe Gln
            100                 105                 110

Glu Ala Phe Glu Ile Val Val Arg His Ala Lys Asn Tyr Thr Asn Ala
        115                 120                 125

Met Phe Lys Asn Asn Tyr Pro Ser Leu Thr Pro Gln Ala Phe Glu Phe
    130                 135                 140

Val Gly Glu Phe Phe Thr Asp Val Ser Leu Tyr Ile Leu Gly Ser Asp
145                 150                 155                 160

Ile Asn Val Asp Asp Met Val Asn Glu Leu Phe Asp Ser Leu Phe Pro
                165                 170                 175

Val Ile Tyr Thr Gln Leu Met Asn Pro Gly Leu Pro Asp Ser Ala Leu
```

180 185 190

Asp Ile Asn Glu Cys Leu Arg Gly Ala Arg Arg Asp Leu Lys Val Phe
195 200 205

Gly Asn Phe Pro Lys Leu Ile Met Thr Gln Val Ser Lys Ser Leu Gln
210 215 220

Val Thr Arg Ile Phe Leu Gln Ala Leu Asn Leu Gly Ile Glu Val Ile
225 230 235 240

Asn Thr Thr Asp His Leu Lys Phe Ser Lys Asp Cys Gly Arg Met Leu
245 250 255

Thr Arg Met Trp Tyr Cys Ser Tyr Cys Gln Gly Leu Met Met Val Lys
260 265 270

Pro Cys Gly Gly Tyr Cys Asn Val Val Met Gln Gly Cys Met Ala Gly
275 280 285

Val Val Glu Ile Asp Lys Tyr Trp Arg Glu Tyr Ile Leu Ser Leu Glu
290 295 300

Glu Leu Val Asn Gly Met Tyr Arg Ile Tyr Asp Met Glu Asn Val Leu
305 310 315 320

Leu Gly Leu Phe Ser Thr Ile His Asp Ser Ile Gln Tyr Val Gln Lys
325 330 335

Asn Ala Gly Lys Leu Thr Thr Thr Glu Thr Glu Lys Lys Ile Trp His
340 345 350

Phe Lys Tyr Pro Ile Phe Phe Leu Cys Ile Gly Leu Asp Leu Gln Ile
355 360 365

Gly Lys Leu Cys Ala His Ser Gln Gln Arg Gln Tyr Arg Ser Ala Tyr
370 375 380

Tyr Pro Glu Asp Leu Phe Ile Asp Lys Lys Val Leu Lys Val Ala His
385 390 395 400

Val Glu His Glu Glu Thr Leu Ser Ser Arg Arg Arg Glu Leu Ile Gln
405 410 415

Lys Leu Lys Ser Phe Ile Ser Phe Tyr Ser Ala Leu Pro Gly Tyr Ile
420 425 430

Cys Ser His Ser Pro Val Ala Glu Asn Asp Thr Leu Cys Trp Asn Gly
435 440 445

Gln Glu Leu Val Glu Arg Tyr Ser Gln Lys Ala Ala Arg Asn Gly Met
450 455 460

Lys Asn Gln Phe Asn Leu His Glu Leu Lys Met Lys Gly Pro Glu Pro
465 470 475 480

Val Val Ser Gln Ile Ile Asp Lys Leu Lys His Ile Asn Gln Leu Leu
485 490 495

Arg Thr Met Ser Met Pro Lys Gly Arg Val Leu Asp Lys Asn Leu Asp
500 505 510

Glu Glu Gly Phe Glu Ser Gly Asp Cys Gly Asp Asp Glu Asp Glu Cys
515 520 525

Ile Gly Gly Ser Gly Asp Gly Met Ile Lys Val Lys Asn Gln Leu Arg
530 535 540

Phe Leu Ala Glu Leu Ala Tyr Asp Leu Asp Val Asp Asp Ala Pro Gly
545 550 555 560

Asn Ser Gln Gln Ala Thr Pro Lys Asp Asn Glu Ile Ser Thr Phe His
565 570 575

Asn Leu Gly Asn Val His Ser Pro Leu Lys Leu Leu Thr Ser Met Ala
580 585 590

Ile Ser Val Val Cys Phe Phe Phe Leu Val His
595 600

<210> 3
<211> 2281
<212> DNA
<213> Homo sapiens

<220>
<221> CDS
<222> (198)..(1892)

<400> 3
agccccgccc tgccccgcgc cgccaagcgg ttcccgccct cgcccagcgc ccaggtagct 60

gcgaggaaac ttttgcagcg gctgggtagc agcacgtctc ttgctcctca gggccactgc 120

caggcttgcc gagtcctggg actgctctcg ctccggctgc cactctcccg cgctctccta 180

gctccctgcg aagcagg atg gcc ggg acc gtg cgc acc gcg tgc ttg gtg 230
Met Ala Gly Thr Val Arg Thr Ala Cys Leu Val

```
                1                   5                   10

gtg gcg atg ctg ctc agc ttg gac ttc ccg gga cag gcg cag ccc ccg        278
Val Ala Met Leu Leu Ser Leu Asp Phe Pro Gly Gln Ala Gln Pro Pro
            15                  20                  25

ccg ccg ccg ccg gac gcc acc tgt cac caa gtc cgc tcc ttc ttc cag        326
Pro Pro Pro Pro Asp Ala Thr Cys His Gln Val Arg Ser Phe Phe Gln
        30                  35                  40

aga ctg cag ccc gga ctc aag tgg gtg cca gaa act ccc gtg cca gga        374
Arg Leu Gln Pro Gly Leu Lys Trp Val Pro Glu Thr Pro Val Pro Gly
    45                  50                  55

tca gat ttg caa gta tgt ctc cct aag ggc cca aca tgc tgc tca aga        422
Ser Asp Leu Gln Val Cys Leu Pro Lys Gly Pro Thr Cys Cys Ser Arg
60                  65                  70                  75

aag atg gaa gaa aaa tac caa cta aca gca cga ttg aac atg gaa cag        470
Lys Met Glu Glu Lys Tyr Gln Leu Thr Ala Arg Leu Asn Met Glu Gln
                80                  85                  90

ctg ctt cag tct gca aag gcc ttt gaa att gtt gtt cgc cat gcc aag        518
Leu Leu Gln Ser Ala Lys Ala Phe Glu Ile Val Val Arg His Ala Lys
            95                  100                 105

aac tac acc aat gcc atg ttc aag aac aac tac cca agc ctg act cca        566
Asn Tyr Thr Asn Ala Met Phe Lys Asn Asn Tyr Pro Ser Leu Thr Pro
        110                 115                 120

caa gct ttt gag ttt gtg ggt gaa ttt ttc aca gat gtg tct ctc tac        614
Gln Ala Phe Glu Phe Val Gly Glu Phe Phe Thr Asp Val Ser Leu Tyr
    125                 130                 135

atc ttg ggt tct gac atc aat gta gat gac atg gtc aat gaa ttg ttt        662
Ile Leu Gly Ser Asp Ile Asn Val Asp Asp Met Val Asn Glu Leu Phe
140                 145                 150                 155

gac agc ctg ttt cca gtc atc tat acc cag cta atg aac cca ggc ctg        710
Asp Ser Leu Phe Pro Val Ile Tyr Thr Gln Leu Met Asn Pro Gly Leu
                160                 165                 170

cct gat tca gcc ttg gac atc aat gag tgc ctc cga gga gca aga cgt        758
Pro Asp Ser Ala Leu Asp Ile Asn Glu Cys Leu Arg Gly Ala Arg Arg
            175                 180                 185

gac ctg aaa gta ttt ggg aat ttc ccc aag ctt att atg acc cag gtt        806
Asp Leu Lys Val Phe Gly Asn Phe Pro Lys Leu Ile Met Thr Gln Val
        190                 195                 200

tcc aag tca ctg caa gtc act agg atc ttc ctt cag gct ctg aat ctt        854
Ser Lys Ser Leu Gln Val Thr Arg Ile Phe Leu Gln Ala Leu Asn Leu
    205                 210                 215

gga att gaa gtg atc aac aca act gat cac ctg aag ttc agt aag gac        902
Gly Ile Glu Val Ile Asn Thr Thr Asp His Leu Lys Phe Ser Lys Asp
220                 225                 230                 235

tgt ggc cga atg ctc acc aga atg tgg tac tgc tct tac tgc cag gga        950
Cys Gly Arg Met Leu Thr Arg Met Trp Tyr Cys Ser Tyr Cys Gln Gly
                240                 245                 250

ctg atg atg gtt aaa ccc tgt ggc ggt tac tgc aat gtg gtc atg caa        998
```

```
Leu Met Met Val Lys Pro Cys Gly Gly Tyr Cys Asn Val Val Met Gln
            255                 260                 265

ggc tgt atg gca ggt gtg gtg gag att gac aag tac tgg aga gaa tac      1046
Gly Cys Met Ala Gly Val Val Glu Ile Asp Lys Tyr Trp Arg Glu Tyr
        270                 275                 280

att ctg tcc ctt gaa gaa ctt gtg aat ggc atg tac aga atc tat gac      1094
Ile Leu Ser Leu Glu Glu Leu Val Asn Gly Met Tyr Arg Ile Tyr Asp
    285                 290                 295

atg gag aac gta ctg ctt ggt ctc ttt tca aca atc cat gat tct atc      1142
Met Glu Asn Val Leu Leu Gly Leu Phe Ser Thr Ile His Asp Ser Ile
300                 305                 310                 315

cag tat gtc cag aag aat gca gga aag ctg acc acc act att ggc aag      1190
Gln Tyr Val Gln Lys Asn Ala Gly Lys Leu Thr Thr Thr Ile Gly Lys
                320                 325                 330

tta tgt gcc cat tct caa caa cgc caa tat aga tct gct tat tat cct      1238
Leu Cys Ala His Ser Gln Gln Arg Gln Tyr Arg Ser Ala Tyr Tyr Pro
            335                 340                 345

gaa gat ctc ttt att gac aag aaa gta tta aaa gtt gct cat gta gaa      1286
Glu Asp Leu Phe Ile Asp Lys Lys Val Leu Lys Val Ala His Val Glu
        350                 355                 360

cat gaa gaa acc tta tcc agc cga aga agg gaa cta att cag aag ttg      1334
His Glu Glu Thr Leu Ser Ser Arg Arg Arg Glu Leu Ile Gln Lys Leu
    365                 370                 375

aag tct ttc atc agc ttc tat agt gct ttg cct ggc tac atc tgc agc      1382
Lys Ser Phe Ile Ser Phe Tyr Ser Ala Leu Pro Gly Tyr Ile Cys Ser
380                 385                 390                 395

cat agc cct gtg gcg gaa aac gac acc ctt tgc tgg aat gga caa gaa      1430
His Ser Pro Val Ala Glu Asn Asp Thr Leu Cys Trp Asn Gly Gln Glu
                400                 405                 410

ctc gtg gag aga tac agc caa aag gca gca agg aat gga atg aaa aac      1478
Leu Val Glu Arg Tyr Ser Gln Lys Ala Ala Arg Asn Gly Met Lys Asn
            415                 420                 425

cag ttc aat ctc cat gag ctg aaa atg aag ggc cct gag cca gtg gtc      1526
Gln Phe Asn Leu His Glu Leu Lys Met Lys Gly Pro Glu Pro Val Val
        430                 435                 440

agt caa att att gac aaa ctg aag cac att aac cag ctc ctg aga acc      1574
Ser Gln Ile Ile Asp Lys Leu Lys His Ile Asn Gln Leu Leu Arg Thr
    445                 450                 455

atg tct atg ccc aaa ggt aga gtt ctg gat aaa aac ctg gat gag gaa      1622
Met Ser Met Pro Lys Gly Arg Val Leu Asp Lys Asn Leu Asp Glu Glu
460                 465                 470                 475

ggg ttt gaa agt gga gac tgc ggt gat gat gaa gat gag tgc att gga      1670
Gly Phe Glu Ser Gly Asp Cys Gly Asp Asp Glu Asp Glu Cys Ile Gly
                480                 485                 490

ggc tct ggt gat gga atg ata aaa gtg aag aat cag ctc cgc ttc ctt      1718
Gly Ser Gly Asp Gly Met Ile Lys Val Lys Asn Gln Leu Arg Phe Leu
            495                 500                 505
```

```
gca gaa ctg gcc tat gat ctg gat gtg gat gat gcg cct gga aac agt       1766
Ala Glu Leu Ala Tyr Asp Leu Asp Val Asp Asp Ala Pro Gly Asn Ser
        510                 515                 520

cag cag gca act ccg aag gac aac gag ata agc acc ttt cac aac ctc       1814
Gln Gln Ala Thr Pro Lys Asp Asn Glu Ile Ser Thr Phe His Asn Leu
    525                 530                 535

ggg aac gtt cat tcc ccg ctg aag ctt ctc acc agc atg gcc atc tcg       1862
Gly Asn Val His Ser Pro Leu Lys Leu Leu Thr Ser Met Ala Ile Ser
540                 545                 550                 555

gtg gtg tgc ttc ttc ttc ctg gtg cac tga ctgcctggtg cccagcacat         1912
Val Val Cys Phe Phe Phe Leu Val His
                560

gtgctgccct acagcaccct gtggtcttcc tcgataaagg gaaccacttt cttatttttt     1972

tctatttttt ttttttgtt atcctgtata cctcctccag ccatgaagta gaggactaac      2032

catgtgttat gttttcgaaa atcaaatggt atcttttgga ggaagataca ttttagtggt     2092

agcatataga ttgtcctttt gcaaagaaag aaaaaaaacc atcaagttgt gccaaattat     2152

tctcctatgt ttggctgcta gaacatggtt accatgtctt tctctctcac tccctccctt     2212

tctatcgttc tctctttgca tggatttctt tgaaaaaaa taaattgctc aaataaaaaa      2272

aaaaaaaaa                                                             2281


<210>  4
<211>  564
<212>  PRT
<213>  Homo sapiens

<400>  4

Met Ala Gly Thr Val Arg Thr Ala Cys Leu Val Val Ala Met Leu Leu
1               5                   10                  15

Ser Leu Asp Phe Pro Gly Gln Ala Gln Pro Pro Pro Pro Pro Pro Asp
            20                  25                  30

Ala Thr Cys His Gln Val Arg Ser Phe Phe Gln Arg Leu Gln Pro Gly
        35                  40                  45

Leu Lys Trp Val Pro Glu Thr Pro Val Pro Gly Ser Asp Leu Gln Val
    50                  55                  60

Cys Leu Pro Lys Gly Pro Thr Cys Cys Ser Arg Lys Met Glu Glu Lys
65                  70                  75                  80

Tyr Gln Leu Thr Ala Arg Leu Asn Met Glu Gln Leu Leu Gln Ser Ala
                85                  90                  95

Lys Ala Phe Glu Ile Val Val Arg His Ala Lys Asn Tyr Thr Asn Ala
```

```
            100                 105                 110

Met Phe Lys Asn Asn Tyr Pro Ser Leu Thr Pro Gln Ala Phe Glu Phe
        115                 120                 125

Val Gly Glu Phe Phe Thr Asp Val Ser Leu Tyr Ile Leu Gly Ser Asp
    130                 135                 140

Ile Asn Val Asp Asp Met Val Asn Glu Leu Phe Asp Ser Leu Phe Pro
145                 150                 155                 160

Val Ile Tyr Thr Gln Leu Met Asn Pro Gly Leu Pro Asp Ser Ala Leu
                165                 170                 175

Asp Ile Asn Glu Cys Leu Arg Gly Ala Arg Arg Asp Leu Lys Val Phe
            180                 185                 190

Gly Asn Phe Pro Lys Leu Ile Met Thr Gln Val Ser Lys Ser Leu Gln
        195                 200                 205

Val Thr Arg Ile Phe Leu Gln Ala Leu Asn Leu Gly Ile Glu Val Ile
    210                 215                 220

Asn Thr Thr Asp His Leu Lys Phe Ser Lys Asp Cys Gly Arg Met Leu
225                 230                 235                 240

Thr Arg Met Trp Tyr Cys Ser Tyr Cys Gln Gly Leu Met Met Val Lys
                245                 250                 255

Pro Cys Gly Gly Tyr Cys Asn Val Val Met Gln Gly Cys Met Ala Gly
            260                 265                 270

Val Val Glu Ile Asp Lys Tyr Trp Arg Glu Tyr Ile Leu Ser Leu Glu
        275                 280                 285

Glu Leu Val Asn Gly Met Tyr Arg Ile Tyr Asp Met Glu Asn Val Leu
    290                 295                 300

Leu Gly Leu Phe Ser Thr Ile His Asp Ser Ile Gln Tyr Val Gln Lys
305                 310                 315                 320

Asn Ala Gly Lys Leu Thr Thr Thr Ile Gly Lys Leu Cys Ala His Ser
                325                 330                 335

Gln Gln Arg Gln Tyr Arg Ser Ala Tyr Tyr Pro Glu Asp Leu Phe Ile
            340                 345                 350
```

Asp Lys Lys Val Leu Lys Val Ala His Val Glu His Glu Glu Thr Leu
355 360 365

Ser Ser Arg Arg Arg Glu Leu Ile Gln Lys Leu Lys Ser Phe Ile Ser
370 375 380

Phe Tyr Ser Ala Leu Pro Gly Tyr Ile Cys Ser His Ser Pro Val Ala
385 390 395 400

Glu Asn Asp Thr Leu Cys Trp Asn Gly Gln Glu Leu Val Glu Arg Tyr
405 410 415

Ser Gln Lys Ala Ala Arg Asn Gly Met Lys Asn Gln Phe Asn Leu His
420 425 430

Glu Leu Lys Met Lys Gly Pro Glu Pro Val Val Ser Gln Ile Ile Asp
435 440 445

Lys Leu Lys His Ile Asn Gln Leu Leu Arg Thr Met Ser Met Pro Lys
450 455 460

Gly Arg Val Leu Asp Lys Asn Leu Asp Glu Glu Gly Phe Glu Ser Gly
465 470 475 480

Asp Cys Gly Asp Asp Glu Asp Glu Cys Ile Gly Gly Ser Gly Asp Gly
485 490 495

Met Ile Lys Val Lys Asn Gln Leu Arg Phe Leu Ala Glu Leu Ala Tyr
500 505 510

Asp Leu Asp Val Asp Asp Ala Pro Gly Asn Ser Gln Gln Ala Thr Pro
515 520 525

Lys Asp Asn Glu Ile Ser Thr Phe His Asn Leu Gly Asn Val His Ser
530 535 540

Pro Leu Lys Leu Leu Thr Ser Met Ala Ile Ser Val Val Cys Phe Phe
545 550 555 560

Phe Leu Val His

<210> 5
<211> 2167
<212> DNA
<213> Homo sapiens

<220>

```
<221>  CDS
<222>  (198)..(1778)

<400>  5
agccccgccc tgccccgcgc cgccaagcgg ttcccgccct cgcccagcgc ccaggtagct      60

gcgaggaaac ttttgcagcg gctgggtagc agcacgtctc ttgctcctca gggccactgc     120

caggcttgcc gagtcctggg actgctctcg ctccggctgc cactctcccg cgctctccta     180

gctccctgcg aagcagg atg gcc ggg acc gtg cgc acc gcg tgc ttg gtg        230
                   Met Ala Gly Thr Val Arg Thr Ala Cys Leu Val
                   1               5                   10

gtg gcg atg ctg ctc agc ttg gac ttc ccg gga cag gcg cag ccc ccg       278
Val Ala Met Leu Leu Ser Leu Asp Phe Pro Gly Gln Ala Gln Pro Pro
            15                  20                  25

ccg ccg ccg ccg gac gcc acc tgt cac caa gtc cgc tcc ttc ttc cag       326
Pro Pro Pro Pro Asp Ala Thr Cys His Gln Val Arg Ser Phe Phe Gln
        30                  35                  40

aga ctg cag ccc gga ctc aag tgg gtg cca gaa act ccc gtg cca gag       374
Arg Leu Gln Pro Gly Leu Lys Trp Val Pro Glu Thr Pro Val Pro Glu
    45                  50                  55

gcc ttt gaa att gtt gtt cgc cat gcc aag aac tac acc aat gcc atg       422
Ala Phe Glu Ile Val Val Arg His Ala Lys Asn Tyr Thr Asn Ala Met
60                  65                  70                  75

ttc aag aac aac tac cca agc ctg act cca caa gct ttt gag ttt gtg       470
Phe Lys Asn Asn Tyr Pro Ser Leu Thr Pro Gln Ala Phe Glu Phe Val
                80                  85                  90

ggt gaa ttt ttc aca gat gtg tct ctc tac atc ttg ggt tct gac atc       518
Gly Glu Phe Phe Thr Asp Val Ser Leu Tyr Ile Leu Gly Ser Asp Ile
            95                  100                 105

aat gta gat gac atg gtc aat gaa ttg ttt gac agc ctg ttt cca gtc       566
Asn Val Asp Asp Met Val Asn Glu Leu Phe Asp Ser Leu Phe Pro Val
        110                 115                 120

atc tat acc cag cta atg aac cca ggc ctg cct gat tca gcc ttg gac       614
Ile Tyr Thr Gln Leu Met Asn Pro Gly Leu Pro Asp Ser Ala Leu Asp
    125                 130                 135

atc aat gag tgc ctc cga gga gca aga cgt gac ctg aaa gta ttt ggg       662
Ile Asn Glu Cys Leu Arg Gly Ala Arg Arg Asp Leu Lys Val Phe Gly
140                 145                 150                 155

aat ttc ccc aag ctt att atg acc cag gtt tcc aag tca ctg caa gtc       710
Asn Phe Pro Lys Leu Ile Met Thr Gln Val Ser Lys Ser Leu Gln Val
                160                 165                 170

act agg atc ttc ctt cag gct ctg aat ctt gga att gaa gtg atc aac       758
Thr Arg Ile Phe Leu Gln Ala Leu Asn Leu Gly Ile Glu Val Ile Asn
            175                 180                 185

aca act gat cac ctg aag ttc agt aag gac tgt ggc cga atg ctc acc       806
Thr Thr Asp His Leu Lys Phe Ser Lys Asp Cys Gly Arg Met Leu Thr
        190                 195                 200

aga atg tgg tac tgc tct tac tgc cag gga ctg atg atg gtt aaa ccc       854
```

```
Arg Met Trp Tyr Cys Ser Tyr Cys Gln Gly Leu Met Met Val Lys Pro
    205                 210                 215

tgt ggc ggt tac tgc aat gtg gtc atg caa ggc tgt atg gca ggt gtg      902
Cys Gly Gly Tyr Cys Asn Val Val Met Gln Gly Cys Met Ala Gly Val
220                 225                 230                 235

gtg gag att gac aag tac tgg aga gaa tac att ctg tcc ctt gaa gaa      950
Val Glu Ile Asp Lys Tyr Trp Arg Glu Tyr Ile Leu Ser Leu Glu Glu
                240                 245                 250

ctt gtg aat ggc atg tac aga atc tat gac atg gag aac gta ctg ctt      998
Leu Val Asn Gly Met Tyr Arg Ile Tyr Asp Met Glu Asn Val Leu Leu
            255                 260                 265

ggt ctc ttt tca aca atc cat gat tct atc cag tat gtc cag aag aat     1046
Gly Leu Phe Ser Thr Ile His Asp Ser Ile Gln Tyr Val Gln Lys Asn
        270                 275                 280

gca gga aag ctg acc acc act att ggc aag tta tgt gcc cat tct caa     1094
Ala Gly Lys Leu Thr Thr Thr Ile Gly Lys Leu Cys Ala His Ser Gln
    285                 290                 295

caa cgc caa tat aga tct gct tat tat cct gaa gat ctc ttt att gac     1142
Gln Arg Gln Tyr Arg Ser Ala Tyr Tyr Pro Glu Asp Leu Phe Ile Asp
300                 305                 310                 315

aag aaa gta tta aaa gtt gct cat gta gaa cat gaa gaa acc tta tcc     1190
Lys Lys Val Leu Lys Val Ala His Val Glu His Glu Glu Thr Leu Ser
                320                 325                 330

agc cga aga agg gaa cta att cag aag ttg aag tct ttc atc agc ttc     1238
Ser Arg Arg Arg Glu Leu Ile Gln Lys Leu Lys Ser Phe Ile Ser Phe
            335                 340                 345

tat agt gct ttg cct ggc tac atc tgc agc cat agc cct gtg gcg gaa     1286
Tyr Ser Ala Leu Pro Gly Tyr Ile Cys Ser His Ser Pro Val Ala Glu
        350                 355                 360

aac gac acc ctt tgc tgg aat gga caa gaa ctc gtg gag aga tac agc     1334
Asn Asp Thr Leu Cys Trp Asn Gly Gln Glu Leu Val Glu Arg Tyr Ser
    365                 370                 375

caa aag gca gca agg aat gga atg aaa aac cag ttc aat ctc cat gag     1382
Gln Lys Ala Ala Arg Asn Gly Met Lys Asn Gln Phe Asn Leu His Glu
380                 385                 390                 395

ctg aaa atg aag ggc cct gag cca gtg gtc agt caa att att gac aaa     1430
Leu Lys Met Lys Gly Pro Glu Pro Val Val Ser Gln Ile Ile Asp Lys
                400                 405                 410

ctg aag cac att aac cag ctc ctg aga acc atg tct atg ccc aaa ggt     1478
Leu Lys His Ile Asn Gln Leu Leu Arg Thr Met Ser Met Pro Lys Gly
            415                 420                 425

aga gtt ctg gat aaa aac ctg gat gag gaa ggg ttt gaa agt gga gac     1526
Arg Val Leu Asp Lys Asn Leu Asp Glu Glu Gly Phe Glu Ser Gly Asp
        430                 435                 440

tgc ggt gat gat gaa gat gag tgc att gga ggc tct ggt gat gga atg     1574
Cys Gly Asp Asp Glu Asp Glu Cys Ile Gly Gly Ser Gly Asp Gly Met
    445                 450                 455
```

```
ata aaa gtg aag aat cag ctc cgc ttc ctt gca gaa ctg gcc tat gat        1622
Ile Lys Val Lys Asn Gln Leu Arg Phe Leu Ala Glu Leu Ala Tyr Asp
460                 465                 470                 475

ctg gat gtg gat gat gcg cct gga aac agt cag cag gca act ccg aag        1670
Leu Asp Val Asp Asp Ala Pro Gly Asn Ser Gln Gln Ala Thr Pro Lys
                480                 485                 490

gac aac gag ata agc acc ttt cac aac ctc ggg aac gtt cat tcc ccg        1718
Asp Asn Glu Ile Ser Thr Phe His Asn Leu Gly Asn Val His Ser Pro
            495                 500                 505

ctg aag ctt ctc acc agc atg gcc atc tcg gtg gtg tgc ttc ttc ttc        1766
Leu Lys Leu Leu Thr Ser Met Ala Ile Ser Val Val Cys Phe Phe Phe
        510                 515                 520

ctg gtg cac tga ctgcctggtg cccagcacat gtgctgccct acagcaccct            1818
Leu Val His
    525

gtggtcttcc tcgataaagg gaaccacttt cttatttttt tctatttttt ttttttgtt     1878

atcctgtata cctcctccag ccatgaagta gaggactaac catgtgttat gttttcgaaa     1938

atcaaatggt atcttttgga ggaagataca ttttagtggt agcatataga ttgtcctttt     1998

gcaaagaaag aaaaaaaacc atcaagttgt gccaaattat tctcctatgt ttggctgcta     2058

gaacatggtt accatgtctt tctctctcac tccctccctt tctatcgttc tctctttgca     2118

tggatttctt tgaaaaaaaa taaattgctc aaataaaaaa aaaaaaaaa                 2167


<210>  6
<211>  526
<212>  PRT
<213>  Homo sapiens

<400>  6

Met Ala Gly Thr Val Arg Thr Ala Cys Leu Val Val Ala Met Leu Leu
1               5                   10                  15

Ser Leu Asp Phe Pro Gly Gln Ala Gln Pro Pro Pro Pro Pro Pro Asp
            20                  25                  30

Ala Thr Cys His Gln Val Arg Ser Phe Phe Gln Arg Leu Gln Pro Gly
        35                  40                  45

Leu Lys Trp Val Pro Glu Thr Pro Val Pro Glu Ala Phe Glu Ile Val
    50                  55                  60

Val Arg His Ala Lys Asn Tyr Thr Asn Ala Met Phe Lys Asn Asn Tyr
65                  70                  75                  80

Pro Ser Leu Thr Pro Gln Ala Phe Glu Phe Val Gly Glu Phe Phe Thr
                85                  90                  95
```

```
Asp Val Ser Leu Tyr Ile Leu Gly Ser Asp Ile Asn Val Asp Asp Met
            100                 105                 110

Val Asn Glu Leu Phe Asp Ser Leu Phe Pro Val Ile Tyr Thr Gln Leu
        115                 120                 125

Met Asn Pro Gly Leu Pro Asp Ser Ala Leu Asp Ile Asn Glu Cys Leu
    130                 135                 140

Arg Gly Ala Arg Arg Asp Leu Lys Val Phe Gly Asn Phe Pro Lys Leu
145                 150                 155                 160

Ile Met Thr Gln Val Ser Lys Ser Leu Gln Val Thr Arg Ile Phe Leu
                165                 170                 175

Gln Ala Leu Asn Leu Gly Ile Glu Val Ile Asn Thr Thr Asp His Leu
            180                 185                 190

Lys Phe Ser Lys Asp Cys Gly Arg Met Leu Thr Arg Met Trp Tyr Cys
        195                 200                 205

Ser Tyr Cys Gln Gly Leu Met Met Val Lys Pro Cys Gly Gly Tyr Cys
    210                 215                 220

Asn Val Val Met Gln Gly Cys Met Ala Gly Val Val Glu Ile Asp Lys
225                 230                 235                 240

Tyr Trp Arg Glu Tyr Ile Leu Ser Leu Glu Glu Leu Val Asn Gly Met
                245                 250                 255

Tyr Arg Ile Tyr Asp Met Glu Asn Val Leu Leu Gly Leu Phe Ser Thr
            260                 265                 270

Ile His Asp Ser Ile Gln Tyr Val Gln Lys Asn Ala Gly Lys Leu Thr
        275                 280                 285

Thr Thr Ile Gly Lys Leu Cys Ala His Ser Gln Gln Arg Gln Tyr Arg
    290                 295                 300

Ser Ala Tyr Tyr Pro Glu Asp Leu Phe Ile Asp Lys Lys Val Leu Lys
305                 310                 315                 320

Val Ala His Val Glu His Glu Glu Thr Leu Ser Ser Arg Arg Arg Glu
                325                 330                 335

Leu Ile Gln Lys Leu Lys Ser Phe Ile Ser Phe Tyr Ser Ala Leu Pro
            340                 345                 350
```

```
Gly Tyr Ile Cys Ser His Ser Pro Val Ala Glu Asn Asp Thr Leu Cys
        355                 360                 365

Trp Asn Gly Gln Glu Leu Val Glu Arg Tyr Ser Gln Lys Ala Ala Arg
    370                 375                 380

Asn Gly Met Lys Asn Gln Phe Asn Leu His Glu Leu Lys Met Lys Gly
385                 390                 395                 400

Pro Glu Pro Val Val Ser Gln Ile Ile Asp Lys Leu Lys His Ile Asn
                405                 410                 415

Gln Leu Leu Arg Thr Met Ser Met Pro Lys Gly Arg Val Leu Asp Lys
            420                 425                 430

Asn Leu Asp Glu Glu Gly Phe Glu Ser Gly Asp Cys Gly Asp Asp Glu
        435                 440                 445

Asp Glu Cys Ile Gly Gly Ser Gly Asp Gly Met Ile Lys Val Lys Asn
    450                 455                 460

Gln Leu Arg Phe Leu Ala Glu Leu Ala Tyr Asp Leu Asp Val Asp Asp
465                 470                 475                 480

Ala Pro Gly Asn Ser Gln Gln Ala Thr Pro Lys Asp Asn Glu Ile Ser
                485                 490                 495

Thr Phe His Asn Leu Gly Asn Val His Ser Pro Leu Lys Leu Leu Thr
            500                 505                 510

Ser Met Ala Ile Ser Val Val Cys Phe Phe Phe Leu Val His
        515                 520                 525

<210>  7
<211>  2329
<212>  DNA
<213>  Homo sapiens

<220>
<221>  CDS
<222>  (198)..(1940)

<400>  7
agccccgccc tgccccgcgc cgccaagcgg ttcccgccct cgcccagcgc ccaggtagct      60

gcgaggaaac ttttgcagcg gctgggtagc agcacgtctc ttgctcctca gggccactgc     120

caggcttgcc gagtcctggg actgctctcg ctccggctgc cactctcccg cgctctccta     180
```

```
gctccctgcg aagcagg atg gcc ggg acc gtg cgc acc gcg tgc ttg gtg         230
                   Met Ala Gly Thr Val Arg Thr Ala Cys Leu Val
                   1               5                   10

gtg gcg atg ctg ctc agc ttg gac ttc ccg gga cag gcg cag ccc ccg        278
Val Ala Met Leu Leu Ser Leu Asp Phe Pro Gly Gln Ala Gln Pro Pro
            15                  20                  25

ccg ccg ccg ccg gac gcc acc tgt cac caa gtc cgc tcc ttc ttc cag        326
Pro Pro Pro Pro Asp Ala Thr Cys His Gln Val Arg Ser Phe Phe Gln
        30                  35                  40

aga ctg cag ccc gga ctc aag tgg gtg cca gaa act ccc gtg cca gga        374
Arg Leu Gln Pro Gly Leu Lys Trp Val Pro Glu Thr Pro Val Pro Gly
    45                  50                  55

tca gat ttg caa gta tgt ctc cct aag ggc cca aca tgc tgc tca aga        422
Ser Asp Leu Gln Val Cys Leu Pro Lys Gly Pro Thr Cys Cys Ser Arg
60                  65                  70                  75

aag atg gaa gaa aaa tac caa cta aca gca cga ttg aac atg gaa cag        470
Lys Met Glu Glu Lys Tyr Gln Leu Thr Ala Arg Leu Asn Met Glu Gln
                80                  85                  90

ctg ctt cag tct gca agt atg gag ctc aag ttc tta att att cag aat        518
Leu Leu Gln Ser Ala Ser Met Glu Leu Lys Phe Leu Ile Ile Gln Asn
            95                  100                 105

gct gcg gtt ttc caa gag gcc ttt gaa att gtt gtt cgc cat gcc aag        566
Ala Ala Val Phe Gln Glu Ala Phe Glu Ile Val Val Arg His Ala Lys
        110                 115                 120

aac tac acc aat gcc atg ttc aag aac aac tac cca agc ctg act cca        614
Asn Tyr Thr Asn Ala Met Phe Lys Asn Asn Tyr Pro Ser Leu Thr Pro
    125                 130                 135

caa gct ttt gag ttt gtg ggt gaa ttt ttc aca gat gtg tct ctc tac        662
Gln Ala Phe Glu Phe Val Gly Glu Phe Phe Thr Asp Val Ser Leu Tyr
140                 145                 150                 155

atc ttg ggt tct gac atc aat gta gat gac atg gtc aat gaa ttg ttt        710
Ile Leu Gly Ser Asp Ile Asn Val Asp Asp Met Val Asn Glu Leu Phe
                160                 165                 170

gac agc ctg ttt cca gtc atc tat acc cag cta atg aac cca ggc ctg        758
Asp Ser Leu Phe Pro Val Ile Tyr Thr Gln Leu Met Asn Pro Gly Leu
            175                 180                 185

cct gat tca gcc ttg gac atc aat gag tgc ctc cga gga gca aga cgt        806
Pro Asp Ser Ala Leu Asp Ile Asn Glu Cys Leu Arg Gly Ala Arg Arg
        190                 195                 200

gac ctg aaa gta ttt ggg aat ttc ccc aag ctt att atg acc cag gtt        854
Asp Leu Lys Val Phe Gly Asn Phe Pro Lys Leu Ile Met Thr Gln Val
    205                 210                 215

tcc aag tca ctg caa gtc act agg atc ttc ctt cag gct ctg aat ctt        902
Ser Lys Ser Leu Gln Val Thr Arg Ile Phe Leu Gln Ala Leu Asn Leu
220                 225                 230                 235

gga att gaa gtg atc aac aca act gat cac ctg aag ttc agt aag gac        950
Gly Ile Glu Val Ile Asn Thr Thr Asp His Leu Lys Phe Ser Lys Asp
                240                 245                 250
```

```
tgt ggc cga atg ctc acc aga atg tgg tac tgc tct tac tgc cag gga       998
Cys Gly Arg Met Leu Thr Arg Met Trp Tyr Cys Ser Tyr Cys Gln Gly
            255                 260                 265

ctg atg atg gtt aaa ccc tgt ggc ggt tac tgc aat gtg gtc atg caa      1046
Leu Met Met Val Lys Pro Cys Gly Gly Tyr Cys Asn Val Val Met Gln
        270                 275                 280

ggc tgt atg gca ggt gtg gtg gag att gac aag tac tgg aga gaa tac      1094
Gly Cys Met Ala Gly Val Val Glu Ile Asp Lys Tyr Trp Arg Glu Tyr
    285                 290                 295

att ctg tcc ctt gaa gaa ctt gtg aat ggc atg tac aga atc tat gac      1142
Ile Leu Ser Leu Glu Glu Leu Val Asn Gly Met Tyr Arg Ile Tyr Asp
300                 305                 310                 315

atg gag aac gta ctg ctt ggt ctc ttt tca aca atc cat gat tct atc      1190
Met Glu Asn Val Leu Leu Gly Leu Phe Ser Thr Ile His Asp Ser Ile
                320                 325                 330

cag tat gtc cag aag aat gca gga aag ctg acc acc act att ggc aag      1238
Gln Tyr Val Gln Lys Asn Ala Gly Lys Leu Thr Thr Thr Ile Gly Lys
            335                 340                 345

tta tgt gcc cat tct caa caa cgc caa tat aga tct gct tat tat cct      1286
Leu Cys Ala His Ser Gln Gln Arg Gln Tyr Arg Ser Ala Tyr Tyr Pro
        350                 355                 360

gaa gat ctc ttt att gac aag aaa gta tta aaa gtt gct cat gta gaa      1334
Glu Asp Leu Phe Ile Asp Lys Lys Val Leu Lys Val Ala His Val Glu
    365                 370                 375

cat gaa gaa acc tta tcc agc cga aga agg gaa cta att cag aag ttg      1382
His Glu Glu Thr Leu Ser Ser Arg Arg Arg Glu Leu Ile Gln Lys Leu
380                 385                 390                 395

aag tct ttc atc agc ttc tat agt gct ttg cct ggc tac atc tgc agc      1430
Lys Ser Phe Ile Ser Phe Tyr Ser Ala Leu Pro Gly Tyr Ile Cys Ser
                400                 405                 410

cat agc cct gtg gcg gaa aac gac acc ctt tgc tgg aat gga caa gaa      1478
His Ser Pro Val Ala Glu Asn Asp Thr Leu Cys Trp Asn Gly Gln Glu
            415                 420                 425

ctc gtg gag aga tac agc caa aag gca gca agg aat gga atg aaa aac      1526
Leu Val Glu Arg Tyr Ser Gln Lys Ala Ala Arg Asn Gly Met Lys Asn
        430                 435                 440

cag ttc aat ctc cat gag ctg aaa atg aag ggc cct gag cca gtg gtc      1574
Gln Phe Asn Leu His Glu Leu Lys Met Lys Gly Pro Glu Pro Val Val
    445                 450                 455

agt caa att att gac aaa ctg aag cac att aac cag ctc ctg aga acc      1622
Ser Gln Ile Ile Asp Lys Leu Lys His Ile Asn Gln Leu Leu Arg Thr
460                 465                 470                 475

atg tct atg ccc aaa ggt aga gtt ctg gat aaa aac ctg gat gag gaa      1670
Met Ser Met Pro Lys Gly Arg Val Leu Asp Lys Asn Leu Asp Glu Glu
                480                 485                 490

ggg ttt gaa agt gga gac tgc ggt gat gat gaa gat gag tgc att gga      1718
Gly Phe Glu Ser Gly Asp Cys Gly Asp Asp Glu Asp Glu Cys Ile Gly
```

```
            495                 500                 505

ggc tct ggt gat gga atg ata aaa gtg aag aat cag ctc cgc ttc ctt       1766
Gly Ser Gly Asp Gly Met Ile Lys Val Lys Asn Gln Leu Arg Phe Leu
        510                 515                 520

gca gaa ctg gcc tat gat ctg gat gtg gat gat gcg cct gga aac agt       1814
Ala Glu Leu Ala Tyr Asp Leu Asp Val Asp Asp Ala Pro Gly Asn Ser
    525                 530                 535

cag cag gca act ccg aag gac aac gag ata agc acc ttt cac aac ctc       1862
Gln Gln Ala Thr Pro Lys Asp Asn Glu Ile Ser Thr Phe His Asn Leu
540                 545                 550                 555

ggg aac gtt cat tcc ccg ctg aag ctt ctc acc agc atg gcc atc tcg       1910
Gly Asn Val His Ser Pro Leu Lys Leu Leu Thr Ser Met Ala Ile Ser
                560                 565                 570

gtg gtg tgc ttc ttc ttc ctg gtg cac tga ctgcctggtg cccagcacat         1960
Val Val Cys Phe Phe Phe Leu Val His
            575                 580

gtgctgccct acagcaccct gtggtcttcc tcgataaagg gaaccacttt cttatttttt     2020

tctatttttt tttttttgtt atcctgtata cctcctccag ccatgaagta gaggactaac     2080

catgtgttat gttttcgaaa atcaaatggt atcttttgga ggaagataca ttttagtggt     2140

agcatataga ttgtcctttt gcaaagaaag aaaaaaaacc atcaagttgt gccaaattat     2200

tctcctatgt ttggctgcta gaacatggtt accatgtctt tctctctcac tccctccctt     2260

tctatcgttc tctctttgca tggatttctt tgaaaaaaaa taaattgctc aaataaaaaa     2320

aaaaaaaaa                                                             2329


<210>  8
<211>  580
<212>  PRT
<213>  Homo sapiens

<400>  8

Met Ala Gly Thr Val Arg Thr Ala Cys Leu Val Val Ala Met Leu Leu
1               5                   10                  15


Ser Leu Asp Phe Pro Gly Gln Ala Gln Pro Pro Pro Pro Pro Pro Asp
            20                  25                  30


Ala Thr Cys His Gln Val Arg Ser Phe Phe Gln Arg Leu Gln Pro Gly
        35                  40                  45


Leu Lys Trp Val Pro Glu Thr Pro Val Pro Gly Ser Asp Leu Gln Val
    50                  55                  60


Cys Leu Pro Lys Gly Pro Thr Cys Cys Ser Arg Lys Met Glu Glu Lys
65                  70                  75                  80
```

```
Tyr Gln Leu Thr Ala Arg Leu Asn Met Glu Gln Leu Leu Gln Ser Ala
                85                  90                  95

Ser Met Glu Leu Lys Phe Leu Ile Ile Gln Asn Ala Ala Val Phe Gln
            100                 105                 110

Glu Ala Phe Glu Ile Val Val Arg His Ala Lys Asn Tyr Thr Asn Ala
        115                 120                 125

Met Phe Lys Asn Asn Tyr Pro Ser Leu Thr Pro Gln Ala Phe Glu Phe
    130                 135                 140

Val Gly Glu Phe Phe Thr Asp Val Ser Leu Tyr Ile Leu Gly Ser Asp
145                 150                 155                 160

Ile Asn Val Asp Asp Met Val Asn Glu Leu Phe Asp Ser Leu Phe Pro
                165                 170                 175

Val Ile Tyr Thr Gln Leu Met Asn Pro Gly Leu Pro Asp Ser Ala Leu
            180                 185                 190

Asp Ile Asn Glu Cys Leu Arg Gly Ala Arg Arg Asp Leu Lys Val Phe
        195                 200                 205

Gly Asn Phe Pro Lys Leu Ile Met Thr Gln Val Ser Lys Ser Leu Gln
    210                 215                 220

Val Thr Arg Ile Phe Leu Gln Ala Leu Asn Leu Gly Ile Glu Val Ile
225                 230                 235                 240

Asn Thr Thr Asp His Leu Lys Phe Ser Lys Asp Cys Gly Arg Met Leu
                245                 250                 255

Thr Arg Met Trp Tyr Cys Ser Tyr Cys Gln Gly Leu Met Met Val Lys
            260                 265                 270

Pro Cys Gly Gly Tyr Cys Asn Val Val Met Gln Gly Cys Met Ala Gly
        275                 280                 285

Val Val Glu Ile Asp Lys Tyr Trp Arg Glu Tyr Ile Leu Ser Leu Glu
    290                 295                 300

Glu Leu Val Asn Gly Met Tyr Arg Ile Tyr Asp Met Glu Asn Val Leu
305                 310                 315                 320

Leu Gly Leu Phe Ser Thr Ile His Asp Ser Ile Gln Tyr Val Gln Lys
                325                 330                 335
```

Asn Ala Gly Lys Leu Thr Thr Thr Ile Gly Lys Leu Cys Ala His Ser
340 345 350

Gln Gln Arg Gln Tyr Arg Ser Ala Tyr Tyr Pro Glu Asp Leu Phe Ile
355 360 365

Asp Lys Lys Val Leu Lys Val Ala His Val Glu His Glu Glu Thr Leu
370 375 380

Ser Ser Arg Arg Arg Glu Leu Ile Gln Lys Leu Lys Ser Phe Ile Ser
385 390 395 400

Phe Tyr Ser Ala Leu Pro Gly Tyr Ile Cys Ser His Ser Pro Val Ala
405 410 415

Glu Asn Asp Thr Leu Cys Trp Asn Gly Gln Glu Leu Val Glu Arg Tyr
420 425 430

Ser Gln Lys Ala Ala Arg Asn Gly Met Lys Asn Gln Phe Asn Leu His
435 440 445

Glu Leu Lys Met Lys Gly Pro Glu Pro Val Val Ser Gln Ile Ile Asp
450 455 460

Lys Leu Lys His Ile Asn Gln Leu Leu Arg Thr Met Ser Met Pro Lys
465 470 475 480

Gly Arg Val Leu Asp Lys Asn Leu Asp Glu Glu Gly Phe Glu Ser Gly
485 490 495

Asp Cys Gly Asp Asp Glu Asp Glu Cys Ile Gly Gly Ser Gly Asp Gly
500 505 510

Met Ile Lys Val Lys Asn Gln Leu Arg Phe Leu Ala Glu Leu Ala Tyr
515 520 525

Asp Leu Asp Val Asp Asp Ala Pro Gly Asn Ser Gln Gln Ala Thr Pro
530 535 540

Lys Asp Asn Glu Ile Ser Thr Phe His Asn Leu Gly Asn Val His Ser
545 550 555 560

Pro Leu Lys Leu Leu Thr Ser Met Ala Ile Ser Val Val Cys Phe Phe
565 570 575

Phe Leu Val His

580

<210> 9
<211> 10
<212> PRT
<213> Homo sapiens

<400> 9

Ser Phe Phe Gln Arg Leu Gln Pro Gly Leu
1 5 10

<210> 10
<211> 9
<212> PRT
<213> Homo sapiens

<400> 10

Phe Phe Gln Arg Leu Gln Pro Gly Leu
1 5

<210> 11
<211> 9
<212> PRT
<213> Homo sapiens

<400> 11

Met Phe Lys Asn Asn Tyr Pro Ser Leu
1 5

<210> 12
<211> 9
<212> PRT
<213> Homo sapiens

<400> 12

Phe Thr Asp Val Ser Leu Tyr Ile Leu
1 5

<210> 13
<211> 10
<212> PRT
<213> Homo sapiens

<400> 13

Lys Phe Ser Lys Asp Cys Gly Arg Met Leu
1 5 10

<210> 14
<211> 9
<212> PRT
<213> Homo sapiens

<400> 14

Trp Tyr Cys Ser Tyr Cys Gln Gly Leu
1 5

<210> 15
<211> 10
<212> PRT
<213> Homo sapiens

<400> 15

Lys Tyr Trp Arg Glu Tyr Ile Leu Ser Leu
1 5 10

<210> 16
<211> 9
<212> PRT
<213> Homo sapiens

<400> 16

Glu Tyr Ile Leu Ser Leu Glu Glu Leu
1 5

<210> 17
<211> 9
<212> PRT
<213> Homo sapiens

<400> 17

Ile Tyr Asp Met Glu Asn Val Leu Leu
1 5

<210> 18
<211> 9
<212> PRT
<213> Homo sapiens

<400> 18

Ala Tyr Tyr Pro Glu Asp Leu Phe Ile
1 5

<210> 19
<211> 9
<212> PRT
<213> Homo sapiens

<400> 19

Phe Tyr Ser Ala Leu Pro Gly Tyr Ile
1 5

<210> 20
<211> 10

<212> PRT
<213> Homo sapiens

<400> 20

Arg Phe Leu Ala Glu Leu Ala Tyr Asp Leu
1               5                   10

<210> 21
<211> 9
<212> PRT
<213> Homo sapiens

<400> 21

Arg Leu Gln Pro Gly Leu Lys Trp Val
1               5

<210> 22
<211> 9
<212> PRT
<213> Homo sapiens

<400> 22

Phe Val Gly Glu Phe Phe Thr Asp Val
1               5

<210> 23
<211> 9
<212> PRT
<213> Homo sapiens

<400> 23

Tyr Ile Leu Gly Ser Asp Ile Asn Val
1               5

<210> 24
<211> 20
<212> DNA
<213> Artificial Sequence

<220>
<223> GPC3 forward primer

<400> 24
gagccagtgg tcagtcaaat 20

<210> 25
<211> 19
<212> DNA
<213> Artificial Sequence

<220>
<223> GPC3 reverse primer

<400> 25

```
cttcatcatc accgcagtc                                                  19


<210>  26
<211>  20
<212>  DNA
<213>  Artificial Sequence

<220>
<223>  GAPDH forward primer

<400>  26
gtggacctga cctgccgtct                                                 20


<210>  27
<211>  20
<212>  DNA
<213>  Artificial Sequence

<220>
<223>  GAPDH reverse primer

<400>  27
ggaggagtgg gtgtcgctgt                                                 20


<210>  28
<211>  25
<212>  RNA
<213>  Artificial Sequence

<220>
<223>  siRNA against GPC3

<400>  28
ggaggcucug gugauggaau gauaa                                           25
```